# EUROPÄISCHE PATENTSCHRIFT

(11) **EP 0 508 234 B1**
(45) Veröffentlichungstag und Bekanntmachung des Hinweises auf die Patenterteilung: **24.07.1996**
(21) Anmeldenummer: 92105263.5
(22) Anmeldetag: 27.03.1992
(51) Int. Cl.: C07D 487/04, C07D 471/14, A61K 31/40, A61K 31/435

(54) **Beta-Lactame**
Beta-lactames
Bêta-lactames

(30) Priorität: 11.04.1991 CH 1083/91; 13.02.1992 CH 429/92
(43) Veröffentlichungstag der Anmeldung: 14.10.1992
(73) Patentinhaber: F. HOFFMANN-LA ROCHE AG, 4002 Basel (CH)
(72) Erfinder: Charnas, Robert, F-68220 Hésingue (FR); Gubernator, Klaus, W-7800 Freiburg (DE); Heinze, Ingrid, W-7802 Merzhausen (DE); Hubschwerlen, Christian, F-68200 Durmenach (FR)
(74) Vertreter: Bertschinger, Christoph, Dr.

(56) Entgegenhaltungen:
- EP-A- 0 076 758
- EP-A- 0 088 488
- EP-A- 0 232 017
- CHEMICAL ABSTRACTS, vol. 100, no. 19, 7. Mai 1984, Columbus, Ohio, US; abstract no. 156408h, KAMETANI, TETSUJI ET AL. 'Studies on the synthesis of carbapenem antibiotics. Stereoselective synthesis of a potential intermediate for 6-amidocarbapenem antibiotics.' Seite 517; & HETEROCYCLES Bd. 20, Nr. 12, 1983, ENG. Seiten 2355-2358

## Beschreibung

Die vorliegende Erfindung betrifft β-Lactame der allgemeinen Formel 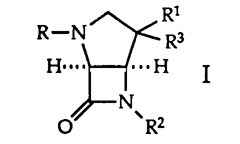
in der R niederes Alkoxycarbonyl, niederes Alkoxycarbonylamino, den Acylrest einer α- oder β-Aminosäure oder einen Rest der allgemeinen Formel

Q-X-Y (a)

bedeutet, worin Q einen 3-6-gliedrigen, gegebenenfalls stickstoff-, schwefel- und/oder sauerstoffhaltigen, gegebenenfalls mit einem ankondensierten Ring verbundenen Ring, X eine direkte Bindung oder einen aus Kohlenstoff, Stickstoff, Sauerstoff und/oder Schwefel bestehenden linearen "Spacer" mit bis zu 6 Atomen bedeutet, wovon bis zu 2 Atomen Stickstoffatome und 1 Atom Sauerstoff oder Schwefel sein können, und Y eine der Gruppen -CO-, -CS-, -CONH- und (falls X als Endglied weder Schwefel noch Carbonyl enthält) -SO₂- darstellt;
und worin ferner R¹ Wasserstoff, Halogen, Carbamoyloxy, niederes Alkanoyloxy oder eine Gruppe der Formel -S-Het, Het eine 5-6-gliedrige, stickstoff-, schwefel- und/oder sauerstoffhaltige heteroaromatische Gruppe und R² die Sulfogruppe -SO₃H darstellt oder R¹ und R² zusammen eine Gruppe der Formel 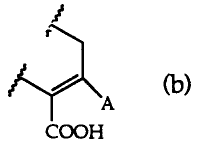
bedeutet, worin A Wasserstoff oder einen in 3-Stellung von Cephalosporinantibiotika verwendbaren Rest darstellt, und worin ferner R³ Wasserstoff oder R¹ und R³ zusammen eine Gruppe der allgemeinen Formel

=CH-R^{a} (c)

darstellen, worin R^{a} eine der Gruppen 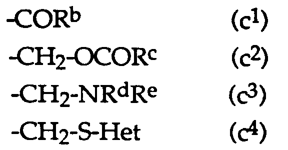
bedeutet, in denen R^{b} niederes Alkoxy oder Amino, R^{c} niederes Alkyl, Phenyl oder Amino, R^{d} und R^{e} jeweils Wasserstoff oder niederes Alkyl oder zusammen mit dem N-Atom einen 5- oder 6-gliedrigen, gegebenenfalls ein weiteres Stickstoff-, Sauerstoff- oder Schwefelatom enthaltenden N-Heterocyclus und Het eine 5-6-gliedrige, stickstoff-, schwefel- und/oder sauerstoffhaltige heteroaromatische Gruppe darstellt,
und pharmazeutisch verträgliche Salze dieser Verbindungen.

Diese Verbindungen sind neu und zeichnen sich durch therapeutisch wertvolle Eigenschaften aus. Insbesondere besitzen sie ausgeprägte β-Lactamase hemmende Eigenschaften und sind somit nützlich bei der Bekämpfung von β-Lactamase bildenden Krankheitserregern in Kombination mit β-Lactam-Antibiotika, wie den Penicillinen und Cephalosporinen.

Der Ausdruck "niederes Alkyl", für sich allein genommen oder in Kombinationen, wie "niederes Alkoxy", "niederes Alkylthio", "niederes Alkylsulfinyl", "niederes Alkylsulfonyl", "niederes Alkoxycarbonyl", "niederes Alkanoyloxy" und dergleichen, bedeutet geradkettige oder verzweigte gesättigte Kohlenwasserstoffreste mit höchstens 7, vorzugsweise höchstens 4, Kohlenstoffatomen, wie Methyl, Aethyl, n-Propyl, Isopropyl, n-Butyl, t-Butyl und dergleichen. "Halogen" bedeutet alle vier Halogene, vorzugsweise aber Chlor oder Fluor. "Amino" kann auch substituiert sein, z.B. durch niederes Alkyl, wie Methylamino, Dimethylamino. "5- bzw. 6-gliedrige, gegebenenfalls ein weiteres Stickstoff-, Sauerstoff- oder Schwefelatom enthaltende Heterocyclen" können auch N- oder C-substituiert sein, z.B. durch niederes Alkyl. Beispiele gesättigter Heterocyclen sind Pyrrolidinyl, Piperidinyl, Piperazinyl, N-Methylpiperazinyl, Morpholinyl und Thiomorpholinyl. Beispiele aromatischer Heterocyclen sind die von Pyridin und (α, β oder γ)-Picolin abgeleiteten Reste, wobei am Stickstoffatom eine positive Ladung ("Zwitterion") vorliegt. Der Ausdruck "Het" steht für eine 5-6-gliedrige heteroaromatische Gruppe, wie z.B. Tetrazolyl, Methyltetrazolyl, Methylthiazolyl.

Die in den Verbindungen der Formel I vorhandenen Gruppen R haben die folgenden Bedeutungen a), b), c) und d):
a) Niederes Alkoxycarbonyl, wie z.B. Methoxycarbonyl, Aethoxycarbonyl oder t-Butoxycarbonyl.
b) Niederes Alkoxycarbonylamino, wie z.B. Methoxycarbonylamino, Aethoxycarbonylamino oder t-Butoxycarbonylamino.
c) Den Arylrest einer α- oder β-Aminosäure, die sowohl eine natürliche wie auch eine unnatürliche Aminosäure sein kann, wie z.B. eine von Glycin, Alanin, β-Alanin, Methionin, Leucin, Isoleucin, Ornitin, Asparaginsäure, Arginin, Lysin, Serin, Cystin, Cystein, Valin, Phenylalanin, 3-Hydroxyprolin, 4-Hydroxyprolin, Prolin, Tyrosin, Tryptophan, Threonin, Asparagin, Glutamin, Sarcosin, 2-(2-Thienyl)-glycin, 2-(2-Amino-4-thiazolyl)-glycin, Histidin, 2-Phenylglycin, p-Hydroxyphenylglycin, m-Hydroxyphenylglycin, o-Fluorphenylglycin, m,p-Dihydroxyphenylglycin oder α-Aminozimtsäure abgeleitete Acylgruppe. Die Aminogruppe kann ggfs. substituiert sein, z.B. durch niederes Alkyl, wie Methyl oder Aethyl, durch Aryl, insbesondere durch Phenyl (wie in N-Phenylglycyl), durch Aryl, insbesondere durch niederes Alkanoyl, wie Acetyl oder Propionyl, durch Benzoyl, Benzyloxycarbonyl, t-Butoxycarbonyl oder N-Heterocyclylcarbonyl, wie (4-Aethyl-2,3-dioxo-1-piperazinyl)-carbonyl oder (4-Hydroxy-6-methyl-3-pyridyl)-carbonyl. Von den Acylresten der α- und β-Aminosäuren sind diejenigen bevorzugt, welche sich von einer α-Aminosäure ableiten.
d) Einen Rest der allgemeinen Formel

   Q-X-Y (a)

   worin Q, X und Y die obige Bedeutung haben.

Unter der Bedeutung von Q versteht man vorzugsweise einen 5- oder 6-gliedrigen, ggfs. stickstoff-, schwefelstoff- und/oder sauerstoffhaltigen, ggfs. mit einem weiteren ankondensierten Ring verbundenen (hetero)-aromatischen Ring. Heteroaromatische Ringe enthalten im allgemeinen 1-4 Stickstoffatome und/oder 1-2 Schwefel- oder Sauerstoffatome. Beispiele (hetero)aromatischer Ringe Q sind: 2-Furyl, 3-Furyl, Thiazolyl, Thiadiazolyl, 2-Thienyl, 3-Thienyl, 2-Pyrrolyl, 3-Pyrrolyl, Phenyl, 2-Pyridyl, 3-Pyridyl, 4-Pyridyl, Imidazolyl, Pyrazolyl, Triazolyl, Tetrazolyl (z.B. 1H-tetrazol-5-yl und 1H-Tetrazol-1-yl), Pyrazinyl, Pyridazinyl und Pyrimidinyl. Die Q-Gruppen können auch substituiert sein, z.B. durch niederes Alkyl, Hydroxy, niederes Alkoxy niederes Alkanoyloxy, Sulfonyloxy, Halogen, Amino, Dimethylamino oder Chloracetylamino, z.B. 4-Tolyl, 3-Methyl-(2-furyloxy), 1-Methyl-1H-tetrazol-5-yl, 4-Hydroxyphenyl, 4-Anisyl, 3,4,5-Trimethoxyphenyl, 4-Chlorphenyl, 4-Fluor-(2-pyridyl), 2-Amino-4-thiazolyl, 5-Methyl-1,3,4-thiadiazol-2-yl, p-Amino-phenyl, p-(Chloracetylamino)-phenyl, 3,4-Disulfonyloxy-phenyl, 3,4-Diacetoxyphenyl. Ein weiterer Ring kann ankondensiert sein, insbesondere ein Phenylring, wie z.B. in Indolyl, 1H-Benzotriazol-1-yl, 2-Oxo-2H-1-benzopyran-7-yl, 2-Oxo-4-(trifluormethyl)-2H-1-benzopyran-7-yl, oder auch ein 5-6-gliedriger Heterocyclus, wie z.B. in Benzimidazol-5-yl, 1H-Benzotriazol-5-yl.

Q kann aber auch einen 3-6-gliedrigen, ggfs. stickstoff-, schwefel- und/oder sauerstoffhaltigen (hetero)gesättigten Ring bedeuten. Heterocyclische Ringe enthalten im allgemeinen 1-4 Stickstoffatome und/oder 1-2 Schwefel- oder Sauerstoffatome. Beispiele (hetero)gesättigter Ringe Q sind: Cyclopropyl, Cyclobutyl, Cyclopentyl, Cyclohexyl, Piperidyl, Piperazinyl, Morpholinyl, Thiomorpholinyl, Tetrahydrothienyl, Tetrahydrofuryl. Diese Gruppen können auch substituiert sein, z.B. durch niederes Alkyl, Oxo usw., z.B. 4-Methyl-1-piperazinyl, 2-Oxo-3-tetrahydro-thienyl.

Der im Rest (a) der Formel Q-X-Y- vorhandene "Spacer" X kann z.B. aus 1-6 Kohlenstoffatomen bestehen, aus 1 Stickstoffatom und 0-5 Kohlenstoffatomen, aus 2 Stickstoffatomen und 0-4 Kohlenstoffatomen, aus 1 Sauerstoffatom und 0-5 Kohlenstoffatomen, aus 1 Schwefelatom und 0-5 Kohlenstoffatomen. Im "Spacer" kann ein Kohlenstoffatom als Ketogruppe (CO) auftreten, analoges gilt für ein vorhandenes Schwefelatom, das als Sulfonylgruppe (SO₂) auftreten kann. Ein Kohlenstoffatom im "Spacer" kann substituiert sein, z.B. durch Hydroxy, Carboxy, Sulfonyloxy, Carbamoyloxy oder C₁-C₄ Alkyl, wie Methyl, Aethyl oder Isopropyl. Beispiele für unter X fallende "Spacer" sind:
-O-, -S-, -NH-, -NH-NH-, -CH₂-, -CO-, -CH₂O-, -CH₂CH₂-, -CH=CH-, -CH₂NH-, -S-CH₂-, -SO₂-CH₂-, -O-CH₂-, -S-CH₂CH₂-, -CH₂CH₂-NH-;
-CH₂-O-NH-CO-CH₂CH₂-, -CHOH-, -CH(COOH)-, -CH(OSO₃H)-, -CH(OCONH₂)-, -CH[CH(CH₃)₂]-.

Bevorzugt sind: -CH=CH-, -CH₂-, -SCH₂-, -NH- und -CH₂NH-.

Stellt Y die Gruppe -SO₂- dar, kann X als Endglied keinen Schwefel und keine Carbonylgruppe enthalten, mit anderen Worten kann die Gruppe -SO₂-nicht direkt mit einem Schwefelatom oder mit einer Sulfonylgruppe oder Carbonylgruppe verbunden sein. Beispiele für "Spacer" X, die mit Y in der Bedeutung -SO₂- verbunden sein können, sind:
-O-, -NH-, -NH-NH-, -CH₂-, -CH₂O-, -CH₂CH₂-, -CH=CH-, -CH₂NH-, -O-CH₂-, -CH₂CH₂-NH-, -CHOH-, -CH[CH(CH₃)₂].

Bevorzugt sind: -CH₂- und -NH-.

Beispiele für Gruppen R sind:
Phenylcarbamoyl,
p-Hydroxy-phenylcarbamoyl,
Benzylcarbamoyl,
Cyclobutylcarbonyl,
2-(m,p-Dihydroxyphenyl)-äthylcarbonyl,
2-Phenylglycyl,
2-(o-Fluorphenyl)glycyl,
N-Phenylglycyl,
α-Amino-(2-thienyl)acetyl,
3-(4-Imidazolyl)acryloyl,
3-(2-Furyl)acryloyl,
3-(2-Thienyl)acryloyl,
3-(3-Indolyl)acryloyl,
3-(4-Hydroxyphenyl)acryloyl,
N-(m-Aminophenyl)glycyl,
[(1-Methyl-1H-tetrazol-5-yl)thio]acetyl,
[(5-Methyl-1,3,4-thiadiazol-2-yl)thio]acetyl,
1H-Tetrazol-1-ylacetyl,
[1H-Benzotriazol-1-yl)thio]acetyl,
Tyrosyl,
2-(p-Hydroxyphenyl)glycyl,
2-(m-Hydroxyphenyl)glycyl,
2-(m,p-Dihydroxyphenyl)glycyl,
Tryptophanyl,
t-Butoxycarbonyl,
3-Pyridylacetyl,
Benzyloxycarbonyl,
2-(2-Amino-4-thiazolyl)glycyl,
2-Amino-4-thiazolglyoxyloyl,
α-Hydroxy-phenylacetyl,
α-(Sulfonyloxy)-phenylacetyl,
3-α-Acetamidocinnamoyl,
2-Carboxy-2-(3-thienyl)acetyl,
2-Indolylcarbonyl,
2-(R,S)-2-Oxo-3-tetrahydrothienyl-carbamoyl,
2-(R)-2-Oxo-3-tetrahydrothienyl-carbamoyl,
2-(S)-2-Oxo-3-tetrahydrothienyl-carbamoyl.

Die Verbindungen der Formel I können in zwei Untergruppen unterteilt werden, und zwar in bicyclische Verbindungen der Formel 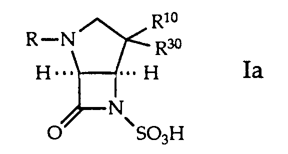
in der R die obige Bedeutung hat, R¹⁰ Wasserstoff, Halogen, Carbamoyloxy, niederes Alkanoyloxy oder eine Gruppe der Formel -S-Het, Het eine 5-6-gliedrige heteroaromatische Gruppe und R³⁰ Wasserstoff oder R¹⁰ und R³⁰ zusammen eine Gruppe der allgemeinen Formel

=CH-R^{a} (c)

darstellen, worin R^{a} eine der Gruppen 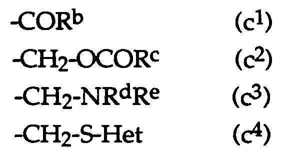
bedeutet, in denen R^{b} niederes Alkoxy oder Amino, R^{c} niederes Alkyl, Phenyl oder Amino, R^{d} und R^{e} jeweils Wasserstoff oder niederes Alkyl oder zusammen mit dem N-Atom einen 5- oder 6-gliedrigen, gegebenenfalls ein weiteres Stickstoff-, Sauerstoff- oder Schwefelatom enthaltenden N-Heterocyclus und Het eine 5-6-gliedrige, stickstoff-, schwefel- und/oder sauerstoffhaltige heteroaromatische Gruppe darstellt,
und deren pharmazeutisch verträgliche Salze, sowie in tricyclische Verbindungen der Formel 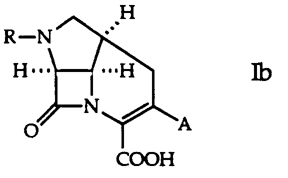
in der R und A die obige Bedeutung haben,
und deren pharmazeutisch verträgliche Salze.

Der als A definierte, in 3-Stellung von Cephalosporinantibiotika verwendbare Rest kann einen üblicherweise in Cephalosporinantibiotika bekannten 3-Substituenten darstellen, z.B. Wasserstoff, Methyl, Methoxy, Chlor oder eine Gruppe -CH₂L, worin L den Rest einer nucleophilen Verbindung bedeutet. Als L-Reste kommen beispielsweise in Betracht: Acetoxy, Carbamoyloxy oder Reste der allgemeinen Formel 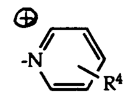
worin R⁴ Wasserstoff oder Carbamoyl darstellen,
oder eine Gruppe -SR⁵ worin R⁵ einen gegebenenfalls substituierten 5- oder 6-gliedrigen Heterocyclus mit 1-4 Heteroatomen, z.B. Sauerstoff, Schwefel, Selen und/oder Stickstoff. Beispiele für R⁵ sind Tetrazolyl, Triazolyl, Thiadiazolyl und Triazinyl. Diese Reste können auch substituiert sein, z.B. durch niederes Alkyl, wie Methyl oder Aethyl, durch Halogen, wie Chlor, Fluor oder Brom, durch Hydroxy oder Oxo-gruppen. Beispiele solcher substituierten Reste sind die 1,2,4-Triazol-3-yl-, 1-Methyl-tetrazol-5-yl-, 2-Methyl-1,3,4-thiadiazol-5-yl-, 1,4,5,6-Tetrahydro-4-methyl-5,6-dioxo-as-triazin-3-yl-, 2,5-Dihydro-6-hydroxy-2-methyl-5-oxo-as-triazin-3-yl- und die 1,2,5,6-Tetrahydro-2-methyl-5,6-dioxo-as-triazin-2-yl-Gruppe.

Gegenstand der vorliegenden Erfindung sind β-Lactame der obigen allgemeinen Formel I und pharmazeutisch verträgliche Salze davon als solche und als pharmazeutische Wirkstoffe, die Herstellung dieser Verbindungen und Zwischenprodukte für die Herstellung dieser Verbindungen, ferner Arzneimittel enthaltend eine Verbindung der allgemeinen Formel I oder pharmazeutisch verträgliche Salze davon und die Herstellung solcher Arzneimittel, sowie die Verwendung von Verbindungen der allgemeinen Formel I und von pharmazeutisch verträglichen Salzen davon bei der Bekämpfung bzw. Verhütung von Krankheiten.

Von den Verbindungen der Formel I, worin R niederes Alkoxycarbonyl darstellt, sind diejenigen, worin R t-Butoxycarbonyl darstellt, und deren pharmazeutisch verträgliche Salze bevorzugt. Besonders bevorzugt sind t-Butoxy-(1S,5R)-7-oxo-6-sulfo-2,6-diazabicyclo[3.2.0]heptan-2-carboxylat und (1aS,3aR,6bR)-1,1a,3a,6b-Tetrahydro-5-methoxy-1-oxo-2,6a-diazacyclobuta-[cd]inden-2,6(3H,4H)-dicarbonsäur-2-t-butylmonoester und deren pharmazeutisch verträgliche Salze.

Weitere bevorzugte Verbindungen der Formel Ia sind:
(1S,5R)-7-Oxo-2-(phenylcarbamoyl)-2,6-diazabicyclo[3.2.0]heptan-6-sulfonsäure,
(1S,5R)-2-(Benzylcarbamoyl)-7-oxo-6-sulfo-2,6-diazabicyclo[3.2.0]heptan-6-sulfonsäure,
(1S,5R)-7-Oxo-2-(N-phenylglycyl)-2,6-diazabicyclo[3.2.0]heptan-6-sulfonsäure,
(1S,5R)-2-Cyclobutylcarbamoyl)-7-oxo-2,6-diazabicyclo[3.2.0]heptan-6-sulfonsäure,
(1S,5R)-7-Oxo-2-(R,S)-(2-oxo-3-tetrahydrothienyl-carbamoyl)-2,6-diazabicyclo[3.2.0]heptan-6-sulfonsäure,
(1S,5R)-7-Oxo-2-(R)-(2-oxo-3-tetrahydrothienyl-carbamoyl)-2,6-diazabicyclo[3.2.0]heptan-6-sulfonsäure,
(1S,5R)-2-(4-Hydroxy-phenylcarbamoyl)-7-oxo-2,6-diazabicyclo[3.2.0]-heptan-6-sulfonsäure
sowie deren pharmazeutisch verträgliche Salze.

Von den Verbindungen der Formel Ia, worin R den Acylrest einer α-Aminosäure darstellt, sind diejenigen der allgemeinen Formel 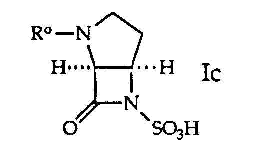
worin R^{o} den Acylrest einer α-Aminosäure der allgemeinen Formel 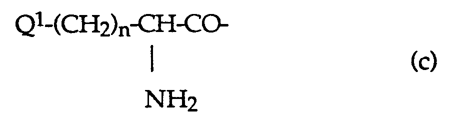
darstellt, und Q¹ einen 5- oder 6-gliedrigen, ggfs. ein Stickstoff-, Schwefel- und/oder Sauerstoffatom enthaltenden, gegebenenfalls mit einem weiteren ankondensierten Phenylring verbundenen (hetero)aromatischen Ring und n die Zahl 0 oder 1 bedeutet,
und deren pharmazeutisch verträgliche Salze bevorzugt.

Unter der Gruppe (c) besonders bevorzugte Gruppen sind diejenigen der Formel 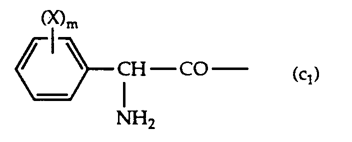
in der X Wasserstoff, Hydroxy oder Fluor und m die Zahl 1 oder 2 darstellt.

In der Gruppe (c) bzw. (c₁) besonders bevorzugte Gruppen Q¹ sind Phenyl, Thienyl, p-Hydroxyphenyl, m-Hydroxyphenyl, m,p-Dihydroxyphenyl, o-Fluorphenyl, m-Fluor-p-hydroxyphenyl, o-Fluor-p-hydroxyphenyl, Indolyl und 2-Amino-4-thiazolyl.

Bevorzugte, die Gruppe (c) enthaltende Verbindungen der Formel Ic sind:
(1S,5R)-7-Oxo-2-L-tyrosyl-2,6-diazabicyclo[3.2.0]heptan-6-sulfonsäure,
(1S,5R)-7-Oxo-2-L-tryptophanyl-2,6-diazabicyclo[3.2.0]heptan-6-sulfonsäure,
(1S,5R)-2-[(R oder S)-α-Amino-(2-thienyl)acetyl]-7-oxo-2,6-diazabicyclo[3.2.0]heptan-6-sulfonsäure,
(1S,5R)-2-[DL-2-(2-Amino-4-thiazolyl)glycyl]-7-oxo-2,6-diaza-bicyclo[3.2.0]heptan-6-sulfonsäure
sowie die pharmazeutisch verträglichen Salze dieser Verbindungen.

Besonders bevorzugte, die Gruppe (c₁) enthaltenden Verbindungen sind;
(1S,5R)-7-Oxo-2-(D-2-phenylglycyl)-2,6-diazabicyclo[3.2.0]heptan-6-sulfonsäure,
(1S,5R)-7-Oxo-2-(L-2-phenylglycyl)-2,6-diazabicyclo[3.2.0]heptan-6-sulfonsäure,
(1S,5R)-2-[D-2-(p-Hydroxyphenyl)glycyl]-7-oxo-2,6-diazabicyclo[3.2.0]-heptan-6-sulfonsäure,
(1S,5R)-2-[R-2-(m-Hydroxyphenyl)glycyl]-7-oxo-2,6-diazabicyclo[3.2.0]-heptan-6-sulfonsäure,
(1S,5R)-2-[DL-2-(m-Hydroxyphenyl)glycyl]-7-oxo-2,6-diazabicyclo[3.2.0]heptan-6-sulfonsäure,
(1S,5R)-2-[DL-2-(o-Fluorphenyl)glycyl]-7-oxo-2,6-diazabicyclo[3.2.0]heptan-6-sulfonsäure,
(1S,5R)-2-[(R,S)-Amino-(3,4-dihydroxy-phenyl)acetyl]-7-oxo-2,6-diazabicyclo[3.2.0]heptan-6-sulfonsäure
sowie die pharmazeutisch verträglichen Salze dieser Verbindungen.

Von den Verbindungen der Formel Ia, worin R einen Rest (a) der Formel Q-X-Y- bedeutet, sind diejenigen der allgemeinen Formel 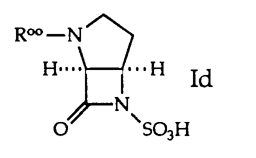
worin R^{oo} eine Gruppe der allgemeinen Formel

Q²-CH=CHCO- (d)

darstellt, und Q² einen Phenylring oder einen 5-6-gliedrigen, Stickstoff, Schwefel und/oder Sauerstoff enthaltenden, gegebenenfalls mit einem vorhandenen Phenylring verbundenen heteroaromatischen Ring bedeutet,
und deren pharmazeutisch verträgliche Salze bevorzugt.

Bevorzugte, die Gruppe (d) enthaltende Verbindungen die Formel Id sind:
(1S,5R)-2-[(E)-3-(2-Furyl)acryloyl]-7-oxo-2,6-diazabicyclo[3.2.0]heptan-6-sulfonsäure,
(1S,5R)-2-[(E)-3-(3-Indolyl)acryloyl]-7-oxo-2,6-diazabicyclo[3.2.0]heptan-6-sulfonsäure,
(1S,5R)-2-[(E)-3-(4-Imidazolyl)acryloyl]-7-oxo-2,6-diazabicyclo[3.2.0]heptan-6-sulfonsäure,
(1S,5R)-2-[(E)-3-(2-Thienyl)acryloyl]-7-oxo-2,6-diazabicyclo[3.2.0]heptan-6-sulfonsäure,
(1S,5R)-(E)-2-[3-(4-Hydroxy-phenyl)acryloyl]-7-oxo-2,6-diazabicyclo[3.2.0]-heptan-6-sulfonsäure
sowie deren pharmazeutische verträgliche Salze.

Weitere bevorzugte Verbindungen der Formel Ia, worin R einen Rest der Formel Q-X-Y- bedeutet, sind Verbindungen der allgemeinen Formel 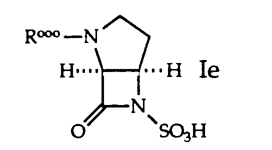
worin R^{ooo} eine Gruppe der allgemeinen Formel

Q³-(S)ₙ-CH₂CO- (e)

darstellt, n 0 oder 1 und Q³ einen Phenylring oder einen 5-6-gliedrigen, gegebenenfalls Schwefel enthaltenden, gegebenenfalls durch niederes Alkyl oder Amino substituierten, gegebenenfalls mit einem ankondensierten Phenylring verbundenen N-Heterocyclus bedeutet,
und deren pharmazeutisch verträgliche Salze.

Bevorzugte, die Gruppe (e) enthaltende Verbindungen der Formel Ie sind:
(1S,5R)-2-[[(1-Methyl-1H-tetrazol-5-yl)thio]acetyl]-7-oxo-2,6-diazabicyclo[3.2.0]heptan-6-sulfonsäure,
18. 1S,5R)-2-[[1H-benzotriazol-1-yl)thio]acetyl]-7-oxo-2,6-diazabicyclo[3.2.0]heptan-6-sulfonsäure,
(1S,5R)-2-[[(5-Methyl-1,3,4-thiadiazol-2-yl)thio]acetyl]-7-oxo-2,6-diazabicyclo[3.2.0]heptan-6-sulfonsäure,
(1S,5R)-7-Oxo-2-(1H-tetrazol-1-ylacetyl)-2,6-diazabicyclo[3.2.0]heptan-6-sulfonsäure,
(1S,5R)-2-[(2-Isopropyl-2H-tetrazol-5-yl)-acetyl]-7-oxo-2,6-diazabicyclo[3.2.0]heptan-6-sulfonsäure,
sowie deren pharmazeutisch verträgliche Salze.

Weitere Verbindungen der Formel Ia worin R einen Rest der Formel Q-X-Y- bedeutet, sind die folgenden:
Benzyl (1S,5R)-7-oxo-6-sulfo-2,6-diazabicyclo[3.2.0]heptan-2-carboxylat,
(1S,5R)-2-[(Z)-3-α-Acetamidocinnamoyl]-7-oxo-2,6-diazabicyclo[3.2.0]heptan-6-sulfonsäure,
(R/S)-α-(1S,5R)-2-[2-carboxy-2-(3-thienyl)acetyl]-7-oxo-2,6-diazabicyclo[3.2.0]heptan-6-sulfonsäure,
(1S,5R)-7-Oxo-2-(3-pyridylacetyl)-2,6-diazabicyclo[3.2.0]heptan-6-sulfonsäure,
(1S,5R)-2-[(R,S)-2-Indolylcarbonyl]-7-oxo-2,6-diazabicyclo[3.2.0]heptan-6-sulfonsäure,
(1S,5R)-2-[(R)-α-Hydroxyphenylacetyl]-7-oxo-2,6-diazabicyclo[3.4.0]heptan-6-sulfonsäure,
(1S,5R)-2-[(S)-α-Hydroxyphenylacetyl]-7-oxo-2,6-diazabicyclo[3.2.0]heptan-6-sulfonsäure,
R(α)-[[(1S,5R)-7-Oxo-6-sulfo-2,6-diazabicyclo[3.2.0]hept-2-yl]carbonyl]-benzylsulfat,
(1S,5R)-2-(2-Amino-4-thiazolglyoxyloyl)-7-oxo-2,6-diazabicyclo[3.2.0]heptan-6-sulfonsäure
sowie die pharmazeutisch verträglichen Salze dieser Verbindungen.

Die erfindungsgemässen β-Lactame der Formel I sowie deren pharmazeutisch verträgliche Salze können erfindungsgemäss hergestellt werden, indem man
(a) eine Verbindung der allgemeinen Formel 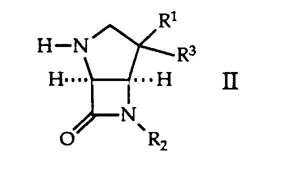 in der R¹, R² und R³ die oben gegebene Bedeutung haben, mit den Rest R abgebenden Mitteln behandelt, oder dass man
(b) zur Herstellung einer Verbindung der Formel I, worin R eine freie Aminogruppe bzw. Hydroxygruppe(n) enthält, in einer Verbindung der allgemeinen Formel 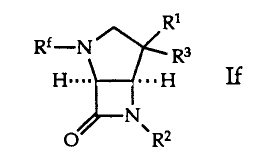 in der R¹, R² und R³ die oben gegebene Bedeutung haben und R^{f} einen unter R definierten Rest mit einer geschützten Aminogruppe bzw. Hydroxygruppe(n) darstellt,
   die Aminoschutzgruppe bzw. Hydroxyschutzgruppe(n) abspaltet, oder dass man
(c) zur Herstellung einer Verbindung der Formel I, worin R¹ und R² von der Gruppe (b) verschieden ist, eine Verbindung der allgemeinen Formel 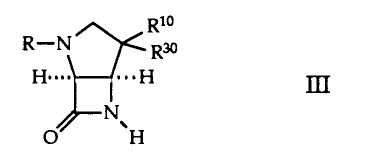 in der R die oben gegebene Bedeutung hat, R¹⁰ Wasserstoff,
   Halogen, Carbamoyloxy, niederes Alkanoyloxy oder eine Gruppe der Formel -S-Het, Het eine 5-6-gliedrige heteroaromatische Gruppe und R³⁰ Wasserstoff oder R¹⁰ und R³⁰ zusammen eine Gruppe der allgemeinen Formel

   =CH-R^{a} (c)

   darstellen, worin R^{a} eine der Gruppen 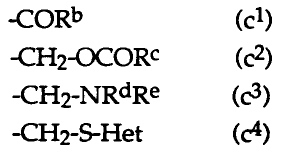 bedeutet, in denen R^{b} niederes Alkoxy oder Amino, R^{c} niederes Alkyl oder Amino, R^{d} und R^{e} jeweils Wasserstoff oder niederes Alkyl oder zusammen mit dem N-Atom einen 5- oder 6-gliedrigen gesättigten, gegebenenfalls ein weiteres Stickstoff-, Sauerstoff- oder Schwefelatom enthaltenden N-Heterocyclus und Het eine 5-6-gliedrige, stickstoff-, schwefel- und/oder sauerstoffhaltige heteroaromatische Gruppe darstellt,
   mit die Sulfogruppe -SO₃H abgebenden Mitteln behandelt, oder dass man
(d) zur Herstellung einer Verbindung der Formel I, worin R¹ und R² zusammen die oben definierte Gruppe (b) darstellt, eine Verbindung der allgemeinen Formel 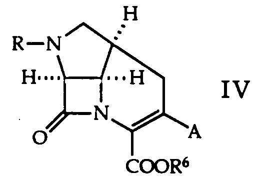 in der R und A die oben angegebenen Bedeutungen haben und R⁶ eine Carbonsäureschutzgruppe darstellt,
   mit die Carbonsäureschutzgruppe R⁶ abspaltenden Mitteln umsetzt, oder dass man
(e) zur Herstellung von pharmazeutisch verträglichen Salzen einer Verbindung der Formel I eine Verbindung der Formel I in ein solches Salz überführt.

Zur Einführung des Restes R in Ausgangsverbindungen der Formel II gemäss Variante (a) des erfindungsgemässen Verfahrens kann man wie folgt vorgehen:
(a¹) Wenn R eine endständige Carbonylgruppe oder Thiocarbonylgruppe enthält, wird eine Verbindung der allgemeinen Formel II mit einer Säure der Formel ROH oder mit einem ihrer reaktionsfähigen Derivate acyliert. Als Acylierungsmittel kommen in Frage: entsprechende Säuren der Formel ROH in Gegenwart von 2-Halogenpyridiniumsalzen, z.B. von 2-Chlor- oder 2-Fluor-1-methylpyridiniumchlorid oder -tosylat, oder auch in Gegenwart von N,N'-Dicyclohexylcarbodiimid oder Carbonyldiimidazol, letzteres bevorzugt zusammen mit N-Hydroxybenztriazol, N-Hydroxysuccinimid oder N-Hydroxyphthalimid. Es können auch entsprechende, reaktionsfähige Derivate der Carbonsäure eingesetzt werden, wie z.B. das Säurehalogenid (vorzugsweise das Chlorid), Säureanhydrid oder Säureazid. Ebenfalls verwendbar sind die entsprechenden Thiolester, wie z.B. 2-Benzthiazolylthioester, wie auch Hydroxybenztriazolester, N-Hydroxysuccinimidester oder N-Hydroxyphthalimidester. Die Umsetzung wird vorzugsweise in einem organischen Lösungsmittel oder Lösungsmittelgemisch, gegebenenfalls im Gemisch mit Wasser, durchgeführt, z.B. Aceton, Methylenchlorid, Tetrahydrofuran, Dioxan, Dimethylacetamid, Dimethylformamid oder Acetonitril, gegebenenfalls im Gemisch mit Wasser. Die Temperatur liegt im allgemeinen zwischen -30°C und Raumtemperatur.
(a²) Wenn R eine endständige Sulfonylgruppe enthält, wird eine Verbindung der allgemeinen Formel III mit einer Säure der Formel ROH oder mit einem ihrer reaktionsfähigen Derivate sulfoniert. Als Sulfonierungsmittel kommen von allem die Sulfonsäurehalogenide, insbesondere die Chloride in Frage. Die Umsetzung erfolgt im wesentlichen sonst wie oben unter (a¹).
(a³) Wenn R eine endständige -CONH-Gruppe enthält, wird eine Verbindung der Formel II mit einem entsprechend substituierten 3-Phenyloxaziridin der allgemeinen Formel 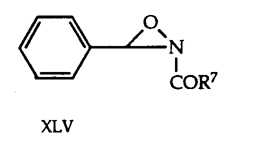 in der R⁷ niederes Alkoxy oder die Gruppe Q-X- und Q und X die obige Bedeutung haben,
umgesetzt. Lösungsmittel und Temperaturbereich sind im wesentlichen wie oben unter (a¹).

Falls die Gruppe Q-X- eingeführt werden soll, wird vorzugsweise eine Ausgangsverbindung der Formel XLV eingesetzt, worin R⁷ niederes Alkoxy, insbesondere Methoxy, darstellt. Das erhaltene Produkt der allgemeinen Formel 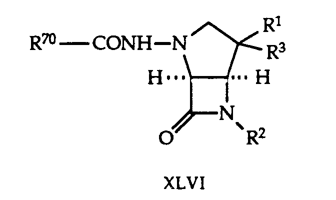
in der R¹, R² und R³ die obige Bedeutung haben und R⁷⁰ niederes Alkoxy darstellt,
wird mit einer Base, z.B. Kaliumhydroxid in wässrigem Tetrahydrofuran bei Raumtemperatur, hydrolysiert und decarboxyliert. Das erhaltene Zwischenprodukt der allgemeinen Formel 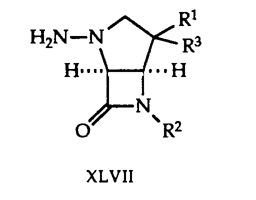
in der R¹, R² und R³ die obige Bedeutung haben,
wird mit einer Säure der allgemeinen Formel Q-X-COOH oder mit einem ihrer reaktionsfähigen Derivate gemäss den Angaben unter (a¹) oben acyliert.

Säuren der Formel ROH, welche Aminogruppen enthalten, sind vorzugsweise an der Aminogruppe geschützt, z.B. durch Benzyloxycarbonyl, t-Butoxycarbonyl oder Chloracetyl. Nach der Acylierung erhaltene entsprechende Verbindungen der Formel If werden erfindungsgemäss gemäss Verfahrensvariante (b) durch Abspaltung der Aminoschutzgruppe in Endprodukte mit einer freien Aminogruppe übergeführt. Die Freisetzung der Aminogruppe erfolgt in an sich bekannter Weise, z.B. durch Hydrierung mit Palladiumkohle oder Behandeln mit Palladiumkohle und 1,4-Cyclohexadien in einem organischen Lösungsmittel, wie Aethanol bei etwa 0-80°C (Benzyloxycarbonyl); mit Trifluoressigsäure, ggfs. in Gegenwart von Anisol, oder mit Chlorwasserstoff in einem organischen Lösungsmittel, wie Dioxan bei etwa -20-0°C (t-Butoxycarbonyl); oder mit Thioharnstoff in einem polaren Lösungsmittel, vorzugsweise in Wasser bei neutralem pH, und etwa 0-30°C (Chloracetyl).

Falls ein Endprodukt der Formel I hergestellt werden soll, in der R zwei vicinale Hydroxygruppen enthält, können in der entsprechenden Ausgangsverbindung der Formel ROH diese Hydroxygruppen geschützt sein; nach erfolgter Umsetzung werden die Hydroxygruppen wieder freigesetzt.

Vorzugsweise schützt man durch Diphenylmethyl (beispielsweise geht dann eine 3,4-Dihydroxyphenylgruppe in die 2,2-Diphenyl-1,3-benzodioxol-5-yl-gruppe über), indem man mit Diphenyldichlormethan erhitzt. Die Abspaltung der Diphenylmethylgruppe erfolgt vorzugsweise durch Einwirken eines sauren Mittels in einer Spur Wasser, z.B. durch konzentrierte wässrige Salzsäure oder, insbesondere durch Trifluoressigsäure mit einer Spur Wasser. Als Reaktionstemperatur eignet sich 0°C bis etwa Raumtemperatur. Wahlweise bewirkt man auch diese Spaltung durch Hydrierung mit Palladiumkohle in einem inerten organischen Lösungsmittel, z.B. Methanol, Aethanol, Tetrahydrofuran oder Aethylacetat, bei einer Temperatur zwischen etwa 0°C und 80°C.

Die Hydroxygruppen können auch durch niedere Alkanoylgruppen, z.B. Acetyl, geschützt werden. Eingeführt wird z.B. durch Behandeln mit einem niederen Alkanoylhalogenid oder-anhydrid, z.B. dem Chlorid, in Gegenwart einer Base, wie Natriumhydroxid, DBU oder Diisopropyläthylamin. Die Abspaltung erfolgt unter milden alkalischen Bedingungen (pH etwa 7-8), z.B. mit Natriumhydroxid oder-carbonat, beietwa 0°C bis 50°C.

Eine weitere Möglichkeit, die Hydroxygruppen zu schützen liegt in der Verwendung von Silylgruppen, z.B. Trimethylsilyl, t-Butyldimethylsilyl. Diese werden vorteilhaft eingeführt durch Behandeln mit dem entsprechenden Silylchlorid. Die Abspaltung kann durch Einwirken eines Fluorids erfolgen, vorzugsweise Tetrabutylammoniumfluorid, in einem organischen Lösungsmittel, z.B. Acetonitril, bei etwa 0°C bis 50°C.

Gemäss Variante (c) des erfindungsgemässen Verfahrens wird eine Verbindung III sulfoniert. Die Sulfonierung kann in an sich bekannter Weise durch Umsetzung mit Schwefeltrioxid oder einem reaktionsfähigen Derivat davon erfolgen, z.B. mit Komplexen von Schwefeltrioxid und einer organischen Base, wie Pyridin, Dimethylformamid, Picolin usw. Die Umsetzung erfolgt z.B. bei etwa -20°C bis +80°C in einem inerten organischen Lösungsmittel, z.B. in einem Aether, wie Dioxan, in einem Ester, wie Aethylacetat, in einem chlorierten Kohlenwasserstoff, wie Methylenchlorid; in Acetonitril, Dimethylformamid oder Pyridin.

Esterschutzgruppen der gemäss Variante (d) des erfindungsgemässen Verfahrens verwendeten Ester der Formel IV sind vorzugsweise solche, welche unter milden Bedingungen in die freie Carboxygruppe umgewandelt werden können, z.B. t-Butyl, p-Nitrobenzyl, Benzyl, Benzhydryl, Allyl, etc. Die Esterschutzgruppen werden z.B. wie folgt abgespalten: Benzyl und p-Nitrobenzyl durch Hydrierung über Palladiumkohle bei etwa 0°C bis 80°C in einem organischen Lösungsmittel wie Aethylacetat, Methanol oder Wasser oder durch Hydrolyse in Gegenwart von Natriumsulfid bei (oder unterhalb) 0°C bis Zimmertemperatur in einem Lösungsmittel, wie z.B. Dimethylformamid (vorzugsweise wässrig); t-Butyl durch Behandeln mit wässriger Salzsäure oder durch Umsetzung mit Trifluoressigsäure in Gegenwart von Anisol bei etwa 0°C bis Zimmertemperatur mit oder ohne zusätzliches Lösungsmittel, wie z.B. Methylenchlorid; Allyl durch Palladium-(O)-katalysierte Transallylierung in Gegenwart eines Natrium- oder Kaliumsalzes der 2-Aethylcapronsäure, siehe z.B. J. Org. Chem., 1982, 47, 587.

Die folgenden Reaktionsschemas I-IV veranschaulichen das Verfahren zur Herstellung der erfindungsgemässen Produkte bzw. der in der Synthese anfallenden Zwischenprodukte. 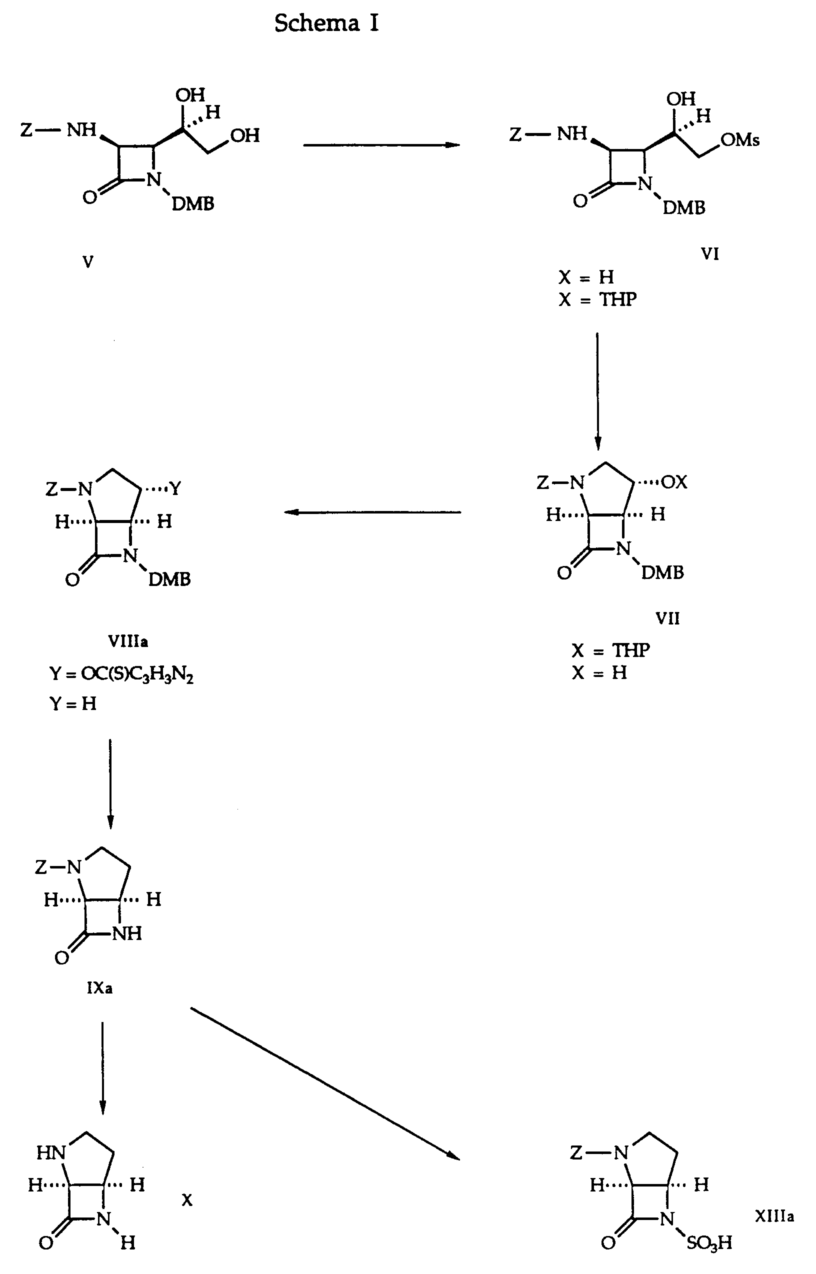 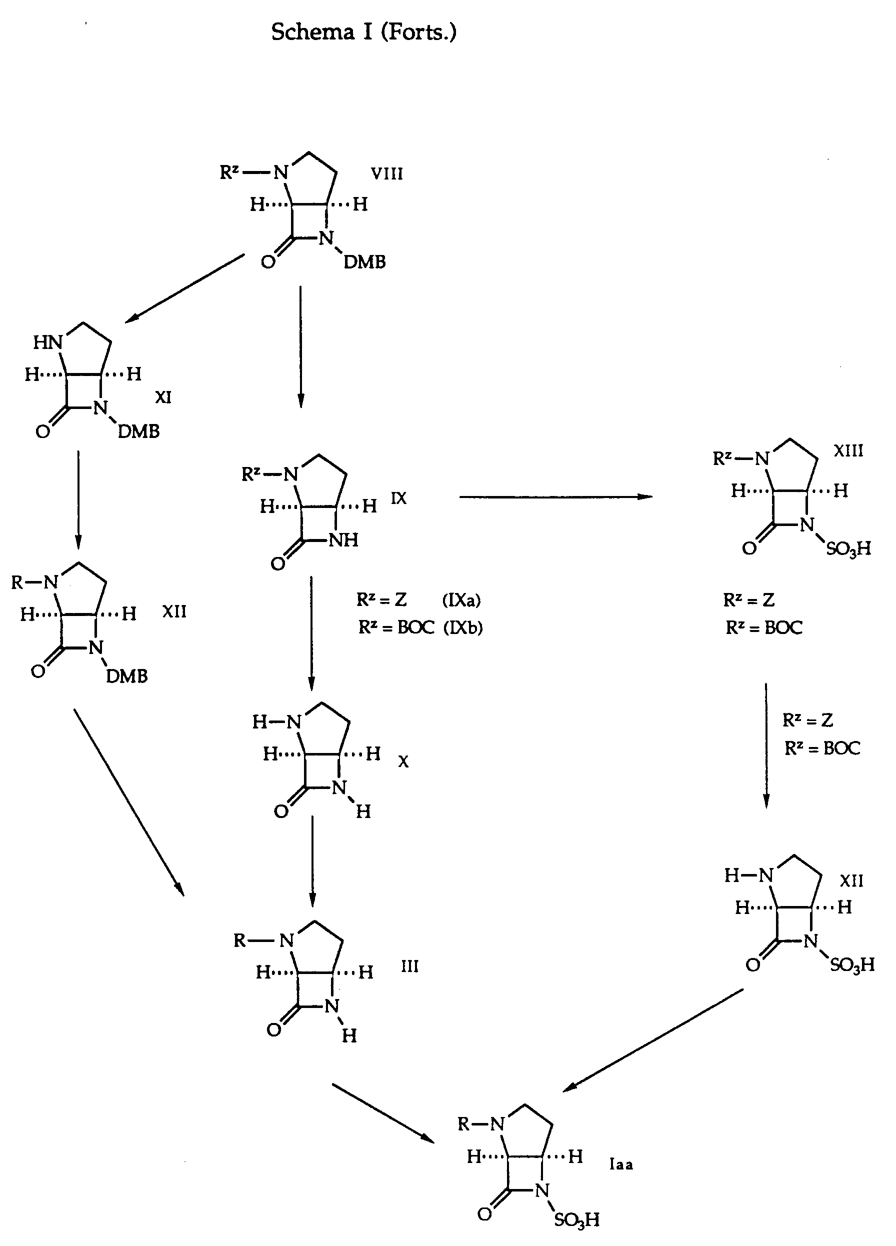 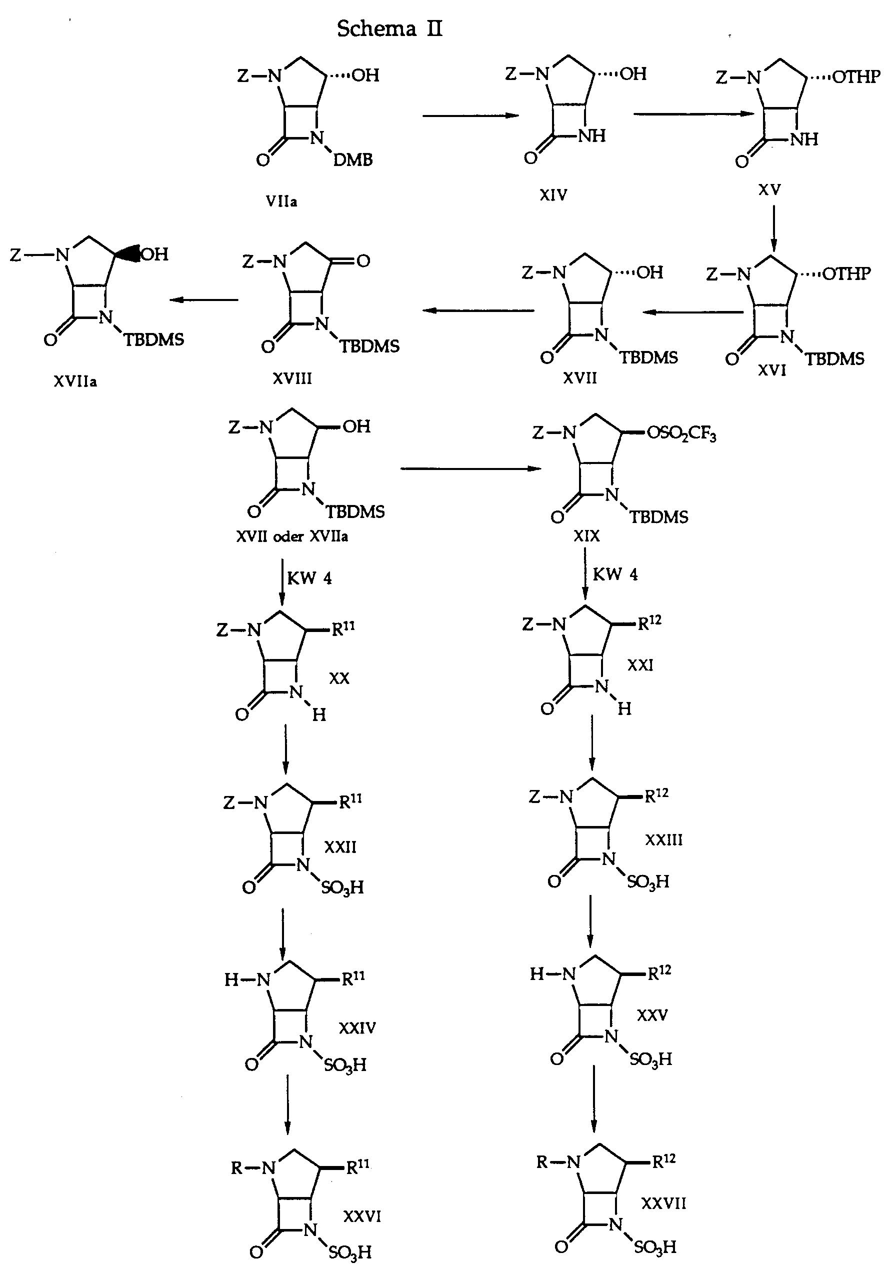 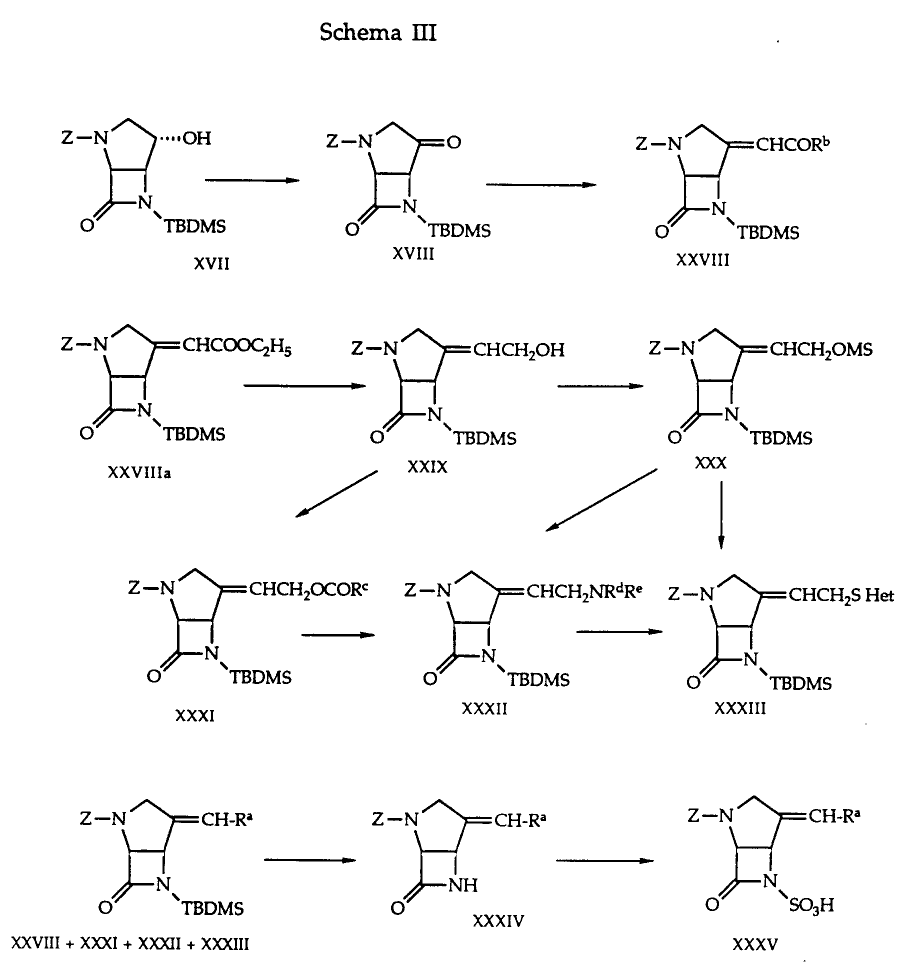 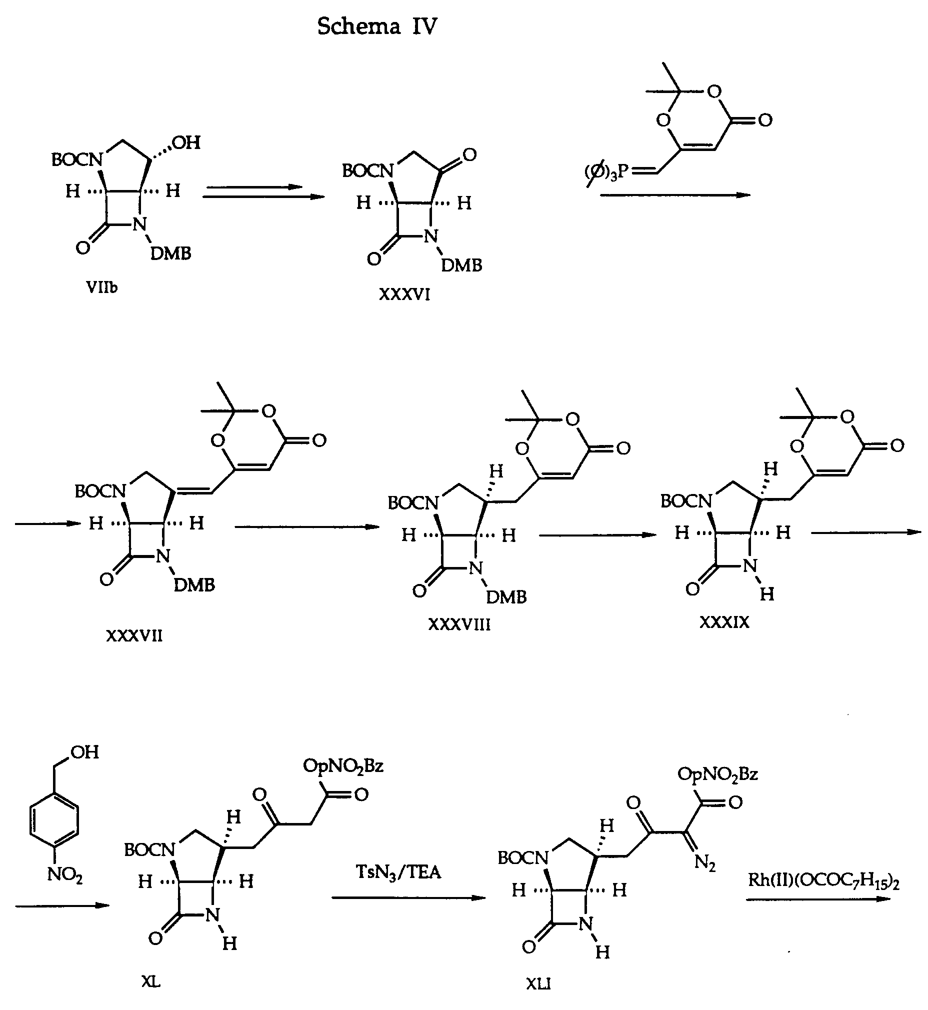 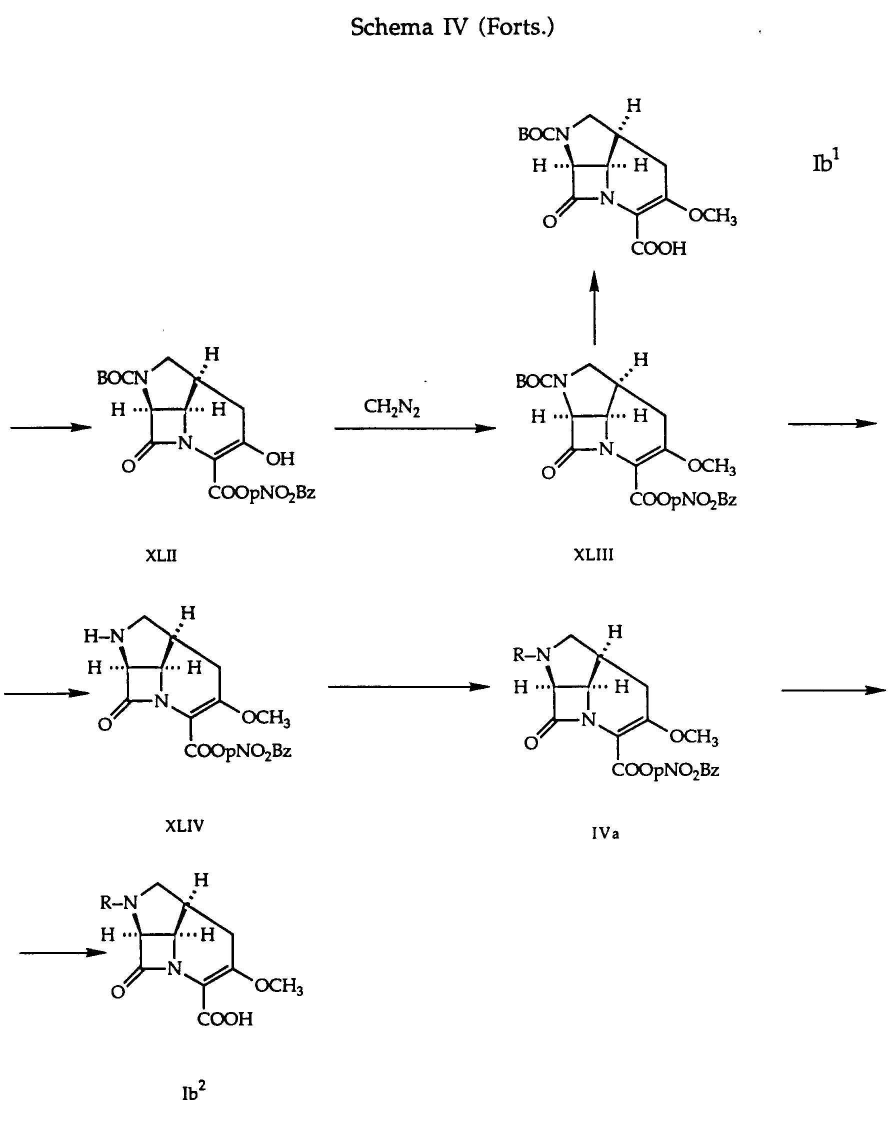
In den Schemas I-IV bedeuten:
- X:: Wasserstoff oder Tetrahydropyranyl (THP)
- THP =: Tetrahydrophenyl
- Ms:: Methansulfonyl
- Y =: Wasserstoff oder [(1-Imidazolyl)thiocarbonyl]oxy
- R^{z} =: Benzyloxycarbonyl oder t-Butoxycarbonyl
- Z =: Benzyloxycarbonyl
- BOC =: t-Butoxycarbonyl
- Ø =: Phenyl
- R, R^{a}, R^{b}, R^{c}, R^{d}, R^{e} =: die obigen, für die Formel I angegebenen Bedeutungen
- DMB =: 3,4-Dimethoxybenzyl oder 2,4-Dimethoxybenzyl
- pNO₂Bz =: p-Nitrobenzyl
- TBDMS =: t-Butyl-dimethylsilanyl
- KW4 =: Konfigurationswechsel in Stellung 4 (vgl. nachstehend)
- R¹¹ =: Carbamoyloxy, niederes Alkanoyloxy
- R¹² =: Hal, -S-Het (Het hat die obige Bedeutung)
Das Schema I beschreibt die Herstellung von Verbindungen VIII mit R^{z} = Benzyloxycarbonyl (= Verbindungen VIIIa). Die Herstellung von Verbindungen VIII mit R^{z} = t-Butoxycarbonyl verläuft in ganz analoger Weise.

Weitere Details über die Herstellung der erfindungsgemäss eingesetzten Ausgangsprodukte II, If, III und IV sind den nachstehenden Ausführungsbeispielen zu entnehmen. In Schema II kann das Zwischenprodukt XVIIa in Analogie zu Beispiel 16 mit Natriumborhydrid hergestellt werden. Die Formeln XIX-XXVI sind der Kürze halber mit einer voll gezogenen Linie (―) in 4-Stellung versehen, wobei je nachdem, ob die 4(S)-Hydroxyverbindung XVII oder die 4(R)-Hydroxyverbindung XVIIa eingesetzt wurde, das entsprechende Isomere erhalten wird, wobei die Zwischenprodukte XX und XXI unter Konfigurationswechsel in Stellung 4 anfallen (im Schema II mit "KW4" bezeichnet). Im übrigen werden die Zwischenprodukte XX z.B. hergestellt, indem man mit einem Isocyanat, z.B. Chloracetylisocyanat, umsetzt, gefolgt von Spaltung der Chloracetylgruppe mit einer Base, wie Kaliumhydroxid (R¹¹ = Carbamoyloxy) oder indem man mit dem entsprechenden Säurechlorid, wie Acetylchlorid, oder Säureanhydrid, wie Acetanhydrid, umsetzt (R¹¹ = niederes Alkanoyloxy).

In Schema III kann das Zwischenprodukt XXIX durch Behandeln des Esters XXVIIIa mit Natriumborhydrid hergestellt werden. Das Produkt XXIX kann mit z.B. Mesylchlorid umgesetzt werden, wobei das Produkt XXX entsteht. Durch Umsetzung des Produktes XXIX mit einem niederen Alkanoylchlorid oder einem Isocyanat (z.B. Chloracetylisocyanat, gefolgt von Spaltung der Chloracetylgruppe mit einer Base) enthält man Produkte XXXI, und durch Umsetzung des Produkts XXX mit dem entsprechenden Amin oder heterocyclischen Thiol in Gegenwart einer Base entstehen die Produkte XXXII bzw. XXXIII. Die Produkte XXVIII, XXXI, XXXII, und XXXIII können alle durch Abspaltung der 6-Schutzgruppe und Sulfonylierung in entsprechende Endprodukte XXXV übergeführt werden. Die Gruppe Z lässt sich nicht ohne Sättigung der Doppelbindung in 4-Stellung entfernen, und man muss deshalb, falls ein Endprodukt mit einem anderen Rest R in Stellung 2 gewünscht wird, anstelle des Z-geschützten Zwischenprodukts XVII von der entsprechenden 2-BOC-geschützten Verbindung ausgehen (erhältlich z.B. durch Abhydrierung von Z und Umsetzung mit t-Butoxycarbonylanhydrid).

Wie bereits eingangs erwähnt, zeigen die erfindungsgemässen Verbindungen der allgemeinen Formel I und pharmazeutisch verträgliche Salze davon mit Basen ausgeprägte β-Lactamase hemmende Aktivitäten gegen β-Lactamasen aus verschiedenen Bakterienstämmen. Diese therapeutisch wertvollen Eigenschaften können, wie nachstehend erläutert, an isolierten β-Lactamasen in vitro bestimmt werden:

### A. Isolierung der β-Lactamasen

Verschiedene β-Lactamasen können aus penicillin- bzw. cephalosporinresistenten Bakterienstämmen, wie Klebsiella pneumoniae NCTC 418, Proteus vulgaris 1028, Bacillus licheniformis 749/C, Escherichia coli SN01 und Citrobacter freundii 1203 isoliert werden. Hierzu werden die entsprechenden Stämme in Tryptic Soy Broth (Difco) kultiviert und durch Zentrifugation in der späten logarithmischen Wachstumsphase geerntet (wenn nötig gibt man dem Medium gegen Ende der log-Phase 50-100 mg/l Ampicillin hinzu, um die β-Lactamase zu induzieren). Die so erhaltene Bakterienmasse wird mit 20 mM Tris-HCl-Puffer (pH 7,0) versetzt; unter Kühlung werden die Zellen mit French Press aufgebrochen. Man zentrifugiert (20'000 U/min.) während 20-30 Minuten und erhält einen klaren Rohextrakt. Die Reinigung der Proteine erfolgt nach der Methode von Cartwright, S.J. & Waley, S.G. [Biochem. J. 221, 505-512 (1980)] und, für B. licheniformis, Ellerby, L.M. et al. [Biochemistry 29, 5797-5806 (1990)].

### B. Bestimmung der β-Lactamase-Aktivität

Die Bestimmung der Aktivität der isolierten β-Lactamasen kann nach der Methode von O'Callaghan, C.H. et al. [Antimicr. Ag. Chemother. 1, 283-288 (1972)] mit dem chromogenen Cephalosporin Nitrocefin (87/312 von Glaxo) durchgeführt werden. Der benötigte Versuchsansatz enthält pro ml Wasser: 50 mM Phosphatpuffer (pH 7,0), 0,1 mM Nitrocefin und genügend Enzym (β-Lactamase) um eine ΔA/min. von ca. 0,1 zu erreichen. Die Spaltung des Substrates, die mit einer Farbänderung verbunden ist, erfolgt bei 37°C und wird bei 482 nm mit einem Spektralphotometer quantitativ verfolgt.

### C. Bestimmung der β-Lactamase hemmenden Wirkung der Verbindungen der allgemeinen Formel I

Die oben beschriebene Spaltung des chromogenen Substrates durch β-Lactamasen (Versuch B.) kann durch Zugabe von Verbindungen der allgemeinen Formel I (Inhibitoren) gehemmt werden. Da es sich zeigte, dass die Inhibitoren die β-Lactamase in einer zeitabhängigen Reaktion irreversibel inaktivieren, wird die Reaktion (Spaltung des Substrates), jeweils nach einer Vorinkubationszeit von β-Lactamase mit Inhibitor von 15 Minuten, durch Zugabe des Substrates gestartet. Als Mass für die Affinität des jeweils getesteten Inhibitors zur β-Lactamase, die ein Mass für die Stärke des Inhibitors darstellt, dient diejenige Konzentration, welche die unter obigen Versuchsbedingungen (Versuch B.) in Abwesenheit eines Inhibitors erfolgende Spaltung des Substrates (Nitrocefin) zu 50% hemmt (IC 50 in nM). Zur Bestimmung der IC 50 wurden 4 bis 6 Versuche mit verschiedenen Konzentrationen an Inhibitor durchgeführt. Die Ermittlung der IC 50 erfolgte graphisch.

Die in obigem Versuch (Versuch C) erhaltenen Resultate sind in der nachfolgenden Tabelle 1 dargestellt.

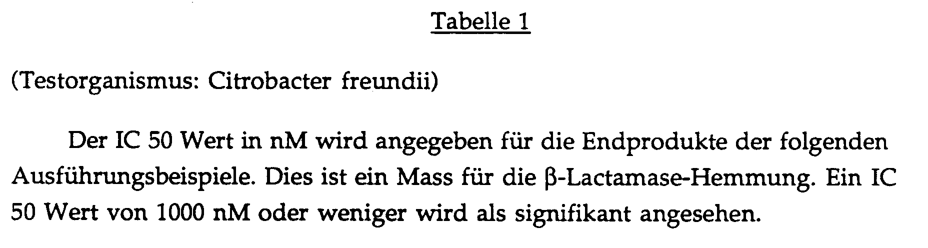

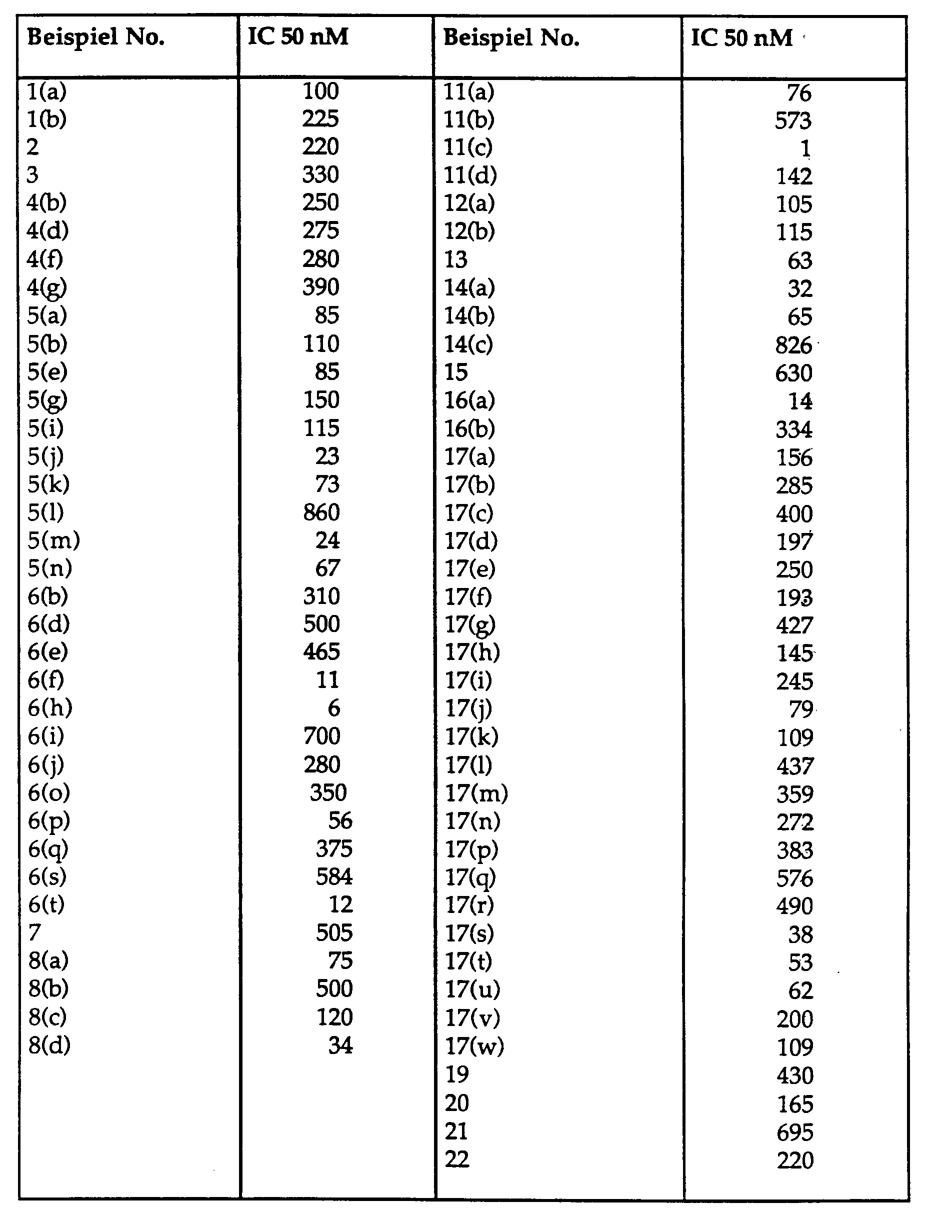

### D. Bestimmung der β-Lactamase hemmender Wirkung durch Kombination einer Verbindung der allgemeinen Formel I mit Ceftriaxon

Die Mindesthemmkonzentration in vitro (MHK in µg/ml) einer 1:4 Kombination von Ceftriaxon mit einer Verbindung der Formel I gegen Citrobacter freundii wird gemessen und in der nachstehenden Tabelle 2 zusammengestellt.

**Tabelle 2**

| **Beispiel No.** | **MHK µg/ml** | **Beispiel No.** | **MHK µg/ml** |
|---|---|---|---|
| 1(a) | 8 | 7 | 4 |
| 1(b) | 8 | 8(a) | 4 |
| 2 | 2 | 8(b) | 1 |
| 3 | 2 | 8(c) | 2 |
| 4(b) | 2 | 8(d) | 0,5 |
| 4(d) | 2 | 11(a) | 8 |
| 4(f) | 4 | 11(b) | 16 |
| 4(i) | 8 | 11(c) | 16 |
| 5(a) | 4 | (11(d) | 4 |
| 5(b) | 2 | 12(a) | 4 |
| 5(e) | 2 | 12(b) | 2 |
| 5(f) | 16 | 13 | 4 |
| 5(g) | 8 | 17(a) | 2 |
| 5(h) | 4 | 17(b) | 2 |
| 5(i) | 2 | 17(c) | 2 |
| 5(j) | 0,5 | 17(d) | 2 |
| 5(k) | 2 | 17(e) | 2 |
| 5(l) | 4 | 17(f) | 2 |
| 5(m) | 0,5 | 17(g) | 2 |
| 6(b) | 4 | 17(j) | 2 |
| 6(d) | 2 | 17(k) | 16 |
| 6(e) | 4 | 17(l) | 4 |
| 6(f) | 4 | 17(m) | 16 |
| 6(h) | 1 | 17(n) | 16 |
| 6(i) | 4 | 17(u) | 2 |
| 6(k) | 8 | 18 | 1 |
| 6(l) | 8 | 19 | 2 |
| 6(o) | 2 | 20 | 4 |
| 6(p) | 2 | 21 | 4 |
| 6(q) | 2 | | |
| 6(r) | 2 | Ceftriaxon allein | 128 |
| 6(s) | 4 | (Kontrolle) | |

Die erfindungsgemässen Produkte können als Heilmittel, z.B. in Form pharmazeutischer Präparate Verwendung finden, welche sie oder ihre Salze in Mischung mit einem für die parenterale oder enterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaestetica oder Puffer. Die Verbindungen der Formel Ia und ihre Salze kommen vorzugsweise für die parenterale Applikation in Betracht und werden zu diesem Zweck bevorzugt als Lyophilisate oder Trockenpulver zur Verdünnung mit üblichen Agenzien wie Wasser oder isotonische Kochsalzlösung, zubereitet. Die Verbindungen der Formel Ib und ihre Salze sowie auch die Verbindungen der Formel Ic, worin R^{o} die Gruppe c¹ darstellt, kommen für die parenterale Applikation sowie auch für die enterale Verabreichung in Betracht.

Wie eingangs erwähnt, kann man die Verbindungen der allgemeinen Formel I und ihre pharmazeutisch verträgliche Salze erfindungsgemäss bei der Bekämpfung bzw. Verhütung von Krankheiten verwenden, insbesondere bei der Bekämpfung von β-Lactamase bildenden Krankheitserregern in Kombination mit β-Lactam-Antibiotika, d.h. Antibiotika welche einen β-Lactamring enthalten, beispielsweise Penicillin, wie Benzylpenicillin, Piperacillin, Phenoxymethylpenicillin, Carbenicillin, Methicillin, Propicillin, Tricarcillin, Ampicillin, Amoxicillin oder Mecillinam, und Cephalosporine, wie Ceftriaxon, Ceftazidim, Cefetamet, Cefatamet Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, (6R,7R)-7-[(Z)-2-(2-Amino-4-thiazolyl)-2-(methoxyimino)-acetamido]-3-(azidomethyl)-8-oxo-5-thia-1-azabicyclo[4.2.0]oct-2-en-2-carbonsäure oder (E)-2-(Isobutoxycarbonyl)-2-pentenyl (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carboxylat. Dabei können die Verbindungen der allgemeinen Formel I oder pharmazeutisch verträgliche Salze davon mit Basen vor, gleichzeitig mit oder nach der Verabreichung bzw. Einnahme von β-Lactam-Antibiotika verabreicht werden. Werden die erfindungsgemässen Produkte gleichzeitig mit einem β-Lactam-Antibiotikum verabreicht, so kann dies durch Verabreichung als ad-hoc-Kombination oder in Form einer pharmazeutischen Kombination erfolgen, welche eine Verbindung der allgemeinen Formel I oder ein pharmazeutisch verträgliches Salz davon mit Base und ein β-Lactam-Antibiotikum enthält; derartige pharmazeutische Kombinationen sind ebenfalls Gegenstand der vorliegenden Erfindung.

Die Dosierung der Verbindungen der allgemeinen Formel I und der pharmazeutisch verträglichen Salze davon mit Basen kann innerhalb weiter Grenzen variieren und ist natürlich in jedem einzelnen Fall den individuellen Gegebenheiten und dem zu bekämpfenden β-Lactamase produzierenden Krankheitserreger anzupassen. Im allgemeinen dürfte eine Tagesdosis von etwa 0,1 bis etwa 2,0 g angemessen sein. Das Verhältnis von β-Lactamase-Inhibitor (Verbindung der Formel I oder pharmazeutisch veträgliches Salz davon mit Base) zu β-Lactam-Antibiotikum kann ebenfalls innerhalb weiter Grenzen variieren und ist in jedem einzelnen Fall den individuellen Gegebenheiten anzupassen. Im allgemeinen dürfte ein Verhältnis von etwa 1:20 bis etwa 1:1 angemessen sein.

Wie eingangs erwähnt, sind Arzneimittel enthaltend eine Verbindung der allgemeinen Formel I oder ein pharmazeutisch verträgliches Salz davon ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch verträgliche Salze davon und gegebenenfalls einen oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Insbesondere sind pharmazeutische Kombinationen enthaltend eine Verbindung der allgemeinen Formel I oder ein pharmazeutisch verträgliches Salz davon und ein β-Lactam-Antibiotikum, z.B. ein Penicillin, wie Benzylpenicillin, Piperacillin, Phenoxymethylpenicillin, Carbenicillin, Methicillin, Propicillin, Tricarcillin, Ampicillin, Amoxicillin oder Mecillinam, oder ein Cephalosporin, wie Ceftriaxon, Ceftazidim, Cefetamet, Cefatamet Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carbonsäure oder (E)-2-(Isobutoxycarbonyl)-2-pentenyl (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carboxylat, Gegenstand der vorliegenden Erfindung. Derartige Kombinationen eignen sich zur Bekämpfung von β-Lactamase bildenden Krankheitserregern.

In den nachfolgenden Beispielen, welche die vorliegende Erfindung näher erläutern, ihren Umfang jedoch in keiner Weise beschränken sollen, sind folgende Abkürzungen verwendet: DMF bedeutet Dimethylformamid, THF bedeutet Tetrahydrofuran, AIBN bedeutet α,α'-Azo-isobutyronitril, DCC bedeutet Dicyclohexylcarbodiimid, HOBT bedeutet 1-Hydroxybenzotriazol und DMSO bedeutet Dimethylsulfoxid.

### Beispiel 1

### (a) Benzyl (1S,5R)-7-oxo-6-sulfo-2,6-diazabicyclo[3.2.0]heptan-2-carboxylat-Natriumsalz

In einer Mischung aus 5 ml Methylenchlorid und 2,5 ml DMF werden 343 mg (1,39 mmol) Benzyl (1S,5R)-7-oxo-2,6-diazabicyclo[3.2.0]heptan-2-carboxylat gelöst und unter Eiskühlung tropfenweise mit 341 mg (2,22 mmol) DMF-SO₃ Komplex, gelöst in 1 ml DMF versetzt. Nach 1,5 Stunden wird unter Kühlung mit gesättigter, wässriger Natriumhydrogencarbonatlösung auf pH 7 eingestellt. Man versetzt mit etwas Wasser, trennt die Phasen und extrahiert die wässrige Phase zweimal mit ca. 5 ml Methylenchlorid. Die wässrige Phase wird durch Eindampfen konzentriert und über ein polymeres, hydrophobes Gel (75-150 µ) mit Wasser als Eluens fraktioniert. Die Fraktionen, die das Produkt enthalten, werden vereinigt und lyophilisiert.
Ausbeute: 330 mg (68%) farbloses Pulver
IR (KBr): 1759, 1707 cm⁻¹

| Elementaranalyse: C₁₃H₁₃N₂O₆SNa | | | | |
|---|---|---|---|---|
| ber. | C 44,83 | H 3,76 | N 8,04 | S 9,20 |
| gef. | C 44,52 | H 3,85 | N 7,94 | S 8,95 |

Das als Ausgangsverbindung eingesetzte Benzyl (1S,5R)-7-oxo-2,6-diazabicyclo[3.2.0]heptan-2-carboxylat kann wie folgt hergestellt werden:

### Benzyl (2S,3S)-1-(3,4-dimethoxybenzyl)-2-[(R)-1-hydroxy-2-[(methylsulfonyl)-oxy]äthyl]-4-oxo-3-azetidincarbamat

Das Reaktionsgemisch aus 215 g (0,5 mol) Benzyl (2S,3S)-1-(3,4-dimethoxybenzyl)-2-[1(R),2-dihydroxyäthyl]-4-oxo-3-azetidincarbamat (europäische Offenlegungsschrift Nr. 73061) und 84 ml (0,6 mol) Triäthylamin in 5,2 l THF wird auf 40-45°C erwärmt bis sich alles Diol gelöst hat. Hierzu tropft man innerhalb 75 Minuten 47 ml (0,6 mol) Mesylchlorid und lässt 30 Minuten weiterrühren, bevor man das warme, leicht trübe Reaktionsgemisch filtriert. Aus dem Filtrat kristallisiert über Nacht im Kühlschrank das Produkt, das abgenutscht und mit 3x100 ml THF nachgewaschen wird. Nach Einengen der Mutterlauge erhält man nochmals ca. 10% an Produkt.
Ausbeute: 244 g (96%)
Smp.: 158°C (Aethanol/Wasser)

| Elementaranalyse: C₂₃H₂₈N₂O₉S | | | | |
|---|---|---|---|---|
| ber. | C 54,32 | H 5,55 | N 5,51 | S 6,30 |
| gef. | C 54,32 | H 5,34 | N 5,57 | S 6,35 |

### Benzyl (2S,3S)-1-(3,4-dimethoxybenzyl)-4-oxo-2-[(R)-2-[(methylsulfonyl)oxy]-1-[[(R,S)-tetrahydro-2H-pyran-2-yl]oxy]äthyl]-3-azetidincarbamat

Zu einer Lösung aus 244 g (0,48 mol) Benzyl (2S,3S)-1-(3,4-dimethoxybenzyl)-2-[(R)-1-hydroxy-2-[(methylsulfonyl)oxy]äthyl]-4-oxo-3-azetidincarbamat und 48 g (0,19 mol) Pyridinium-p-toluolsulfonat in 5,5 l Methylenchlorid werden bei 12°C 53 ml (0,58 mol) Dihydropyran, gelöst in 150 ml Methylenchlorid, zugetropft. Man rührt über Nacht bei Raumtemperatur, extrahiert danach mit 2x1000 ml Wasser, trocknet die organische Phase über Magnesiumsulfat und engt ein. Zurück bleibt ein gelbliches Oel.
Ausbeute: 267 g (94%)
IR (Film): 1760, 721 cm⁻¹
MS: (M+H-DHP)⁺ 509

### Benzyl (1S,4S,5S)-6-(3,4-dimethoxybenzyl)-7-oxo-4[[(R,S)-tetrahydro-2H-pyran-2-yl]oxy]-2,6-diazabicyclo[3.2.0]heptan-2-carboxylat

Eine Lösung aus 267 g (0,45 mol) Benzyl (2S,3S)-1-(3,4-dimethoxybenzyl)-4-oxo-2-[(R)-2-[(methylsulfonyl)oxy]-1-[[(R,S)-tetrahydro-2H-pyran-2-yl]oxy]-äthyl]-3-azetidincarbamat in 5 l THF wird auf 12°C gekühlt und portionsweise mit 18 g (0,45 mol) Natriumhydrid (60%ige Suspension in Oel) versetzt. Man lässt bei Raumtemperatur für 3 Stunden weiterrühren und versetzt anschliessend mit 2 l Wasser und 5 l Aether. Nach Trennung der Phasen extrahiert man die wässrige Phase einmal mit 500 ml Aether, wäscht die vereinigten organischen Phasen einmal mit 1,5 l gesättigter, wässriger Natriumchloridlösung und trocknet sie über Magnesiumsulfat. Nach Einengen erhält man 225 g eines gelben trüben Oels, das noch ca. 7 g Mineralöl aus der Natriumhydridsuspension enthält. Es kann ohne weitere Reinigung weiterverarbeitet werden.
Ausbeute: 218 g (98%)
IR (Film): 1772, 1708 cm⁻¹
MS: (M+H)⁺ 497

### Benzyl (1S,4S,5S)-6-(3,4-dimethoxybenzyl)-4-hydroxy-7-oxo-2,6-diazabicyclo[3.2.0]heptan-2-carboxylat

Das noch mit Mineralöl verunreinigte Benzyl (1S,4S,5S)-6-(3,4-dimethoxybenzyl)-7-oxo-4-[[(R,S)-tetrahydro-2H-pyran-2-yl]oxy]-2,6-diazabicyclo[3.2.0]heptan-2-carboxylat (218 g, 0,44 mol) wird in 5,5 l Methanol gelöst, mit 14 g (56 mmol) Pyridinium-p-toluolsulfonat versetzt und über Nacht bei 53°C gerührt. Danach engt man die Reaktionslösung ein und chromatographiert mit Aethylacetat über 1150 g Kieselgel (0,065-0,2 mm Korngrösse). Das so gewonnene reine Oel kann aus Isopropanol kristallisiert oder direkt weiterverarbeitet werden.
Ausbeute: 166 g (92%)
Smp.: 100-102°C (Isopropanol)

| Elementaranalyse: C₂₂H₂₄N₂O₆ | | | |
|---|---|---|---|
| ber. | C 64,07 | H 5,87 | N 6,79 |
| gef. | C 64,00 | H 5,80 | N 6,80 |

### Benzyl (1S,4S,5S)-6-(3,4-dimethoxybenzyl)-7-oxo-4-[[(1-imidazolyl)thiocarbonyl]oxy]-2,6-diazabicyclo[3.2.0]heptan-2-carboxylat

In 1,3 l THF werden 166 g (0,4 mol) Benzyl (1S,4S,5S)-6-(3,4-dimethoxybenzyl)-4-hydroxy-7-oxo-2,6-diazabicyclo[3.2.0]heptan-1-carboxylat und 144 g (0,8 mol) 1,1'-Thiocarbonyldiimidazol gelöst und über Nacht unter Rückfluss zum Sieden erhitzt. Anschliessend entfernt man das Lösungsmittel unter vermindertem Druck und trennt das dunkelbraune Oel durch Chromatographie mit Aethylacetat: n-Hexan 2:1 über 1200 g Kieselgel (0,063-0,2 mm Korngrösse).
Ausbeute: 200 g (95%) orangefarbenes Oel
IR (Film): 1766, 1709 cm⁻¹
MS (M+H+1-Thioglycerin)⁺: 631

### Benzyl (1S,5R)-6-(3,4-dimethoxybenzyl)-7-oxo-2,6-diazabicyclo[3.2.0]heptan-2-carboxylat

Es werden 200 g (0,38 mol) des Benzyl (1S,4S,.5S)-6-(3,4-dimethoxybenzyl)-7-oxo-4-[[(1-imidazolyl)thiocarbonyl]oxy]-2,6-diazabicyclo[3.2.0]heptan-2-carboxylat in 4,2 l Toluol auf 80°C erhitzt vorgelegt. Unter Rühren tropft man hierzu eine Lösung von 213 ml (0,8 mol) Tributylzinnhydrid und 6,9 g (0,04 mol) AIBN in 320 ml Toluol. Nach Beendigung des Zutropfens rührt man noch weitere 45 Minuten bei dieser Temperatur, bevor man das Lösungsmittel unter vermindertem Druck entfernt. Es verbleibt ein gelblich trübes Oel, das über 1100 g Kieselgel (0,063-0,2 mm Korngrösse) mit 1) Aethylacetat:Hexan 1:10; 2) Aethylacetat:n-Hexan 2:1 chromatographiert wird.
Ausbeute: 135 g (90%) farbloses Oel
IR (Film): 1755, 1705 cm⁻¹
MS (EI): (M-Dimethoxybenzylisocyanat) 203

### Benzyl (1S,5R)-7-oxo-2,6-diazabicyclo[3.2.0]heptan-2-carboxylat

In 1,5 l Acetonitril werden 135 g (0,34 mol) Benzyl (1S,5R)-6-(3,4-dimethoxybenzyl)-7-oxo-2,6-diazabicyclo[3.2.0]heptan-2-carboxylat vorgelegt. Hierzu gibt man eine Lösung von 77 g (0,44 mol) Dikaliumhydrogenphosphat und 128 g (0,47 mol) Kaliumperoxodisulfat in 660 ml Wasser und erhitzt das ganze auf 100°C. Die Reaktionszeit beträgt 2,5 Stunden. Während dieser Zeit wird der pH-Wert des Gemisches saurer. Mit portionsweiser Zugabe von weiterem Dikaliumhydrogenphosphat wird dieser bei ca. pH 5 gehalten. Nach Beendigung der Reaktion entfernt man das Acetonitril an einem Rotationsverdampfer und extrahiert den wässrigen Rückstand mit 3x250 ml Methylenchlorid. Die vereinigten organischen Phasen werden je einmal mit 500 ml 3%iger Natriumhydrogencarbonatlösung und 750 ml gesättigter, wässriger Natriumchloridlösung gewaschen und abschliessend über Magnesiumsulfat getrocknet. Nach dem Einengen erhält man ein braunes Oel, das chromatographisch gereinigt wird [Kieselgel, 0,063-0,2 mm Korngrösse, Elution mit 1) Aethylacetat:n-Hexan=2:1; 2) Aethylacetat].
Ausbeute: 37 g (45%)
IR (KBr): 1755, 1689 cm⁻¹
MS (EI): (M-CONH) 203
In Analogie zu Beispiel 1 wird hergestellt:

### (b) t-Butoxy-(1S,5R)-7-oxo-6-sulfo-2,6-diazabicyclo[3.2.0]heptan-2-carboxylat-Natriumsalz

IR (KBr): 1758, 1690 cm⁻¹
MS (M⁻): 291

### Beispiel 2

### (1S,5R)-7-Oxo-2-(phenylcarbamoyl)-2,6-diazabicyclo[3.2.0]heptan-6-sulfonsäure-Natriumsalz

wird in der gleichen Weise, wie in Beispiel 1 angegeben, hergestellt.
IR (KBr): 1751, 1657 cm⁻¹
MS: (M-Na)⁻ 310
Das hierfür als Ausgangsverbindung verwendete (1S,5R)-7-Oxo-2-(phenylcarbamoyl)-2,6-diazabicyclo[3.2.0]heptan kann wie folgt hergestellt werden:

### (1S,5R)-2,6-Diazabicyclo[3.2.0]heptan-7-on

In 600 ml Aethanol löst man 34 g (0,14 mol) Benzyl (1S,5R)-6-(3,4-dimethoxybenzyl)-7-oxo-2,6-diazabicyclo[3.2.0]heptan-2-carboxylat, gibt 34 g 10%igem Pd/C und 130 ml 1,4-Cyclohexadien hinzu und rührt diese Suspension unter Argon. Nach ca. 30 Minuten beginnt die exotherme Reaktion, die sehr heftig werden kann. Gegebenenfalls sollte man auf 50°C abkühlen. Nach etwa einer weiteren Stunde ist die Reaktion vollständig verlaufen, man filtriert die Suspension über einen Faltenfilter und engt das Filtrat ein. Der Rückstand wird aus Aethanol umkristallisiert.
Ausbeute: 13,6 g (88%) farblose Kristalle, die an der Luft schnell bräunlich werden
Smp.: 148-150°C (Aethanol)

| Elementaranalyse: C₅H₈N₂O | | | |
|---|---|---|---|
| ber. | C 53,56 | H 7,19 | N 24,98 |
| gef. | C 53,27 | H 7,28 | N 24,67 |

### (1S,5R)-7-Oxo-2-(phenylcarbamoyl)-2,6-diazabicyclo[3.2.0]heptan

In 5 ml Chloroform werden 100 mg (0,89 mmol) (1S,5R)-2,6-Diazabicyclo[3.2.0]heptan-7-on gelöst und unter Rückfluss zum Sieden erhitzt. Man fügt Phenylisocyanat (0,1 ml, 0,89 mmol) und 1 Tropfen Triäthylamin hinzu und lässt bei Siedehitze für 1 Stunde reagieren. Nach dem Einengen wird mit Aethylacetat:Aethanol=3:2 über Kieselgel (0,063-0,2 mm Korngrösse) chromatographiert.
Ausbeute: 134 mg (65%)
¹H-NMR (250 MHz, CDCl₃): δ [ppm] = 1,68-1,86 (4-H, m, 1H); 1,98 (4-H, dd, 1H, J=6 Hz, 14 Hz); 3,42 (3-H, m, 1H); 4,18 (3-H, dd, 1H, J=8Hz); 4,30 (5-H, t, 1H, J=4Hz); 5,37 (1-H, t, 1H, J=4Hz); 6,97 (arom., 1H); 7,23 (arom., 2H); 7,46 (arom., 2H); 7,76 (NH); 8,08 (NH)

### Beispiel 3

### (1S,5R)-2-(Benzylcarbamoyl)-7-oxo-2,6-diazabicyclo[3.2.0]heptan-6-sulfonsäure-Natriumsalz

wird in der gleichen Weise, wie in Beispiel 2 angegeben, hergestellt.
IR (KBr): 1746, 1636 cm⁻¹

| Elementaranalyse: C₁₃H₁₄N₃O₅SNa | | | | |
|---|---|---|---|---|
| ber. | C 44,96 | H 4,06 | N 12,10 | S 9,23 |
| gef. | C 45,45 | H 4,12 | N 12,27 | S 9,07 |

Das hierfür als Ausgangsverbindung eingesetzte (1S,5R)-2-(Benzylcarbamoyl)-7-oxo-2,6-diazabicyclo[3.2.0]heptan wird analog Beispiel 2 hergestellt:
¹H-NMR (250 MHz, CDCl₃): δ [ppm] = 1.68-1.86 (4-H, m, 1H); 1.95 (4-H, dd, 1H, J= 6 Hz, 14 Hz); 3.40 (3-H, m, 1H); 4.08 (3-H, dd br., 1H, J= 8 Hz); 4.31 (5-H, t, 1H, J= 4 Hz); 4.42 (PhCH₂, dd, 2H, J= 6 Hz, 8 Hz); 4.97 (1-H, t, 1H, J= 4 Hz); 5.24 (NH, t br.); 6.34 (NH, s br.); 7.3 (arom., 5H, m)

### Beispiel 4

### (a) Benzyl [(R)-α-[[(1S,5R)-7-oxo-6-sulfo-2,6-diazabicyclo[3.2.0]hept-2-yl]carbonyl]benzyl]carbamat-Natriumsalz

Gemäss Beispiel 1 werden 1,80 g (4,74 mmol) Benzyl [(R)-α-[[(1S,5R)-7-oxo-2,6-diazabicyclo[3.2.0]hept-2-yl]carbonyl]benzyl]carbamat sulfoniert.
Ausbeute: 1,03 g (45%) farbloses Pulver
IR (KBr): 1760, 1717, 1652 cm⁻¹

| Elementaranalyse: C₂₁H₂₀N₃O₇SNa | | | |
|---|---|---|---|
| ber. | C 52,39 | H 4,19 | N 8,73 |
| gef. | C 52,53 | H 4,16 | N 8,74 |

### (b) (1S,5R)-7-Oxo-2-(D-2-phenylglycyl)-2,6-diazablcyclo[3.2.0]heptan-6-sulfonsäure

400 mg (0.80 mmol) Benzyl[(R)-α-[[(1S,5R)-7-oxo-6-sulfo-2,6-diazabicyclo[3.2.0]hept-2-yl]carbonyl]benzyl]carbamat-Natriumsalz werden in 8 ml Wasser gelöst, mit 0,47 ml Eisessig versetzt und über 40 mg 10%iger Pd/C bei 25-30°C 6,5 Stunden hydriert. Man filtriert und fraktioniert mit 1) Wasser und 2) Wasser:Methanol 4:1 über ein polymeres, hydrophobes Gel. Die das Produkt enthaltenden Fraktionen werden vereinigt und lyophilisiert.
Ausbeute: 270 mg (80%) farbloses Pulver
IR (KBr): 1765, 1661 cm⁻¹

| Elementaranalyse: C₁₃H₁₅N₃O₅S | | | | |
|---|---|---|---|---|
| ber. | C 47,99 | H 4,65 | N 12,91 | S 9,85 |
| gef. | C 48,15 | H 4,50 | N 12,88 | S 9,50 |

Das oben eingesetzte Benzyl [(R)-α-[[(1S,5R)-7-oxo-2,6-diazabicyclo[3.2.0]hept-2-yl]carbonyl]benzyl]carbamat kann wie folgt hergestellt werden:

### (1S,5R)-6-(3,4-Dimethoxybenzyl)-7-oxo-2,6-diazabicyclo[3.2.0]heptan

Diese Verbindung wird analog Beispiel 2 aus Benzyl (1S,5R)-6-(3,4-dimethoxybenzyl)-7-oxo-2,6-diazabicyclo[3.2.0]heptan-2-carboxylat durch Behandeln mit Pd/C und Cyclohexadien hergestellt.
IR (Film): 1731 cm⁻¹
MS (M-DMB)⁺ 111

### Benzyl [(R)-α-[[(1S,5R)-7-oxo-2,6-diazabicyclo[3.2.0]hept-2-yl]carbonyl]benzyl]-carbamat

In 45 ml Methylenchlorid löst man 2,00 g (7,62 mmol) (1S,5R)-6-(3,4-Dimethoxybenzyl)-7-oxo-2,6-diazabicyclo[3.2.0]heptan und 2,1 ml (15,25 mmol) Triäthylamin. Unter Eiskühlung fügt man nacheinander 2,61 g (9,15 mmol) N-Benzyloxycarbonyl-D-Phenylglycin, 2,05 g (9,91 mmol) DCC und 1,67 g (9,91 mmol) HOBT hinzu, rührt für 1 Stunde bei Eiskühlung und über Nacht bei Raumtemperatur. Zur Aufarbeitung wird die entstandene Suspension mit nochmals 100 ml Methylenchlorid in Lösung gebracht, anschliessend extrahiert man mit je einmal 60 ml 2%iger Zitronensäurelösung, 50 ml gesättigter, wässriger Natriumhydrogencarbonatlösung, 100 ml Wasser und 100 ml gesättigter, wässriger Natriumchloridlösung. Es wird über Magnesiumsulfat getrocknet und eingeengt. Mit Aethylacetat auf 120 g Kieselgel (Korngrösse 0,063-0,2 mm) wird der ölige Rest chromatographiert. Daraus erhält man 3,24 g (80%) Benzyl [(R)-α-[[(1S,5R)-6-(3,4-dimethoxybenzyl)-7-oxo-2,6-diazabicyclo[3.2.0]hept-2-yl]carbonyl]benzyl]carbamat. Analog Beispiel 1 entfernt man die Dimethoxybenzylschutzgruppe durch Behandeln mit Kaliumperoxodisulfat und erhält 1,80 g (87,6%) des Produkts.
IR (Kbr): 1768, 1717, 1648 cm⁻¹
MS: (M+Na)⁺ 402
In Analogie hierzu werden hergestellt:

### (c) Benzyl [(S)-α-[[(1S,5R)-7-oxo-6-sulfo-2,6-diazabicyclo[3.2.0]hept-2-yl]carbonyl]benzyl]carbamat-Natriumsalz

IR (KBr): 1760, 1716 cm⁻¹

| Elementaranalyse: C₂₁H₂₀N₃O₇SNa | | | | |
|---|---|---|---|---|
| ber. | C 52,39 | H 4,19 | N 8,73 | S 6,66 |
| gef. | C 52,72 | H 4,28 | N 8,76 | S 6,42 |

### (d) (1S,5R)-7-Oxo-2-(L-2-phenylglycyl)-2,6-diazabicyclo[3.2.0]heptan-6-sulfonsäure

IR (KBr): 1766, 1660 cm⁻¹
MS: (M-Na)⁻: 324

### (e) (1S,5R)-2-[N-[(Benzyloxy)carbonyl]-3-phenyl-D-alanyl]-7-oxo-2,6-diazabicyclo[3.2.0]heptan-6-sulfonsäure-Natriumsalz

IR (KBr): 1759, 1717, 1650 cm⁻¹

| Elementaranalyse: C₂₂H₂₂N₃O₇SNa | | | | |
|---|---|---|---|---|
| ber. | C 53,33 | H 4,48 | N 8,48 | S 6,47 |
| gef. | C 53,82 | H 4,54 | N 8,59 | S 6,09 |

### (f) (1S,5R)-7-Oxo-2-(3-phenyl-D-alanyl)-2,6-diazabicyclo[3.2.0]heptan-6-sulfonsäure

IR (KBr): 1765, 1658 cm⁻¹
MS: (M+H)⁺: 340

### (g) Benzyl [[[(1S,5R)-7-oxo-6-sulfo-2,6-diazabicyclo[3.2.0]hept-2-yl]carbonyl]-methyl]carbamat

IR (KBr): 1758, 1717, 1653 cm⁻¹
MS: (M-Na)⁻ 382

### Beispiel 5

### (a) (1S,5R)-2-[(E)-3-(2-Furyl)acryloyl]-7-oxo-2,6-diazabicyclo[3.2.0]heptan-6-sulfonsäure-Natriumsalz

wird durch Sulfonierung in der gleichen Weise, wie in Beispiel 1 angegeben, hergestellt.
IR (KBr): 1753, 1650, 1605 cm⁻¹

| Elementaranalyse: C₁₂H₁₁N₂O₆Na | | | | |
|---|---|---|---|---|
| ber. | C 43,12 | H 3,22 | N 8,38 | S 9,59 |
| gef. | C 43,39 | H 3,13 | N 8,46 | S 9,44 |

Das hierfür verwendete (1S,5R)-2-[(E)-3-(2-Furyl)acryloyl]-2,6-diazabicyclo[3.2.0]heptan-7-on kann wie folgt hergestellt werden:
In 50 ml Dichlormethan werden 500 mg (4,46 mmol) (1S,5R)-2,6-Diazabicyclo[3.2.0]heptan-7-on und 1,4 ml (9,82 mmol) Triäthylamin gelöst und unter Argon mit Eis gekühlt. Hierzu gibt man nacheinander 678 mg (4,91 mmol) 3-(2-Furyl)acrylsäure, 1,20 g (5,80 mmol) DCC und 980 mg (5,80 mmol) HOBT, lässt 1 Stunde bei Eiskühlung und über Nacht bei Raumtemperatur reagieren. Danach wird das Gemisch mit je einmal 2%iger Zitronensäurelösung, gesättigter Natrium-hydrogencarbonatlösung, Wasser und gesättigter Natriumchloridlösung extrahiert. Die organische Phase wird über Magnesiumsulfat getrocknet und eingeengt. Der ölige Rest wird mit Aethylacetat:Aethanol = 3:2 über Kieselgel (Merck, 0,063-0,2 mm Korngrösse) chromatographiert.
Ausbeute: 450 mg (43%)
¹H-NMR (250 MHz, CDCl₃): (Rotamere im Verhältnis 2:1) δ [ppm] = 1,02-1,46 (4-H,m,br.,1H); 2,05 (4-H,m,br.,1H); 3,49 und 3,68 (3-H,m,br.,1H); 4,13-4,52 (3-H,7-H,m br.,2H); 5,24 und 6,71 (1-H,s br.,1H); 6,46 (Furan-β-H,m,2H); 6,59 (Furan-α-H,d,1H,J=4Hz); 6,75 (=CH, d,1H,J=16Hz); 7,46 (NH, s,1H); 7,50 (=CH, d,1H,J=16Hz).

In Analogie zu Beispiel 5(a) werden hergestellt:

### (b) (1S,5R)-7-Oxo-2-(N-phenylglycyl)-2,6-diazabicyclo[3.2.0]heptan-6-sulfonsäure-Natriumsalz

IR (KBr): 1756, 1650 cm⁻¹

| Elementaranalyse: C₁₃H₁₄N₃O₅SNa | | | | |
|---|---|---|---|---|
| ber. | C 44,96 | H 4,06 | N 12,10 | S 9,23 |
| gef. | C 45,20 | H 3,99 | N 12,15 | S 9,01 |

### (c) Benzyl [(RS)-α-[[(1S,5R)-7-oxo-6-sulfo-2,6-diazabicyclo[3.2.0]hept-2-yl]-carbonyl]-2-thienyl]carbamat-Natriumsalz

IR (KBr): 1760, 1716, 1655 cm⁻¹

| Elementaranalyse: C₁₉H₁₈N₃O₇S₂Na | | | | |
|---|---|---|---|---|
| ber. | C 46,81 | H 3,72 | N 8,62 | S 13,15 |
| gef. | C 46,73 | H 3,62 | N 8,63 | S 13,00 |

### (d) t-Butyl [(R oder S)-α-[[(1S,5R)-7-oxo-6-sulfo-2,6-diazabicyclo[3.2.0]hept-2-yl]-carbonyl]-2-thienyl]carbamat-Natriumsalz

IR (KBr): 1762, 1709, 1656 cm⁻¹

| Elementaranalyse: C₁₆H₂₀N₃O₇S₂Na | | | | |
|---|---|---|---|---|
| ber. | C 42,38 | H 4,45 | N 9,27 | S 14,14 |
| gef. | C 42,17 | H 4,60 | N 9,29 | S 13,95 |

### (e) (1S,5R)-2-[(R oder S)-α-Amino-(2-thienyl)acetyl]-7-oxo-2,6-diazabicyclo[3.2.0]heptan-6-sulfonsäure

IR (KBr): 1767, 1664 cm⁻¹

| Elementaranalyse: C₁₁H₁₃N₃O₅S₂ | | | | |
|---|---|---|---|---|
| ber. | C 44,96 | H 4,06 | N 12,10 | S 9,23 |
| gef. | C 45,20 | H 3,99 | N 12,15 | S 9,01 |

### (f) (1S,5R)-2-[(Z)-3-α-Acetamidocinnamoyl]-7-oxo-2,6-diazabicyclo[3.2.0]heptan-6-sulfonsäure-Natriumsalz

IR (KBr): 1759, 1628 cm⁻¹

| Elementaranalyse: C₁₆H₁₆N₃O₆SNa | | | | |
|---|---|---|---|---|
| ber. | C 47,88 | H 4,02 | N 10,47 | S 7,99 |
| gef. | C 47,01 | H 4,13 | N 10,42 | S 7,73 |

### (g) (1S,5R)-2-[D- oder L-N-Acetyl-2-phenylglycyl]-7-oxo-2,6-diazabicyclo[3.2.0]heptan-6-sulfonsäure-Natriumsalz

IR (KBr): 1761, 1648 cm⁻¹

| Elementaranalyse: C₁₅H₁₆N₃O₆SNa | | | | |
|---|---|---|---|---|
| ber. | C 46,27 | H 4,14 | N 10,79 | S 8,23 |
| gef. | C 45,50 | H 4,00 | N 10,55 | S 8,03 |

### (h) (1S,5R)-2-[(R,S)-2-Benzamido-4-(methylthio)butyryl]-7-oxo-2,6-diazabicyclo[3.2.0]heptan-6-sulfonsäure-Natriumsalz

IR (KBr): 1760, 1641 cm⁻¹

| Elementaranalyse: C₁₇H₂₀N₃O₆S₂Na | | | | |
|---|---|---|---|---|
| ber. | C 45,43 | H 4,49 | N 9,35 | S 14,27 |
| gef. | C 45,66 | H 4,82 | N 9,37 | S 14,13 |

### (i) (1S,5R)-2-[N-(m-Aminophenyl)glycyl]-7-oxo-2,6-diazabicyclo[3.2.0]heptan-6-sulfonsäure

¹H-NMR (250 MHz, DMSO-d₆): (Rotamere im Verhältnis von 3:2) δ [ppm]: 1,72 (4-H,m,1H); 2,34 (4-H,m,1H); 3,14 (3-H,m,1H); 3,86 (3-H,m,1H); 4,02 (Ar-CH₂, m,2H); 4,34 und 4,52 (5-H,t,1H,J=4Hz); 5,22 und 5,26 (1-H,d,1H, J=4Hz); 6,00 (3 arom. H); 6,62 (1 arom. H).

### (j) (1S,5R)-2-[[(1-Methy-1H-tetrazol-5-yl)thio]acetyl]-7-oxo-2,6-diazabicyclo[3.2.0]heptan-6-sulfonsäure-Natriumsalz

IR (KBr): 1758, 1646 cm⁻¹
MS (M-Na)⁻: 347

### (k) (1S,5R)-2-[[1H-benzotriazol-1-yl)oxy]acetyl]-7-oxo-2,6-diazabicyclo[3.2.0]heptan-6-sulfonsäure-Natriumsalz

IR (KBr): 1760, 1663 cm⁻¹

| Elementaranalyse: C₁₃H₁₂N₅O₆SNa | | | |
|---|---|---|---|
| ber. | C 40,11 | H 3,11 | N 17,99 |
| gef. | C 40,29 | H 3,12 | N 17,62 |

### (l) (1S,5R)-2-[(2-Isopropyl-2H-tetrazol-5-yl)-acetyl]-7-oxo-2,6-diazabicyclo[3.2.0]heptan-6-sulfonsäure-Natriumsalz

IR(KBr): 1760, 1652 cm⁻¹
MS (ISN): 342,8 (M-Na)⁻

### (m) (1S,5R)-2-[[(5-Methyl-1,3,4-thiadiazol-2-yl)thio]acetyl]-7-oxo-2,6-diazabicyclo[3.2.0]heptan-6-sulfonsäure-Natriumsalz

IR (KBr): 1757, 1642 cm⁻¹

| Elementaranalyse: C₁₀H₁₁N₄O₅S₃Na | | | |
|---|---|---|---|
| ber. | C 31,09 | H 2,87 | N 14,50 |
| gef. | C 30,93 | H 3,11 | N 14,62 |

### (n) (1S,5R)-2-[N-[2-Oxo-4-(trifluormethyl)-2H-1-benzopyran-7-yl]glycyl]-7-oxo-2,6-diazabicyclo[3.2.0]heptan-6-sulfonsäur-Natriumsalz

IR (KBr): 1748, 1642 cm⁻¹

| Elementaranalyse: C₁₇H₁₃F₃N₃O₇SNa | | | | |
|---|---|---|---|---|
| ber. | C 42,24 | H 2,71 | N 8,69 | S 6,63 |
| gef. | C 42,61 | H 2,66 | N 8,74 | S 6,75 |

### (o) [(1S,5R)-2-[[N-[(Benzyloxy)carbonyl]propionyl]-7-oxo-6-sulfo-2,6-diazabicyclo[3.2.0]heptan-2-yl]äthyl]carbamat-Natriumsalz.

IR (KBr): 1758, 1712, 1637 cm⁻¹
MS (M+H)⁺: 420.

### Beispiel 6

### (a) t-Butyl [(S)-p-hydroxy-α-[[(1S,5R)-7-oxo-6-sulfo-2,6-diazabicyclo[3.2.0]hept-2-yl]carbonyl]phenäthyl]carbamat-Triäthylaminsalz

Eine Lösung von (1S,5R)-7-Oxo-2,6-diazabicyclo[3.2.0]heptan-6-sulfonsäure-Trifluoracetat (360 mg, 1,17 mmol), gelöst in abs. DMF (30 ml) wird mit t-Butylcarbonyl-L-tyrosin-hydroxysuccinimid (440 mg, 1,16 mmol) und Triäthylamin (120 mg, 1,2 mmol) versetzt. Nach 4-tägigem Rühren wird das DMF eingeengt. Der Rückstand wird über ein polymeres, hydrophobes Gel chromatographiert (Elutionsmittel Wasser, dann Wasser:Methanol 4:1). Die das Produkt enthaltenden Fraktionen werden vereinigt und lyophilisiert.
Ausbeute: 250 mg (38%)
IR (KBr): 1771, 1709, 1650 cm⁻¹

| Elementaranalyse: C₂₅H₄₀N₄O₈S | | | |
|---|---|---|---|
| ber. | C 53,94 | H 7,24 | N 10,06 |
| gef. | C 54,05 | H 7,18 | N 10,16 |

### (b) (1S,5R)-7-Oxo-2-L-tyrosyl-2,6-diazabicyclo[3.2.0]heptan-6-sulfonsäure

t-Butyl [(S)-p-hydroxy-α-[[(1S,5R)-7-oxo-6-sulfo-2,6-diazabicyclo[3.2.0]hept-2-yl]carbonyl]phenäthyl]carbamat-Triäthylaminsalz (180 mg, 0,32 mmol), auf -10°C vorgekühlt, wird mit Trifluoressigsäure (4 ml) versetzt. Nach 45 Minuten wird der Ueberschuss an Trifluoressigsäure unter vermindertem Druck entfernt. Der Rückstand wird über ein polymeres, hydrophobes Gel (75-150 µ) chromatographiert (Elutionsmittel Wasser, dann Wasser:Methanol 10:1). Die das Produkt enthaltenen Fraktionen werden vereinigt und lyophilisiert.
Ausbeute: 80 mg (70%)
IR (KBr): 1756, 1657, 1613 cm⁻¹

| Elementaranalyse: C₁₄H₁₇N₃O₆S | | | |
|---|---|---|---|
| ber. | C 47,32 | H 4,82 | N 11,82 |
| gef. | C 46,58 | H 4,94 | N 11,52 |

Das als Ausgangsverbindung verwendete (1S,5R)-7-Oxo-2,6-diazabicyclo[3.2.0]heptan-6-sulfonsäure-Trifluoracetat kann wie folgt hergestellt werden:
t-Butoxy (1S,5R)-7-oxo-6-sulfo-2,6-diazabicyclo[3.2.0]heptan-2-carboxylat-Natriumsalz (1,1 g, 3,5 mmol), auf -10°C vorgekühlt, wird mit Trifluoressigsäure (10 ml) versetzt. Nach einer Stunde wird das Reaktionsgemisch mit Aether verdünnt. Die erhaltenen Kristalle werden abgenutscht und mit Aether gewaschen.
Ausbeute: 1 g (93%).
IR (KBr): 1752 cm⁻¹
MS: (M+Na) 237
In Analogie zu Beispiel 6 wird hergestellt:

### (c) t-Butyl [(R)-p-hydroxy-α-[[(1S,5R)-7-oxo-6-sulfo-2,6-diazabicyclo[3.2.0]hept-2-yl]carbonyl]benzyl]carbamat-Triäthylaminsalz (1:1)

IR (KBr): 1770, 1708, 1651 cm⁻¹
MS: (M-TEA) 439

### (d) (1S,5R)-2-[D-2-(p-Hydroxyphenyl)glycyl]-7-oxo-2,6-diazabicyclo[3.2.0]heptan-6-sulfonsäure

IR (KBr): 3418, 1766, 1660 cm⁻¹
MS: (M-H) 340

### (e) (R/S)-α-(1S,5R)-2-[2-carboxy-2-(3-thienyl)acetyl]-7-oxo-2,6-diazabicyclo[3.2.0]heptan-6-sulfonsäure-Triäthylaminsalz (1:1)

IR (KBr): 3437, 1768, 1647 cm⁻¹

### (f) (1S,5R)-2-[(E)-3-(3-Indolyl)acryloyl]-7-oxo-2,6-diazabicyclo[3.2.0]heptan-6-sulfonsäure-Triäthylaminsalz (1:1)

IR: 3418, 1764, 1640 cm⁻¹
MS: (M-TEA) 360

### (g) t-Butyl [(S)-2-indol-3-yl-1-[[(1S,5R)-7-oxo-6-sulfo-2,6-diazabicyclo[3.2.0]hept-2-yl]carbonyl]äthyl]carbamat-Triäthylaminsalz (1:1)

IR (KBr): 1767, 1707, 1648 cm⁻¹
MS: (M+TEA) 681; (M+H) 580

| Elementaranalyse: C₂₇H₄₁N₅O₇S | | | |
|---|---|---|---|
| ber. | C 55,94 | H 7,13 | N 12,08 |
| gef. | C 55,59 | H 7,08 | N 12,11 |

### (h) (1S,5R)-7-Oxo-2-L-tryptophanyl-2,6-diazabicyclo[3.2.0]heptan-6-sulfonsäure

IR (KBr): 1764, 1657 cm⁻¹
MS: (M+H) 379

### (i) (1S,5R)-7-Oxo-2-(3-pyridylacetyl)-2,6-diazabicyclo[3.2.0]heptan-6-sulfonsäure-Natriumsalz

IR: 1760, 1649, 1555 cm⁻¹
MS: (M-Na) 309

### (j) (1S,5R)-2-[(RS)-2-Indolylcarbonyl]-7-oxo-2,6-diazabicyclo[3.2.0]heptan-6-sulfonsäure-Triäthylaminsalz (1:1)

IR (KBr): 1765, 1652 cm⁻¹
MS: (M-TEA) 336

| Elementaranalyse: C₂₀H₃₀N₄O₅S | | | |
|---|---|---|---|
| ber. | C 54,78 | H 6,90 | N 12,78 |
| gef. | C 54,76 | H 7,12 | N 13,09 |

### (k) (1S,5R)-2-[(R)-α-Hydroxyphenylacetyl]-7-oxo-2,6-diazobicyclo[3.4.0]heptan-6-sulfonsäure-Natriumsalz (1:1)

IR (KBr): 1759, 1649 cm⁻¹
MS: (M+H) 349

### (l) (1S,5R)-2-[(S)-α-Hydroxyphenylacetyl]-7-oxo-2,6-diazabicyclo[3.2.0]heptan-6-sulfonsäure-Triäthylaminsalz (1:1)

IR (KBr): 1765, 1646 cm⁻¹
MS: (M+H) 428; (M+TEA) 529

### (m) 5-Benzyl-1-t-butyl [(S)-1-[[(1S,5R)-7-oxo-6-sulfo-2,6-diazabicyclo[3.2.0]hept-2-yl]carbonyl]pentamethylen]dicarbamat-Triäthylaminsalz (1:1)

IR: 1768, 1711, 1650 cm⁻¹
MS: (M-TEAH⁺) 553

| Elementaranalyse: C₃₀H₄₉N₅O₉S | | | |
|---|---|---|---|
| ber. | C 54,94 | H 7,73 | N 10,68 |
| gef. | C 54,25 | H 7,36 | N 10,57 |

### (n) (1S,5R)-2-[N6-[(Benzyloxy)carbonyl]-L-lysyl]-7-oxo-2,6-diazobicyclo[3.2.0]heptan-6-sulfonsäure

IR (KBr): 1768, 1695, 1665 cm⁻¹
MS: 453

| Elementaranalyse: C₁₉H₂₆N₄O₇S | | | |
|---|---|---|---|
| ber. | C 50,21 | H 5,77 | N 12,33 |
| gef. | C 50,08 | H 5,74 | N 12,22 |

### (o) (1S,5R)-2-[(2-Amino-4-thiazolyl)acetyl]-7-oxo-2,6-diazabicyclo[3.2.0]heptan-6-sulfonsäure-Triäthylaminsalz

IR(KBr): 1767, 1635 cm⁻¹
MS=434 (M+H)

### (p) (1S,5R)-2-[R-2-(m-Hydroxyphenyl)glycyl]-7-oxo-2,6-diazabicyclo[3.2.0]heptan-6-sulfonsäure

IR (KBr): 1767, 1661 cm⁻¹

| Elementaranalyse: C₁₃H₁₅N₃O₆S | | | |
|---|---|---|---|
| ber. | C 45,74 | H 4,43 | N 12,31 |
| gef. | C 45,82 | H 4,09 | N 12,22 |

### (q) (1S,5R)-2-[DL-2-(m-Hydroxyphenyl)glycyl-7-oxo-2,6-diazabicyclo[3.2.0]heptan-6-sulfonsäure

IR (KBr): 1765, 1658 cm⁻¹

| Elementaranalyse: C₁₃H₁₅N₃O₆S | | | |
|---|---|---|---|
| ber. | C 45,74 | H 4,43 | N 12,31 |
| gef. | C 45,30 | H 4,29 | N 11,85 |

### (r) (1S,5R)-2-[DL-2-(2-Amino-4-thiazolyl)glycyl]-7-oxo-2,6-diazabicyclo[3.2.0]heptan-6-sulfonsäure-Triäthylaminsalz

IR(KBr): 1767, 1670 cm⁻¹
MS=348 (M+H)

### (s) (1S,5R)-2-[R,S-Amino-(4-hydroxyphenyl)-acetyl]-7-oxo-2,6-diazabicyclo[3.2.0]heptan-6-sulfonsäure-Natriumsalz

IR(KBr): 1766, 1659 cm⁻¹
MS=340 (M-H)

### (t) (1S,5R)-(E)-2-[3-(4-Hydroxy-phenyl)acryloyl]-7-oxo-2,6-diazabicyclo[3.2.0]heptan-6-sulfonsäure-Natriumsalz

IR(KBr): 1753, 1644 cm⁻¹
MS=336 (M-Na).

### Beispiel 7

### (1aS,3aR,6bR)-1,1a,3a,6b-Tetrahydro-5-methoxy-1-oxo-2,6a-diazacyclobuta[cd]inden-2,6(3H,4H)-dicarbonsäure-2-t-butylmonoester

Eine Lösung von 2-t-Butyl-6-(p-nitrobenzyl)-(1aS,3aR,6bR)-1,1a,3a,6b-tetrahydro-5-methoxy-1-oxo-2,6a-diazabicyclobuta[cd]inden-2,6(3H,4H)-dicarboxylat (200 mg, 0,43 mmol) in Aethylacetat (20ml) wird über 10% Pd/C hydriert. Der Katalysator wird abfiltriert und die Lösung eingedampft. Der Rückstand wird in CH₂Cl₂ aufgenommen und mit einer NaHCO₃-Lösung (160 mg in 10 ml Wasser) versetzt. Die wässrige Phase wird mit CH₂Cl₂ (2x50 ml) extrahiert und schliesslich mit 1N HCl (2 ml) auf pH 4 eingestellt. Die wässrige Phase wird mit CH₂Cl₂ (3x100 ml) extrahiert. Nach dem Trocknen, dem Abdampfen und der Kristallisierung (Aethylacetat/n-Hexan) erhält man 70 mg (49%) (1aS,3aR,6bR)-1,1a,3a,6b-Tetrahydro-5-methoxy-1-oxo-2,6a-diazabicyclobuta[cd]inden-2,6-(3H,4H)-dicarbonsäure-2-t-butylmonoester.
IR (KBr): 1761, 1669 cm⁻¹

| Elementaranalyse: C₁₅H₂₀N₂O₆ | | | |
|---|---|---|---|
| ber. | C 55,55 | H 6,22 | N 8,64 |
| gef. | C 55,12 | H 6,25 | N 8,57 |

Das als Ausgangsverbindung verwendete 2-t-Butyl-6-(p-nitrobenzyl)-(1aS,3aR,6bR)-1,1a,3a,6b-tetrahydro-5-methoxy-1-oxo-2,6a-diazabicyclobuta[cd]inden-2,6(3H,4H)-dicarboxylat kann wie folgt hergestellt werden:

### Benzyl (1S,5S)-6-(3,4-dimethoxybenzyl)-4,7-dioxo-2,6-diazabicyclo[3.2.0]heptan-2-carboxylat

In 50 ml Methylenchlorid löst man 3,7 g (47 mmol) DMSO und kühlt unter Argon auf -78°C ab. Dann setzt man 5 ml (35 mmol) Trifluoressigsäureanhydrid tropfenweise hinzu, rührt für 15 Minuten und tropft langsam die Lösung von 9,7 g (23,5 mmol) Benzyl (1S,4S,5S)-6-(3,4-dimethoxybenzyl)-4-hydroxy-7-oxo-2,6-diazabicyclo[3.2.0]heptan-2-carboxylat in 50 ml Methylenchlorid hinzu und lässt 1,5 Stunden reagieren. Bevor man auf Raumtemperatur aufwärmen lässt, setzt man 12 ml (70 mmol) Aethyldiisopropylamin hinzu. Das Reaktionsgemisch wird dann je einmal mit verdünnter wässriger Salzsäure, gesättigter wässriger Natriumhydrogencarbonatlösung und gesättigter wässriger Natriumchloridlösung extrahiert, und die organische Phase wird über Magnesiumsulfat getrocknet. Nach dem Einengen erhält man 9,5 g (98%) eines gelben Oels.
IR (Film): 1760, 1709 cm⁻¹
MS: (M⁺) 410
In der gleichen Weise wird hergestellt:

### t-Butyl-(1S,5S)-6-(2,4-dimethoxybenzyl)-4,7-dioxo-2,6-diazabicyclo[3.2.0]heptan-2-carboxylat

Ausbeute 90%
NMR (250 MHz, δ ppm, CDCl₃): 1,49 (s,9H), 3,80 und 3,82 (2s,2x3H), 3,86 (d,J=4Hz,1Hz), 4,05 (breit s,2H), 4,10 (d,J=16Hz,1H), 4,54 (d,J=16Hz,1Hz), 5,11 und 5,40 (breit, 1H), 6,44 (m,2H), 7,13 (m,1H)

### t-Butyl (1S,5R)-6-(2,4-dimethoxybenzyl)-4-[(2,2-dimethyl-4-oxo-4H-m-dioxan-6-yl)methylen]-7-oxo-2,6-diazabicyclo[3.2.0]heptan-2-carboxylat

Eine Lösung von t-Butyl-(1S,5S)-6-(2,4-dimethoxybenzyl)-4,7-dioxo-2,6-diazabicyclo[3.2.0]heptan-2-carboxylat (1,61 g, 4,28 mmol) in Acetonitril (50 ml) wird mit 2,2-Dimethyl-6-[(triphenylphosphoranyliden)methyl]-4H-1,3-dioxan-4-on (2,06 g, 5,13 mmol) versetzt und 48 Stunden gerührt. Das Lösungsmittel wird abgedampft und der Rückstand über Kieselgel chromatographiert (Eluierungsmittel CH₂Cl₂:Aethylacetat 65:35) und aus Aethylacetat kristallisiert. Man erhält 2,07 g (97%) t-Butyl-(1S,5R)-6-(2,4-dimethoxybenzyl)-4-[(2,2-dimethyl-4-oxo-4H-m-dioxan-6-yl)methylen]-7-oxo-2,6-diazabicyclo[3.2.0]heptan-2-carboxylat. Smp. 171-172°C.
IR (KBr): 1747, 1721, 1696 cm⁻¹

| Elementaranalyse: C₂₆H₃₂N₂O₈ | | | |
|---|---|---|---|
| ber. | C 62,39 | H 6,44 | N 5,60 |
| gef. | C 61,85 | H 6,63 | N 5,21 |

### t-Butyl-(1S,4R,5R)-6-(2,4-dimethoxybenzyl)-4-[(2,2-dimethyl-4-oxo-4H-m-dioxan-6-yl)methyl]-7-oxo-2,6-diazabicyclo[3.2.0]heptan-2-carboxylat

Eine Lösung von t-Butyl (1S,5R)-6-(2,4-dimethoxybenzyl)-4-[(2,2-dimethyl-4-oxo-4H-m-dioxan-6-yl)-methylen]-7-oxo-2,6-diazabicyclo[3.2.0]heptan-2-carboxylat (9,85 g, 19,68 mmol) wird in THF:Aethylacetat 1:1 (300 ml) gelöst und über Pd/C hydriert. Nach dem Abfiltrieren des Katalysators und dem Abdampfen der Lösungsmittel wird der Rückstand aus Aethylacetat kristallisiert. Man erhält 7,67 g (77,5%) t-Butyl-(1S,4R,5R)-6-(2,4-dimethoxybenzyl)-4-[(2,2-dimethyl-4-oxo-4H-m-dioxan-6-yl)-methyl]-7-oxo-2,6-diazabicyclo[3.2.0]heptan-2-carboxylat. Smp. 152-154°C.
IR (KBr): 1748, 1725, 1690 cm⁻¹

| Elementaranalyse: C₂₆H₃₄N₂O₈ | | | |
|---|---|---|---|
| ber. | C 62,14 | H 6,82 | N 5,57 |
| gef. | C 62,18 | H 6,98 | N 5,58 |

### t-Butyl (1S,4R,5R)-4-[(2,2-dimethyl-4-oxo-4H-m-dioxan-6-yl)methyl]-7-oxo-2,6-diazabicyclo[3.2.0]heptan-2-carboxylat

t-Butyl (1S,4R,5R)-4-[(2,2-dimethyl-4-oxo-4H-m-dioxan-6-yl)methyl]-7-oxo-2,6-diazabicyclo[3.2.0]heptan-2-carboxylat wird in Analogie zu Beispiel 1 durch Behandeln von t-Butyl (1S,4R,5R)-6-(2,4-dimethoxybenzyl)-4-[(2,2-dimethyl-4-oxo-4H-m-dioxan-6-yl)methyl]-7-oxo-2,6-diazabicyclo[3.2.0]heptan-2-carboxylat mit Kaliumperoxodisulfat in 69% Ausbeute erhalten.
IR (KBr): 1774, 1731, 1701 cm⁻¹

| Elementaranalyse: C₁₇H₂₄N₂O₆ | | | |
|---|---|---|---|
| ber. | C 57,94 | H 6,87 | N 7,95 |
| gef. | C 57,63 | H 6,68 | N 7,64 |

### p-Nitrobenzyl (1S,4R,5R)-2-(t-butoxycarbonyl)-β,7-dioxo-2,6-diazabicyclo[3.2.0]heptan-4-butyrat

Eine Lösung von t-Butyl (1S,4R,5R)-4-[(2,2-dimethyl-4-oxo-4H-m-dioxan-6-yl)methyl]-7-oxo-2,6-diazabicyclo[3.2.0]heptan-2-carboxylat (3,5 g, 9,93 mmol) wird in Toluol gelöst (25 ml), mit p-Nitrobenzylalkohol (1,52 g, 9,93 mmol) versetzt und auf 110°C während einer Stunde erwärmt. Nach dem Abdampfen des Lösungsmittels wird der Rückstand an Kieselgel chromatographiert (Eluierungsmittel Aethylacetat:n-Hexan 8:2) und aus Aethylacetat:n-Hexan umkristallisiert. Man erhält 3,31 g (74%) p-Nitrobenzyl (1S,4R,5R)-2-(t-butoxycarbonyl)-β,7-dioxo-2,6-diazabicyclo[3.2.0]heptan-4-butyrat. Smp. 107-110°C.
IR (KBr): 1770, 1749, 1694 cm⁻¹

| Elementaranalyse: C₂₁H₂₅N₃O₈ | | | |
|---|---|---|---|
| ber. | C 56,37 | H 5,63 | N 9,39 |
| gef. | C 56,89 | H 5,83 | N 9,21 |

### t-Butyl-(1S,4R,5R)-4-[3-diazo-3-[[(p-nitrobenzyl)oxy]carbonyl]acetonyl]-7-oxo-2,6-diazabicyclo[3.2.0]heptan-2-carboxylat

Eine Lösung von p-Nitrobenzyl (1S,4R,5R)-2-(t-butoxycarbonyl)-β,7-dioxo-2,6-diazabicyclo[3.2.0]heptan-4-butyrat (2,28 g, 5,1 mmol) wird in Acetonitril (100 ml) gelöst und bei ca. 0°C mit Triäthylamin (0,85 ml, 6,11 mmol) und Tosylazid (1,10 g, 5,61 mmol) versetzt. Nach einer Stunde wird das Lösungsmittel abgedampft. Der Rückstand wird in Methylenchlorid aufgenommen und mit 10%iger wässriger Kochsalzlösung gewaschen. Nach dem Trocknen und dem Abdampfen des Lösungsmittels wird der Rückstand an Kieselgel chromatographiert (Eluierungsmittel Aethylacetat:n-Hexan 6:4) und umkristallisiert (Aethylacetat/n-Hexan). Man erhält 2,08 g (86%) t-Butyl (1S,4R,5R)-4-[3-diazo-3-[[(p-nitrobenzyl)oxy]carbonyl]acetonyl]-7-oxo-2,6-diazabicyclo[3.2.0]heptan-2-carboxylat, Smp. 132-134°C.
IR (KBr): 2135, 1762, 1710, 1655 cm⁻¹

| Elementaranalyse: C₂₁H₂₃N₅O₈ | | | |
|---|---|---|---|
| ber. | C 53,38 | H 4,90 | N 14,79 |
| gef. | C 53,29 | H 4,89 | N 14,78 |

### 2-t-Butyl-6-(p-nitrobenzyl)-(1aS,3aR,6bR)-1,1a,3a,6b-tetrahydro-5-hydroxy-1-oxo-2,6a-diazabicyclo[cd]inden-2,6(3H,4H)-dicarboxylat

Eine Lösung von (447 mg, 0,9 mmol) t-Butyl (1S,4R,5R)-4-[3-diazo-3-[[(p-nitrobenzyl)oxy]carbonyl]acetonyl]-7-oxo-2,6-diazabicyclo[3.2.0]heptan-2-carboxylat in Methylenchlorid (20 ml) wird mit Rhodium(II)caprylat (20 mg) versetzt. Nach einer Stunde wird das Lösungsmittel abgedampft und der Rückstand an Kieselgel chromatographiert (Eluierungsmittel Aethylacetat/n-Hexan 6:4) und umkristallisiert (Aether). Man erhält 180 mg (43%) 2-t-Butyl-6-(p-nitrobenzyl)-(1aS,3aR,6bR)-1,1a,3a,6b-tetrahydro-5-hydroxy-1-oxo-2,6a-diazabicyclo[cd]inden-2,6(3H,4H)-dicarboxylat. Smp. 162-164°C.
IR (KBr): 1769, 1710 cm⁻¹

| Elementaranalyse: C₂₁H₂₃N₃O₈ | | | |
|---|---|---|---|
| ber. | C 56,63 | H 5,20 | N 9,43 |
| gef. | C 56,54 | H 5,49 | N 9,29 |

### 2-t-Butyl-6-(p-nitrobenzyl)-(1aS,3aR,6bR)-1,1a,3a,6b-tetrahydro-5-methoxy-1-oxo-2,6a-diazabicyclo[cd]inden-2,6(3H,4H)-dicarboxylat

Eine Lösung von 2-t-Butyl-6-(p-nitrobenzyl)-(1aS,3aR,6bR)-1,1a,3a,6b-tetrahydro-5-hydroxy-1-oxo-2,6a-diazabicyclo[cd]inden-2,6(3H,4H)-dicarboxylat (359 mg, 0,91 mmol) in THF (2 ml) wird mit einer ätherischen Diazomethanlösung (3 ml, 1%) versetzt und über Nacht weitergerührt. Nach dem Abdampfen der Lösungsmittel wird der Rückstand über Kieselgel chromatographiert (Eluierungsmittel Aethylacetat:n-Hexan 8:2) und umkristallisiert (Aethylacetat/n-Hexan). Man erhält 222 mg (60%) 2-t-Butyl-6-(p-nitrobenzyl)-(1aS,3aR,6bR)-1,1a,3a,6b-tetrahydro-5-methoxy-1-oxo-2,6a-diazabicyclo[cd]inden-2,6(3H,4H)-dicarboxylat. Smp. 160-161°C.
IR: 1760, 1713, 1690 cm⁻¹

| Elementaranalyse: C₂₂H₂₅N₃O₈ | | | |
|---|---|---|---|
| ber. | C 57,51 | H 5,48 | N 9,15 |
| gef. | C 57,43 | H 5,48 | N 9,01 |

### Beispiel 8

### (a) (1S,5R)-2-[(E)-3-(2-Thienyl)acryloyl]-7-oxo-2,6-diazabicyclo[3.2.0]heptan-6-sulfonsäure wird durch Sulfonierung in der gleichen Weise wie in Beispiel 1 angegeben, hergestellt.

IR (KBr): 1751, 1639 cm⁻¹
MS (M-Na): 327
Das hierfür verwendete (1S,5R)-2-[(E)-3-(2-Thienyl)acryloyl]-2,6-diazabicyclo[3.2.0]heptan-7-on kann wie folgt hergestellt werden:
Zu 200 mg (1,78 mmol) (1S,5R)-2,6-Diazabicyclo[3.2.0]heptan-7-on und 0,25 ml (1,78 mmol) Triäthylamin in 8 ml Methylenchiorid gelöst, tropft man unter Eiskühlung die Lösung von 510 mg (2,14 mmol) Pivaloyl-3-(2-thienyl)-acryloylanhydrid in 2 ml Methylenchlorid. Man lässt 2 Stunden bei dieser Temperatur rühren, engt danach ein und nimmt den öligen Rest in Aethylacetat auf. Es wird nachfolgend mit Wasser, 2%iger wässriger Zitronensäurelösung, gesättigter wässriger Natriumhydrogencarbonatlösung und gesättigter wässriger Kochsalzlösung gewaschen, über Magnesiumsulfat getrocknet und eingeengt. Man erhält ein gelbes Oel, das kristallisiert.
Ausbeute 150 mg (34%)
IR (KBr): 1751, 1715, 1645 cm⁻¹
MS (M-CONH): 205
In der gleichen Weise werden hergestellt:

### (b) (1S,5R)-7-Oxo-2-(1H-tetrazol-1-ylacetyl)-2,6-diazabicyclo[3.2.0]heptan-6-sulfonsäure-Natriumsalz.

IR(KBr): 1762, 1665 cm⁻¹
MS (EI): 301,1 (M-Na)⁻

### (c) (1S,5R)-2-[DL-2-(o-Fluorphenyl)glycyl]-7-oxo-2,6-diazabicyclo[3.2.0]heptan-6-sulfonsäure-Natriumsalz.

IR(KBr): 1768, 1667 cm⁻¹

| Elementaranalyse: C₁₃H₁₄FN₃O₅S | | | | |
|---|---|---|---|---|
| ber. | C 45,48 | H 4,11 | N 12,24 | S 9,34 |
| gef. | C 45,74 | H 4,16 | N 12,16 | S 9,16 |

### (d) (1S,5R)-2-[(E)-3-(4-Imidazolyl)acryloyl]-7-oxo-2,6-diazabicyclo[3.2.0]heptan-6-sulfonsäure-Natriumsalz.

IR(KBr): 1756, 1652 cm⁻¹

| Elementaranalyse: C₁₁H₁₁N₄O₅SNa | | | | |
|---|---|---|---|---|
| ber. | C 39,52 | H 3,32 | N 16,67 | S 9,59 |
| gef. | C 39,66 | H 3,17 | N 16,81 | S 9,55 |

### Beispiel 9

R(α)-[[(1S,5R)-7-Oxo-6-sulfo-2,6-diazabicyclo[3.2.0]hept-2-yl]carbonyl]-benzylsulfat-Natriumsalz wird in Analogie zu Beispiel 1a hergestellt, aber man setzt die zweifache Menge DMF.SO₃ Komplex für die Sulfonierung ein.
IR (KBr): 1760, 1649 cm⁻¹
Das als Ausgangsverbindung verwendete (1S,5R)-2-[(R)-Hydroxyphenylacetyl]-7-oxo-2,6-diazabicyclo[3.2.0]heptan wird in Analogie zu Beispiel 6a aus (1S,5R)-2,6-Diazabicyclo[3.2.0]heptan-7-on hergestellt.
NMR (250 MHz, δ ppm, DMSO-d₆): 1,42-1,5 (m,1H), 1,80 (m,1H), 3,15 (m,1H), 4,05 (m,1H), 4,3 und 5,55 (2t,J=5,6Hz,1H), 5,3 (d,J=4Hz,1H), 5,3 und 5,4 (2d,J=6,5Hz,1H), 5,82 und 5,97 (2d,J=6,5Hz,1H), 7,2-7,5 (m,5H), 8,13 und 8,22 (2s broad,1H)

### Beispiel 10

### (1S,5R)-2-(2-Amino-4-thiazolglyoxyloyl)-7-oxo-2,6-diazabicyclo[3.2.0]heptan-6-sulfonsäure-Natriumsalz

Eine Lösung von (1S,5R)-7-Oxo-2,6-diazabicyclo[3.2.0]heptan-6-sulfonsäure (170 mg, 0,49 mmol) in Wasser (20 ml) wird mit Natriumbicarbonat (40 mg, 0,5 mmol) versetzt. Die Lösung wird mit THF verdünnt (20 ml) und mit S-(2-Benzothiazolyl)-2-aminothio-4-thiazolglyoxylat (346 mg, 0,53 mmol) versetzt. Nach 24-stündigem Rühren wird das THF eingeengt und die wässrige Phase mit Methylenchlorid extrahiert. Die wässrige Phase wird über ein polymeres hydrophobes Gel chromatographiert (Elutionsmittel Wasser dann Wasser:Methanol 4:1). Die das Produkt enthaltenen Fraktionen werden vereinigt und lyophilisiert. Ausbeute 240 mg (66%).

| Elementaranalyse: C₁₀H₉N₄O₆S₂Na | | | | |
|---|---|---|---|---|
| ber. | C 32,61 | H 2,46 | N 15,21 | S 17,41 |
| gef. | C 32,93 | H 2,57 | N 15,39 | S 17,75 |

### Beispiel 11

### (a) (1S,5R)-(E)-3-Phenyl-acryloyl-2,6-diazabicyclo[3.2.0]heptan-6-sulfonsäure-Natriumsalz

wird durch Sulfonierung in gleicher Weise wie in Beispiel 1 beschrieben hergestellt.
IR(KBr): 1756, 1650 cm⁻¹

| Elementaranalyse: C₁₄H₁₃N₂O₅S₂Na | | | |
|---|---|---|---|
| ber. | C 48,84 | H 3,81 | N 8,14 |
| gef. | C 48,84 | H 3,85 | N 8,16 |

Das hierfür verwendete (1S,5R)-(E)-3-Phenyl-acryloyl-2,6-diazabicyclo[3.2.0]heptan-7-on kann wie folgt hergestellt werden:
(1S,5R)-2,6-Diazabicyclo[3.2.0]heptan-7-on (Beispiel 2) (500 mg., 4,5 mmol) werden in 50 ml Dichlormethan suspendiert. Hierzu tropft man unter Eiskühlung 0,75 ml (5,4 mmol) Triäthylamin und anschliessend 890 mg (5,4 mmol) Zimtsäurechlorid und lässt 30 Minuten unter Kühlung und 3,5 Stunden bei Zimmertemperatur rühren. Danach wird je einmal mit gesättigter wässriger Natriumcarbonatlösung, 2%iger wässriger Zitronensäurelösung, Wasser und gesättigter wässriger Kochsalzlösung extrahiert. Die vereinigten organischen Phasen werden über Magnesiumsulfat getrocknet und eingeengt. Die Reinigung erfolgt durch Flash-Chromatographie mit Aethylacetat über Kieselgel. Ausbeute: 560 mg (52%).
IR(KBr): 1752, 1648 cm⁻¹
MS (EI): 199 (M-CONH)⁺.

In gleicher Weise werden hergestellt:

### (b) (1S,5R)-7-Oxo-2-(3-phenyl-propionyl)-2,6-diazabicyclo[3.2.0]heptan-6-sulfonsäure-Natriumsalz

IR(KBr): 1756, 1640 cm⁻¹

| Elementaranalyse: C₁₄H₁₅N₂O₅S₂Na | | | |
|---|---|---|---|
| ber. | C 46,55 | H 4,37 | N 8,09 |
| gef. | C 48,30 | H 4,66 | N 7,75 |

### (c) (1S,5R)-2-[(E)-3,4-Bis(sulfoxy)cinnamoyl]-7-oxo-2,6-diazabiyclo[3.2.0]heptan-6-sulfonsäure-Natriumsalz

(wurde mit dreifacher Menge Dimethylformamid-Schwefeltrioxid-Komplex hergestellt)
IR(KBr): 1758, 1648 cm⁻¹
¹H-NMR (250 MHz, DMSO-d₆): (Rotamere im Verhältnis von ca. 3:2) δ [ppm] = 1,72 (4-H, m, br, 1H); 2,34 (4-H, m, br, 1H); 3,16 (3-H, m, br, 1H); 4,16 (3-H, dd, br, 1H); 4,30 (3-H, dd, br, 1H); 4,36 (5-H, t, br, 1H); 4,50 (5-H, t, br, 1H); 5,36 (1-H, d, 1H, J=5Hz); 5,51 (1-H, d, 1H, J=5Hz); 6,84 (CH=, d, 1H,J=15Hz); 7,36 (1H arom., br, s); 7,40 (CH=, 1H, J=15Hz); 7,60 (1H arom., d, J=9Hz); 7,71 (1H arom., s, br).

### (d) (1S,5R)-2-(Benzylsulfonyl)-7-oxo-2,6-diazabicyclo[3.2.0]heptan-6-sulfonsäure-Natriumsalz

IR(KBr): 1764 cm⁻¹

| Elementaranalyse: C₁₂H₁₃N₂O₆S₂Na | | | | |
|---|---|---|---|---|
| ber. | C 39,13 | H 3,56 | N 7,61 | S 17,41 |
| gef. | C 39,24 | H 3,52 | N 7,64 | S 17,13 |

### Beispiel 12

### (a) (1S,5R)-2-[(E)-p-Aminocinnamoyl]-7-oxo-2,6-diazabicyclo[3.2.0]heptan-6-sulfonsäure

200 mg (0,46 mmol) (1S,5R)(E)-2-[3-(4-Chloracetylamino-phenyl)-acryloyl]-7-oxo-2,6-diazabicyclo[3.2.0]heptan-6-sulfonsäure-Natriumsalz und 35 mg (0,46 mmol) fein zerstossener Thioharnstoff in 5 ml Aethanol werden 5 Minuten bei Raumtemperatur, 18 Stunden bei 60°C und 1 Stunde unter Rückfluss gehalten. Es entsteht eine grünlich-gelbe Suspension, die eingeengt und mit 4 ml Wasser versetzt wird. Man stellt auf pH 3-4 ein und erhitzt 40 Minuten auf 100°C. Die dabei entstehende gelbe Lösung wird dreimal mit Aethylacetat extrahiert, die wässrige Phase eingeengt und über eine hydrophobe Gelsäule gereinigt. Ausbeute: 42 mg (27%).
IR(KBr): 1758, 1642 cm⁻¹
MS (ISN): 336,1 (M-H)⁻
Das als Ausgangsmaterial eingesetzte (1S,5R)(E)-2-[3-(4-Chloracetylaminophenyl)-acryloyl]-7-oxo-2,6-diazabicyclo[3.2.0]heptan-6-sulfonsäure-Natriumsalz kann wie folgt hergestellt werden:

### (E)-3-(4-Chloracetylamino-phenyl)-acrylsäure

4-Aminozimtsäure-hydrochlorid (5 g, 25 mmol) werden in 30 ml Methylenchlorid suspendiert, auf 8°C gekühlt und mit 3 ml Pyridin und 4,47 g (26 mmol) Chloressigsäureanhydrid versetzt. Man lässt dann bei Raumtemperatur über Nacht rühren. Die Suspension wird abgenutscht und der Filterkuchen in Wasser gewaschen und filtriert. Ausbeute: 5,1 g (85%).
IR(KBr): 1686, 1659 cm⁻¹

| Elementaranalyse: C₁₁H₁₀ClNO₃ | | | |
|---|---|---|---|
| ber. | C 55,13 | H 4,21 | N 5,84 |
| gef. | C 55,40 | H 4,11 | N 5,74 |

### (1S,5R)-(E)-2-Chlor-N-[4-[3-oxo-3-(7-oxo-2,6-diazabicyclo[3.2.0]hept-2-yl)-propenyl]-phenyl]acetamid.

(E)-3-(4-Chloracetylamino-phenyl)-acrylsäure (1,44 g, 6,0 mmol) wird in 50 ml Tetrahydrofuran suspendiert, mit 0,66 ml (6,0 mmol) N-Methylmorpholin versetzt und auf -10°C gekühlt. Anschliessend tropft man langsam 0,8ml (6,0 mmol) Chlorameisensäureisobutylester hinzu, rührt 30 Minuten unter Kühlung und dann bei Raumtemperatur über Nacht. Das Lösungsmittel wird im Vakuum entfernt, der Rest in Aethylacetat aufgenommen und zweimal mit Wasser und einmal mit gesättigter wässriger Kochsalzlösung extrahiert. Man trocknet die organische Phase über Magnesiumsulfat und engt ein. Das so erhaltene gemischte Anhydrid wird in 25 ml Dichlormethan verdünnt und zu einer Lösung von 500 mg (4,5 mmol) (1S,5R)-2,6-Diazabicyclo[3.2.0]heptan-7-on (Beispiel 2), gelöst in 25 ml Dichlormethan und 4 ml Dimethylformamid, getropft. Man lässt über Nacht reagieren und nutscht das dabei ausgefallene Produkt ab. Letzteres wird durch Chromatographie (Kieselgel, 1. Aethylacetat, 2. Aethylacetat/Aethanol = 3:2) gereinigt. Ausbeute: 1,0 g (50%).
IR(KBr): 1756, 1696, 1651 cm⁻¹
MS (ISP): 334,3 (M+H)⁺

### (1S,5R)(E)-2-[3-(4-Chloracetylamino-phenyl)-acryloyl]-7-oxo-2,6-diazabicyclo[3.2.0]heptan-6-sulfonsäure-Natriumsalz

Diese Verbindung wird aus (1S,5R)-(E)-2-Chlor-N-[4-[3-oxo-3-(7-oxo-2,6-diazabicyclo[3.2.0]hept-2-yl)-propenyl]-phenyl]acetamid analog der in Beispiel 1 beschriebenen Methode (Sulfonierung) hergestellt.
IR(KBr): 1757, 1686 cm⁻¹
MS (ISN): 412,0 (M-Na)⁻
In gleicher Weise wird hergestellt:

### (b) (1S,5R)-7-Oxo-2-(1,2,4-triazol-3-yl-thio-acetyl)-2,6-diazabicyclo[3.2.0]heptan-6-sulfonsäure.

IR(KBr): 1756, 1636 cm⁻¹
MS (ISN): 332,0 (M-H)⁻

### Beispiel 13

### 4-[(E)-2-[[(1S,5R)-7-Oxo-6-sulfo-2,6-diazabicylo[3.2.0]heptan-2-yl]carbonyl]-vinyl]-o-phenylen-diacetat-Natriumsalz

wird durch Sulfonierung in der gleichen Weise wie in Beispiel 1 angegeben hergestellt.
IR(KBr): 1764, 1650 cm⁻¹
MS (M-Na)⁻ 436,7
Das hierfür verwendete 4-[(E)-2-[[(1S,5R)-7-Oxo-2,6-diazabicyclo[3.2.0]heptan-2-yl]carbonyl]-vinyl]-o-phenylen-diacetat kann wie folgt hergestellt werden:
3-[3,4-Bis(acetoxy)phenyl]-2-propensäureanhydrid (365 mg, 0,72 mmol) und (1S,5R)-2,6-Diazabicyclo[3.2.0]heptan-7-on werden zusammen in 5 ml DMF bei Raumtemperatur über Nacht gerührt. Danach entfernt man das Lösungsmittel bei 70°C im Hochvakuum, nimmt den öligen Rest in Wasser auf und extrahiert mit Aethylacetat. Die organische Phase wird über Magnesiumsulfat getrocknet, eingeengt und der dabei anfallende kristalline Rückstand in Aethylacetat digeriert und abgenutscht.
Ausbeute: 120 mg (47%)
Smp. 160-165°C (Aethylacetat)
IR(KBr): 1769, 1648 cm⁻¹

### Beispiel 14

### (a) (1S,5R)-(E)-4-Methoxycarbonylmethylen-7-oxo-6-sulfo-2,6-diazabicyclo[3.2.0]heptan-2-carbonsäur-benzylester-Natriumsalz

wird durch Sulfonierung in der gleichen Weise, wie in Beispiel 1 angegeben, hergestellt.
IR(KBr): 1749, 1700 cm⁻¹
MS (ISN): 395,1 (M-Na)⁻
Der hierfür verwendete (1S,5R)-(E)-4-Methoxycarbonylmethylen-7-oxo-2,6-diazabicyclo[3.2.0]heptan-2-carbonsäure-benzylester kann wie folgt hergestellt werden:

### Benzyl (1S,4S,5S)-4-hydroxy-7-oxo-2,6-diazabicyclo[3.2.0]heptan-2-carboxylat

wird in gleicher Weise aus Benzyl (1S,4S,5S)-4-hydroxy-6-(3,4-dimethoxybenzyl)-7-oxo-2,6-diazabicyclo[3.2.0]heptan-2-carboxylat (Beispiel 1) wie für Benzyl (1S,5R)-7-oxo-2,6-diazabicyclo[3.2.0]heptan-2-carboxylat (Beispiel 1) beschrieben hergestellt.
IR(KBr): 1764, 1720, 1660 cm⁻¹

| Elementaranalyse: C₁₃H₁₄N₂O₄ | | | |
|---|---|---|---|
| ber. | C 59,54 | H 5,38 | N 10,68 |
| gef. | C 59,47 | H 5,50 | N 10,38 |

### (1S,4S,5S)-2-Benzyloxycarbonyl-4-[(R)- und (S)-tetrahydropyran-2-yloxy]-2,6-diazabicyclo[3.2.0]heptan-7-on (1:1 Gemisch)

Benzyl(1S,4S,5S)-4-hydroxy-7-oxo-2,6-diazabicyclo[3.2.0]heptan-2-carboxylat (40.0 g, 153 mmol) wird in 500 ml Dimethylformid vorgelegt, mit 2.9 g (15 mmol) p-Toluosulfonsäur-Monohydrat versetzt und mit 28 ml (305 mmol) Dihydropyran behandelt. Nach 24 stündiger Reaktionszeit entfernt man das Lösungsmittel im Hochvakuum und reinigt das verbleibende braune Oel durch Chromatographie [Kieselgel, Aethylacetat/n-Hexan (3:2)].
Ausbeute: 40 g (76%)
IR (Film): 1775, 1714 cm⁻¹
MS (CI): 364 (M+NH₄)⁺

### (1S,4S,5S)-6-(t-butyl-dimethyl-silanyl)-4-[(R)- und (S)-tetrahydropyran-2-yloxy]-7-oxo-2,6-diaza-bicyclo[3.2.0]heptan-2-carbonsäurebenzylester (1:1 Gemisch).

(1S,4S,5S)-2-Benzyloxycarbonyl-4-[(R)- und (S)-tetrahydropyran-2-yloxy]-2,6-diaza-bicyclo[3.2.0]heptan-7-on (1:1 Gemisch)(39.85 g, 115 mmol) wird in 800 ml Dichlormethan gelöst, mit 24.1 ml (173 mmol) Triäthylamin und 26.0 g (173 mmol) t-Butyl-dimethylchlorsilan versetzt und 24 Stunden bei Raumtemperatur gerührt. Danach wird das Reaktionsgemisch zweimal mit Wasser und einmal mit gesättigter, wässriger Kochsalzlösung extrahiert, über Magnesiumsulfat getrocknet und eingeengt. Die Reinigung erfolgt durch Chromatographie über Kieselgel mit Aethylacetat/n-Hexan (3:2).
Ausbeute: 46.8 g (88 %)
IR (Film): 1757, 1711 cm⁻¹
MS (CI): 478 (M+NH₄)⁺

### Benzyl(1S,4S,5S)-6-(t-butyl-dimethyl-silanyl)-4-hydroxy-7-oxo-2,6-diazabicyclo[3.2.0]heptan-2-carboxylat

2,6 g (5.64 mmol) (1S,4S,5S)-6-(t-butyl-dimethyl-silanyl)-4-[(R)- und (S)-tetrahydropyran-2-yloxy]-7-oxo-2,6-diaza-bicyclo[3.2.0]heptan-2-carbonsäurebenzylester benzylester (1:1 Gemisch) werden zusammen mit 140 mg (0.56 mmol) Pyridinium-p-toluolsulfonat in 75 ml Methanol bei 50°C einen Tag behandelt. Danach wird das Lösungsmittel im Vakuum entfernt und der ölige Rest durch Säulenchromatographie gereinigt (140 g Kieselgel, Korngrösse 0.04-0.063 mm, Aethylacetat/n-Hexan=2:1). Man erhält ein farbloses Oel, das beim Stehenlassen kristallisiert.
Ausbeute: 1.39 g (65 %)
Smp. 99.5-101°C

| Elementaranalyse: C₁₉H₂₈N₂O₄Si | | | |
|---|---|---|---|
| ber. | C 60.61 | H 7.50 | N 7.44 |
| gef. | C 60.48 | H 7.64 | N 7.32 |

### (1S,5S)-6-(t-Butyl-dimethyl-silanyl)-4,7-dioxo-2,6-diazabicyclo[3.2.0]heptan-2-carbonsäure-benzylester

wird in gleicher Weise wie für Benzyl(1S,5S)-6-(3,4-dimethoxybenzyl)-4,7-dioxo-2,6-diazabicyclo[3.2.0.]heptan-2-carboxylat (Beispiel 7) beschrieben aus Benzyl(1S,4S,5S)-6-(t-butyldimethylsilanyl)-4-hydroxy-7-oxo-2,6-diazabicyclo[3.2.0]heptan-2-carboxylat hergestellt.
IR(Film): 1760, 1712 cm⁻¹
MS(FAB): 505.2 (M+Na)⁺

### (1S,5R)(E)-6-(t-Butyl-dimethyl-silanyl)-4-methoxycarbonyl-methylen-7-oxo-2,6-diazabicyclo[3.2.0.]heptan-2-carbonsäure-benzylester.

In 30 ml absolutem Toluol werden 327 mg (7.37 mmol) Natriumhydrid (55%ige Suspension in Mineralöl) suspendiert und unter Argon auf 12°C gekühlt. Hierzu tropft man 1.34 g (7.37 mmol) 'Phosphonoessigsäuretrimethylester und lässt 1.5 Stunden reagieren. Danach werden 2.76 g (7.37 mmol) (1S,5S)-6-(t-Butyl-dimethylsilanyl)-4,7-dioxo-2,6-diazabicyclo[3.2.0]heptan-2-carbonsäure-benzylester, gelöst in 30ml Toluol so zugetropft, dass die Temperatur 30°C nicht übersteigt. Anschliessend rührt man 30 Minuten bei Raumtemperatur und 1 Stunde bei 65°C. Man setzt dann Wasser zu und extrahiert dreimal mit gesättigter wässriger Natriumhydrogencarbonatlösung und einmal mit gesättigter wässriger Kochsalzlösung. Nach Trocknen über Magnesiumsulfat und Entfernen des Lösungsmittel im Vakuum erhält man ein Oel, das durch Chromatographie gereinigt wird (Kieselgel, Aethylacetat/n-Hexan=2:1).
Ausbeute: 2.17 g (68 %)
IR(Film): 1756, 1716 cm⁻¹
MS (CI): 448 (M+NH₄⁺)

### (1S,5R)(E)-4-Methoxycarbonylmethylen-7-oxo-2,6-diazabicyclo-[3.2.0.]heptan-2-carbonsäure-benzylester.

(1S,5R)(E)-6-(t-Butyl-dimethyl-silanyl)-4-methoxycarbonyl-methylen-7-oxo-2,6-diazabicyclo[3.2.0.]heptan-2-carbonsäure-benzylester (2.13 g, 4.95 mmol) wird in 30 ml Methanol zusammen mit 183 mg (4.95 mmol) Ammoniumfluorid 75 Minuten bei Raumtemperatur gerührt. Die Lösung wird im Vakuum eingeengt und der Rückstand in Aethylacetat aufgenommen. Man extrahiert zweimal mit Wasser und einmal mit gesättigter wässriger Kochsalzlösung, trocknet die organische Phase über Magnesiumsulfat und entfernt das Lösungsmittel im Vakuum.
Ausbeute: 1.32 g (84 %)
IR(KBr): 1774, 1713 cm⁻¹
MS (ISP): 334.1 (M+NH₄⁺)
In analoger Weise werden hergestellt:

### b) (1S,5R)(E)-2-Benzyloxycarbonyl-4-carbamoylmethylen-7-oxo-2,6-diazabicyclo[3.2.0]heptan-6-sulfonsäure-Natriumsalz (unter Verwendung von Triphenylphosphoranylidenacetamid anstelle von Phosphonoessigsäuretrimethylester).

IR(KBr): 1768, 1693, 1655 cm⁻¹

| Elementaranalyse: C₁₅H₁₄N₃O₇SNa | | | | |
|---|---|---|---|---|
| ber. | C 44.67 | H 3.50 | N 10.42 | S 7.95 |
| gef. | C 44.91 | H 3.49 | N 10.47 | S 7.66 |

### (c) (1S,5R)(Z)-2-Benzyloxycarbonyl-4-carbamoylmethylen-7-oxo-2,6-diazabicyclo[3.2.0]heptan-6-sulfonsäure-Natriumsalz

IR(KBr): 1769, 1694, 1659 cm⁻¹
MS (ISN): 380.1 (M⁻)

| Elementaranalyse: C₁₅H₁₄N₃O₇SNa | | | | |
|---|---|---|---|---|
| ber. | C 44.67 | H 3.50 | N 10.42 | S 7.95 |
| gef. | C 45.24 | H 3.63 | N 10.46 | S 7.73 |

### Beispiel 15

### (1S,4R,5S)-2-Benzyloxycarbonyl-4-chlor-7-oxo-2,6-diazabicyclo[3.2.0]heptan-6-sulfonsäure-Natriumsalz

wird durch Sulfonierung von (1S,4R,5S)-4-Chlor-7-oxo-2,6-diazabicyclo[3.2.0]heptan-2-carbonsäure-benzylester in gleicher Weise wie in Beispiel 1 angegeben hergestellt.
IR(KBr): 1770, 1710 cm⁻¹
MS(ISN): 359.0 (M-Na)⁻
Der hierfür verwendete (1S,4R,5S)-4-Chlor-7-oxo-2,6-diazabicyclo[3.2.0]heptan-2-carbonsäure-benzylester kann wie folgt hergestellt werden:

### (1S,4R,5S)-6-(t-Butyl-dimethyl-silanyl)-7-oxo-4-trifluormethansulfonyloxy-2,6-diazabicyclo[3.2.0.]heptan-2-carbonsäure-benzylester

Benzyl(1S,4S,5S)-6-(t-butyl-dimethyl-silanyl)-4-hydroxy-7-oxo-2,6-diazabicyclo-[3.2.0]heptan-2-carboxylat (Beispiel 14) (5.0 g, 13.28 mmol) wird zusammen mit 1.18 ml (14.61 mmol) Pyridin in 50 ml Dichlormethan gelöst und auf -3°C abgekühlt. Hierzu tropft man langsam eine Lösung aus 2.46 ml (14.61 mmol) Trifluormethansulfonsäureanhydrid in 5 ml Dichlormethan, wobei die Temperatur nicht höher als 0°C steigen soll. Man lässt 2 Stunden unter Kühlung weiterrühren und extrahiert danach den Reaktionsansatz mit je einmal verdünnter wässriger Natriumhydrogencarbonatlösung, Wasser und gesättigter wässriger Kochsatzlösung. Nach dem Filtrieren der organischen Phase durch einen hydrophoben Faltenfilter und Einengen im Vakuum erhält man ein orangefarbenes Oel.
Ausbeute: 6.0 g (89%)
IR(Film): 1765, 1716 cm⁻¹
MS (ISP): 526.4 (M+NH₄⁺)

### (1S,4R,5S)-4-Chlor-7-oxo-2,6-diazabicyclo[3.2.0]heptan-2-carbonsäurebenzylester

(1S,4R,5S)-6-(t-Butyl-dimethyl-silanyl)-7-oxo-4-trifluormethansulfonyloxy-2,6-diazabicyclo[3.2.0]heptan-2-carbonsäure-benzylester (509 mg, 1 mmol) und Tetrabutylammoniumchlorid (695 mg, 2.5 mmol) werden zusammen in 5 ml Dimethylformamid 2 Stunden bei 60°C gerührt. Danach entfernt man das Lösungsmittel im Vakuum und reinigt den Rest durch Chromatographie (Kieselgel, Aethylacetat/n-Hexan =2:1).
Ausbeute: 76 mg (27%)
IR(Film) 1774, 1707 cm⁻¹
MS (CI): 298 (M+NH₄⁺)

### Beispiel 16

### (a) (1S,4S,5S)-4-Fluor-7-oxo-6-sulfo-2,6-diazabicyclo[3.2.0]heptan-2-carbonsäure-benzylester-Natriumsalz

wird durch Sulfonierung in gleicher Weise wie in Beispiel 1 beschrieben hergestellt.
IR(KBr): 1769, 1698 cm⁻¹

| Elementaranalyse: C₁₃H₁₂FN₂O₆SNa | | | | |
|---|---|---|---|---|
| ber. | C 42.63 | H 3.30 | N 7.65 | S 8.75 |
| gef. | C 42.23 | H 3.47 | N 7.58 | S 8.59 |

Der hierfür verwendete (1S,4S,5S)-4-Fluor-7-oxo-2,6-diazabicyclo[3.2.0]heptan-2-carbonsäure-benzylester kann wie folgt hergestellt werden:

### Benzyl(1S,4R,5S)-6-(3,4-dimethoxybenzyl)-4-hydroxy-7-oxo-2,6-diazabicyclo[3.2.0]heptan-2-carboxylat

In 50 ml Ethanol löst man 1.8 g (4.4 mmol) Benzyl(1S,5S)-6-(3,4-dimethoxybenzyl)-4,7-dioxo-2,6-diazabicyclo[3.2.0]heptan-2-carboxylat (Beispiel 7) und fügt unter Eiskühlung 166 mg (4.4 mmol) Natriumborhydrid hinzu. Anschließend rührt man über Nacht bei Raumtemperatur, säuert mit Eisessig an und entfernt das Lösungsmittel im Vakuum. Der ölige Rest wird in Methylenchlorid aufgenommen, je einmal mit gesättigter wässriger Natriumhydrogencarbonatlösung und Wasser gewaschen, über Magnesiumsulfat getrocknet und eingeengt. Durch Filtration mit Aethylacetat über eine kurze Kieselgelsäule (Merck, 0.063-0.2 mm) wird ein farbloses Oel gewonnen, das beim Stehenlassen kristallisiert.
Ausbeute: 1.2 g (66%)
Smp: 118-120°C

| Elementaranalyse: C₂₂H₂₄N₂O₆ | | | |
|---|---|---|---|
| ber. | C 64.07 | H 5.87 | N 6.79 |
| gef. | C 64.07 | H 5.95 | N 6.82 |

### (1S,4R,5S)-6-(3,4-Dimethoxy-benzyl)-7-oxo-4-trifluormethansulfonyloxy-2,6-diazabicyclo[3.2.0.]heptan-2-carbonsäure-benzylester

wird analog zu (1S,4S,5S)-6-(t-Butyl-dimethyl-silanyl)-7-oxo-4-trifluormethansulfonyloxy-2,6-diazabicyclo[3.2.0]heptan-2-carbonsäure-benzylester (Beispiel 14) aus Benzyl (1S,4R,5S)-4-hydroxy-6-(3,4-dimethoxybenzyl)-7-oxo-2,6-diazabicyclo-[3.2.0]heptan-2-carboxylat hergestellt.
IR(KBr): 1772, 1713 cm⁻¹
MS (EI): 544 (M⁺)

### (1S,4S,5S)-4-Fluor-6-(3,4-dimethoxy-benzyl)-7-oxo-2,6-diazabicyclo[3.2.0]heptan-2-carbonsäure-benzylester

(1S,4R,5S)-6-(3,4-Dimethoxy-benzyl)-7-oxo-4-trifluormethan-sulfonyloxy-2,6-diaza-bicyclo[3.2.0]heptan-2-carbonsäure-benzylester (1.1 g, 2.02 mmol) und Tetrabutylammoniumfluorid-trihydrat (704 mg, 2.23 mmol) werden zusammen 10 min in 10 ml Tetrahydrofuran bei Raumtemperatur gerührt. Danach entfernt man das Lösungsmittel im Vakuum , nimmt den öligen Rest in Aethylacetat auf und extrahiert dreimal mit Wasser und einmal mit gesättigter wässriger Kochsalzlösung. Die organische Phase wird über Magnesiumsulfat getrocknet und eingeengt. Das verbleibende braune Oel chromatographiert man mit Aethylacetat über Kieselgel und erhält ein hellgelbes Oel.
Ausbeute: 700 mg (92 %)
IR(Film): 1764, 1708 cm⁻¹
MS (ISP): 437.2 (M+Na)⁺

### (1S,4S,5S)-4-Fluor-7-oxo-2,6-diazabicyclo[3.2.0heptan-2-carbonsäurebenzylester

wird analog zu Benzyl(1S,5R)-7-oxo-2,6-diazabicyclo[3.2.0]heptan-2-carboxylat (Beispiel 1) aus (1S,4S,5S)-4-Fluor-6-(3,4-dimethoxy-benzyl)-7-oxo-2,6-diazabicyclo[3.2.0]heptan-2-carbonsäure-benzylester hergestellt.
IR(Film): 1772, 1707 cm⁻¹
MS (ISN): 263.1 (M-H)⁻
In Analogie hierzu wird hergestellt:

### (b) (1S,4R,5S)-2-Benzyloxycarbonyl-4-chlor-7-oxo-2,6-diazabicyclo[3.2.0]heptan-6-sulfonsäure-Natriumsalz.

IR(KBr): 1769, 1710 cm⁻¹
MS (ISP): 359.0 (M⁻)

### Beispiel 17

### (a) (1S, 5R)-2-(2-Furan-2-yl-methylcarbamoyl)-7-oxo-2,6-diaza-bicyclo[3.2.0]heptan-6-sulfonsäure-Natriumsalz.

Eine Lösung von (1S,5R)-7-Oxo-2,6-diazabicyclo[3.2.0]heptan-6-sulfonsäure (Beispiel 6;192 mg, 1 mmol) in Wasser (5 ml) wird mit Natriumbicarbonat (84 mg, 1 mmol) behandelt. Nach 10 Minuten wird eine Lösung von Furan-2-yl-methyl-carbaminsäure-2,5-dioxo-tetrahydropyrrol-1-yl-ester (250mg, 1.05 mmol) in Acetonitril zugegeben und das Ganze noch 3 Stunden weitergerührt. Das organische Lösungsmittel wird im Vakuum entfernt und die erhaltene wässerige Phase über ein polymeres, hydrophobes Gel gereinigt. Die das Produkt enthaltenden Fraktionen werden vereinigt und lyophilisiert.
Ausbeute: 193 mg (57%).
IR(KBr):1745,1639 cm⁻¹.

| Elementaranalyse: C₁₁H₁₃N₃O₆SNa | | | |
|---|---|---|---|
| ber. | C 39.17 | H 3.59 | N 12.46 |
| gef. | C 39.20 | H 3.61 | N 12.45 |

Der oben eingesetzte Furan-2-yl-methyl-carbaminsäure-2,5-dioxotetrahydropyrrol-1-yl-ester kann wie folgt hergestellt werden:
N,N'-Disuccinimidocarbonat (0.48g, 5 mMol) wird in Acetonotril gelöst und mit Furfurylamin (2,5g, 10 mMol) versetzt. Man lässt 2 Stunden weiterrühren und engt danach das Lösungsmittel ein. Der Rückstand wird in Methylenchlorid gelöst und mit Wasser gewaschen. Die organische Phase wird über Magnesiumsulfat getrocknet und aus Methylenchlorid/ n-Hexan kristallisiert.
Smp 115-116°C.
IR(KBr):1765,1738 cm^{-1.}

| Elementaranalyse: C₁₀H₁₀N₂O₅ | | | |
|---|---|---|---|
| ber. | C 50.42 | H 4.23 | N 11.76 |
| gef. | C 50.10 | H 4.17 | N 11.60 |

In Analogie zu Beispiel 17(a) werden hergestellt:

### (b) (1S,5R)-7-Oxo-2-[2-(pyridin-2-yl)-äthylcarbamoyl]-2,6-diazabicyclo[3.2.0]heptan-6-sulfonsäure-Natriumsalz

IR(KBr):1756,1638 cm^{-1.}
MS: 339 (M-Na)

### (c) (1S,5R)-7-Oxo-2-[(2-pyridylmethyl)carbamoyl]-2,6-diazabicyclo[3.2.0]heptan-6-sulfonsäure-Natriumsalz

IR(KBr):1754,1640 cm⁻¹.
MS: 325 (M-Na)

### (d) (1S,5R)-7-Oxo-2-(2-phenyl-carbazoyl)-2,6-diazabicyclo[3.2.0]heptan-6-sulfonsäure-Natriumsalz

IR(KBr):1755,1650 cm⁻¹.
MS=325 (M-Na)

| Elementaranalyse: C₁₂H₁₃N₄O₅SNa | | | |
|---|---|---|---|
| ber. | C 41.38 | H 3.76 | N 16.09 |
| gef. | C 41.60 | H 3.77 | N 16.16 |

### (e) (1S,5R)-2-(3,4-Dihydroxy-benzyl-carbamoyl)-7-oxo-2,6-diazabicyclo[3.2.0]heptan-6-sulfonsäure-Natriumsalz

IR(KBr):1755,1634 cm⁻¹.
MS=356 (M-Na)

| Elementaranalyse: C₁₃H₁₄N₃O₇SNa | | | |
|---|---|---|---|
| ber. | C 41.16 | H 3.72 | N 11.08 |
| gef. | C 41.35 | H 3.71 | N 11.07 |

### (f) (1S,5R)-2-(3-Hydroxy-5,6-dimethyl-pyridazin-4-yl-methylcarbamoyl)-7-oxo-2,6-diazabicyclo[3.2.0]heptan-6-sulfonsäure-Natriumsalz

IR(KBr):1755,1648 cm⁻¹.
MS=370 (M-Na)

| Elementaranalyse: C₁₃H₁₆N₅O₆SNa | | | |
|---|---|---|---|
| ber. | C 39.70 | H 4.10 | N 17.80 |
| gef. | C 39.93 | H 4.53 | N 17.70 |

### (g) (1S,5R)-7-Oxo-2-(4-sulfamoyl-benzylcarbamoyl)-2,6-diazabicyclo[3.2.0]heptan-6-sulfonsäure-Natriumsalz

IR(KBr):1755,1639 cm⁻¹.

| Elementaranalyse: C₁₃H₁₅N₄O₇S₂Na | | | |
|---|---|---|---|
| ber. | C 36.62 | H 3.55 | N 13.14 |
| gef. | C 37.01 | H 3.43 | N 13.19 |

### (h) (1S,5R)-2-Cyclobutylcarbamoyl)-7-oxo-2,6-diazabicyclo[3.2.0]heptan-6-sulfonsäure-Natriumsalz

IR(KBr):1755,1637 cm⁻¹.

| Elementaranalyse: C₁₀H₁₄N₃O₅SNa | | | |
|---|---|---|---|
| ber. | C 38.58 | H 4.53 | N 13.50 |
| gef. | C 38.62 | H 4.45 | N 13.47 |

### (i) (1S,5R)-2-Cyclopropylcarbamoyl)-7-oxo-2,6-diazabicyclo[3.2.0]heptan-6-sulfonsäure-Natriumsalz

IR(KBr):1754,1640 cm⁻¹.

| Elementaranalyse: C₉H₁₂N₃O₅SNa (+0.78 Mol Aethanol) | | | |
|---|---|---|---|
| ber. | C 38.07 | H 5.05 | N 12.60 |
| gef. | C 37.83 | H 5.37 | N 12.45 |

### (j) (1S, 5R)-7-Oxo-2-(R,S)-(2-oxo-3-tetrahydrothienyl-carbamoyl)-2,6-diazabicyclo[3.2.0]heptan-6-sulfonsäure-Natriumsalz

IR(KBr):1754,1695, 1645 cm⁻¹..
MS=333.9 (M-Na)

| Elementaranalyse: C₁₀H₁₂N₃O₆S₂Na | | | |
|---|---|---|---|
| ber. | C 33.61 | H 3.39 | N 11.76 |
| gef. | C 33.85 | H 3.31 | N 12.06 |

### (k) (1S, 5R)-2-[2-(3,4-Dihydroxy-phenyl)-äthyl-carbamoyl]-7-oxo-2,6-diazabicyclo[3.2.0]heptan-6-sulfonsäure-Natriumsalz

IR(KBr):1754,1630 cm⁻¹

| Elementaranalyse: C₁₄H₁₆N₃O₇SNa | | | |
|---|---|---|---|
| ber. | C 42.75 | H 4.10 | N 10.26 |
| gef. | C 43.00 | H 4.16 | N 10.69 |

### (l) (1S,5R)-2-(4-Methoxy-phenylcarbamoyl)-7-oxo-2,6-diazabicyclo[3.2.0]heptan-6-sulfonsäure-Natriumsalz

IR(KBr):1751,1647 cm⁻¹

| Elementaranalyse: C₁₃H₁₄N₃O₆SNa | | | |
|---|---|---|---|
| ber. | C 42.98 | H 3.88 | N 11.57 |
| gef. | C 43.12 | H 4.02 | N 11.57 |

### (m) (1S,5R)-2-Cyclohexylmethylcarbamoyl-7-oxo-2,6-diazabicyclo[3.2.0]heptan-6-sulfonsäure-Natriumsalz

IR(KBr):1754,1638 cm⁻¹

| Elementaranalyse: C₁₃H₂₀N₃O₅SNa | | | |
|---|---|---|---|
| ber. | C 44.19 | H 5.71 | N 11.89 |
| gef. | C 44.31 | H 6.02 | N 11.85 |

### (n) (1S, 5R)-7-Oxo-2-(thien-2-yl-methoxy-carbonyl)-2,6-diazabicyclo[3.2.0]heptan-6-sulfonsäure-Natriumsalz

IR(KBr):1758,1705 cm⁻¹

| Elementaranalyse: C₁₁H₁₁N₂O₆S₂Na | | | |
|---|---|---|---|
| ber. | C 37.29 | H 3.13 | N 7.91 |
| gef. | C 37.46 | H 3.11 | N 7.96 |

### (o) (1S, 5R)-2-(1-Morpholinylcarbamoyl)-7-oxo-2,6-diazabicyclo[3.2.0]heptan-6-sulfonsäure-Natriumsalz

IR(KBr):1756,1650 cm⁻¹

| Elementaranalyse: C₁₀H₁₅N₄O₆SNa | | | |
|---|---|---|---|
| ber. | C 35.09 | H 4.42 | N 16.37 |
| gef. | C 35.11 | H 4.41 | N 16.33 |

### (p) (1S, 5R)-2-(2-Thien-2-ylmethylcarbamoyl)-7-oxo-2,6-diazabicyclo[3.2.0]heptan-6-sulfonsäure-Natriumsalz

IR(KBr):1753,1640 cm⁻¹

| Elementaranalyse: C₁₁H₁₂N₃O₅S₂Na | | | |
|---|---|---|---|
| ber. | C 37.39 | H 3.42 | N 11.89 |
| gef. | C 37.54 | H 3.47 | N 11.96 |

### (q) (1S,5R)-2-(Furan-2-ylcarbonyl-carbazoyl)-7-oxo-2,6-diazabicyco[3.2.0]heptan-6-sulfonsäure-Natriumsalz

IR(KBr):1736,1589 cm⁻¹
MS = 343.1 (M-NA)

| Elementaranalyse: C₁₁H₁₁N₄O₇SNa + 5% H₂O | | | |
|---|---|---|---|
| ber. | C 34.27 | H 3.44 | N 14.53 |
| gef. | C 34.34 | H 3.50 | N 14.53 |

### (r) (1S,5R)-2-[(R)-Hydroxy-2-oxo-pyrrolidin-3-yl-carbamoyl]-7-oxo-2,6-diazabicyclo[3.2.0]heptan-6-sulfonsäure-Natriumsalz

IR(KBr):1758,1703,1637 cm⁻¹
MS = 332.8 (M-Na)

| Elementaranalyse: C₁₀H₁₃N₄O₇S₂Na | | | |
|---|---|---|---|
| ber. | C 33.71 | H 3.68 | N 15.73 |
| gef. | C 33.34 | H 3.95 | N 15.87 |

### (s) (1S,5R)-2-(3-Hydroxy-phenylcarbamoyl)-7-oxo-2,6-diazabicyclo[3.2.0]heptan-6-sulfonsäure-Natriumsalz

IR(KBr):1752,1652 cm⁻¹

| Elementaranalyse: C₁₂H₁₂N₃O₆SNa | | | |
|---|---|---|---|
| ber. | C 41.26 | H 3.46 | N 12.03 |
| gef. | C 40.85 | H 3.67 | N 12.32 |

### (t) (1S,5R)-7-Oxo-2-(S)-(2-oxo-3-tetrahydrothienyl-carbamoyl)-2,6-diazabicyclo[3.2.0]heptan-6-sulfonsäure-Natriumsalz

IR(KBr):1754,1695,1646 cm⁻¹
MS = 333.9 (M-Na)

| Elementaranalyse: C₁₀H₁₂N₃O₆S₂Na | | | |
|---|---|---|---|
| ber. | C 33.61 | H 3.39 | N 11.76 |
| gef. | C 33.67 | H 3.33 | N 11.78 |

### (u) (1S,5R)-7-Oxo-2-(R)-(2-oxo-3-tetrahydrothienyl-carbamoyl)-2,6-diazabicyclo[3.2.0]heptan-6-sulfonsäure-Natriumsalz

IR(KBr):1754,1694,1644 cm⁻¹
MS = 333.9 (M-Na)

| Elementaranalyse: C₁₀H₁₂N₃O₆S₂Na | | | |
|---|---|---|---|
| ber. | C 33.61 | H 3.39 | N 11.76 |
| gef. | C 34.08 | H 3.53 | N 12.01 |

### (v) (1S,5R)-2-(4-Dimethylamino-benzylcarbamoyl)-7-oxo-2,6-diazabicyclo[3.2.0]heptan-6-sulfonsäure-Natriumsalz

IR(KBr):1752,1638 cm⁻¹

| Elementaranalyse: C₁₃H₁₅N₄O₇S₂Na | | | |
|---|---|---|---|
| ber. | C 36.62 | H 3.55 | N 13.14 |
| gef. | C 37.01 | H 3.43 | N 13.19 |

### (w) (1S,5R)-2-(4-Hydroxy-phenylcarbamoyl)-7-oxo-2,6-diazabicyclo[3.2.0]heptan-6-sulfonsäure-Natriumsalz

IR(KBr):1755,1647 cm⁻¹

| Elementaranalyse: C₁₃H₁₅N₄O₇S₂Na | | | |
|---|---|---|---|
| ber. | C 41.26 | H 3.46 | N 12.03 |
| gef. | C 41.55 | H 3.55 | N 12.19 |

### Beispiel 18

### Methyl (1S, 5R)-7-oxo-6-sulfo-2,6-diazabicyclo[3.2.0]heptan-2-carbamat-Natriumsalz

wird in Analogie zu Beispiel 1 durch Sulfonierung von Methyl-(1S,5R)-7-Oxo-2,6-diaza-bicyclo[3.2.0]heptan-2-carbamat hergestellt.
IR(KBr):1749,1268 cm⁻¹
MS: 286 (M-H), 264 (M-Na)
Das oben eingesetzte Methyl (1S,5R)-(7-Oxo-2,6-diaza-bicyclo[3.2.0]heptan-2-carbamat kann wie folgt hergestellt werden:
Eine Lösung von (1S,5R)-2,6-Diazabicyclo[3.2.0]heptan-on (Beispiel 2, 0.4g, 3.56 mmol) in Acetonitril wird mit N-Methoxycarbonyl-3-phenyloxaziridin (0.7g, 3.9 mmol) [J. Chem. Soc. Chem. Commun. 1991 435-437 (1983)] versetzt und zwei Tage gerührt. Die Lösung wird eingeengt und der Rückstand dreimal mit n-Hexan gewaschen; das n-Hexan wird jeweils dekantiert. Der Rückstand wird aus Aethylacetat/THF umkristallisiert. Ausbeute: 280mg (43%).
Smp 152-55°C
IR(KBr):1751,1704 cm^{-1.}

| Elementaranalyse: C₇H₁₁N₃O₃ | | | |
|---|---|---|---|
| ber. mit 0.08 Mol THF | C 46.04 | H 6.14 | N 22.01 |
| gef. | C 45.72 | H 6.10 | N 21.81 |

### Beispiel 19

### (1S, 5R)-7-Oxo-2-[2-(furan-2-ylmethoxycarbamoyl)-propionyl]-2,6-diazabicyclo [3.2.0]heptan-6-sulfonsäure-Natriumsalz

wird in Analogie zu Beispiel 17(a), ausgehend von (1S,5R)-7-Oxo-2,6-diazabicyclo [3.2.0]heptan-6-sulfonsäure und 1-(Furan-2-yl-methoxy)-pyrrolidin-2,5-dion in DMF in Gegenwart von Natrium 2-Aethylhexanoat hergestellt. Ausbeute 54%.
IR(KBr):1758,1643 cm⁻¹

| Elementaranalyse: C₁₄H₁₇N₃O₈SNa | | | |
|---|---|---|---|
| ber. | C 41.08 | H 3.94 | N 10.27 |
| gef. | C 41.22 | H 4.08 | N 10.29 |

Das oben eigesetzte 1-(Furan-2-yl-methoxy)-pyrrolidin-2,5-dion wird in Analogie zu Beispiel 17(a) ausgehend von N,N'-Disuccinimido-carbonat und 2-Hydroxymethylfuran hergestellt. Ausbeute 47%

| Elementaranalyse: C₉H₉NO₄ | | | |
|---|---|---|---|
| ber. | C 55.39 | H 4.65 | N 7.18 |
| gef. | C 55.29 | H 4.82 | N 7.10 |

### Beispiel 20

### (1S,5R)-2-[[(3,4-Dihydroxyphenyl)sulfonyl]acetyl]-7-oxo-2,6-diazabicyclo[3.2.0]heptan-6-sulfonsäure-Natriumsalz.

Es wird 1 g (1.69 mmol) (1S,5R)-2-[[(2,2-Diphenyl-1,3-benzodioxol-5-yl)sulfonyl]acetyl]-7-oxo-2,6-diazabicyclo[3.2.0]heptan-6-sulfonsäure-Natriumsalz in 100 ml Methanol/Wasser = 1:1 mit 550 mg Pd/C-10% mit Wasserstoff 1 Stunde behandelt. Man filtriert, engt das Filtrat ein und trennt über eine hydrophobe Gelsäule auf.
Ausbeute: 100 mg (14%)
IR(KBr): 1760, 1642 cm⁻¹

| Elementaranalyse: C₁₃H₁₃N₂O₉S₂Na | | | | |
|---|---|---|---|---|
| ber. | C 36.45 | H 3.06 | N 6.54 | S 14.97 |
| gef. | C 36.59 | H 3.08 | N 6.57 | S 14.75 |

Das als Ausgangsverbindung eingesetzte (1S,5R)-2-[[(2,2-Diphenyl-1,3-benzodioxol-5-yl)sulfonyl]acetyl]-7-oxo-2,6-diazabicyclo[3.2.0]heptan-6-sulfonsäure-NatriumsaIz kann in Analogie zu Beispiel 1 bzw. 5 durch Sulfonierung hergestellt werden:
IR(KBr): 1744, 1653 cm⁻¹
MS (ISN): 569.0 (M-Na)=

### Beispiel 21

### (1S,5R)-2-[(R,S)-Amino-(3,4-dihydroxy-phenyl)-acetyl]-7-oxo-2,6-diazabicyclo[3.2.0]heptan-6-sulfonsäure-Natriumsalz

wird ausgehend von (1S,5R)-2-[(R,S)-t-Butoxycarbonyl]amino-(3,4-dihydroxyphenyl)-acetyl]-7-oxo-2,6-diazabicyclo[3.2.0]heptan-6-sulfonsäure-Natriumsalz wie in Beispiel 6(b) hergestellt.
IR(KBr): 1765, 1659 cm⁻¹.

| Elementaranalyse: C₁₃H₁₄N₃O₇SNa | | | |
|---|---|---|---|
| ber. | C 43,70 | H 4.23 | N 11.76 |
| gef. | C 43.79 | H 4.12 | N 11.48 |

Das hierfür eingesetzte (1S,5R)-2-[(R,S)-t-Butoxycarbonyl]amino(3,4-dihydroxy-phenyl)-acetyl]-7-oxo-2,6-diazabicyclo[3.2.0]heptan-6-sulfonsäure-Natriumsalz
wird wie in Beispiel 20 hergestellt (Hydrierung) ausgehend von (1S,5R)-2-[(R,S)-t-Butoxycarbonyl]amino-2[(2,2-Diphenyl-benzo[1,3]dioxol-5-yl)]-acetyl]-7-oxo-2,6-diazabicyclo[3.2.0]heptan-6-sulfonsäure-Natriumsalz
IR(KBr): 1760, 1702, 1652 cm⁻¹
MS=456 (M-Na)
Das (1S,5R)-2-[(R,S)-t-Butoxycarbonyl]amino-1[(2,2-Diphenylbenzol[1,3]dioxol-5-yl)]-acetyl-7-oxo-2,6-diazabicyclo[3.2.0]heptan-6-sulfonsäure-Natriumsalz
wird wie in Beispiel 1(a) hergestellt (Sulfonierung) ausgehend von N-[(1R,S)-1-(2,2-Diphenylbenzo[1,3]dioxol-5-yl)-2-oxo-2-((1S,5R)-7-oxo-2,6-diazabicyclo[3.2.0]hept-2-yl)-äthyl]carbaminsäure-t-butylester, Ausbeute 80%.

Der eingesetzte N-[(1R,S)-1-(2,2-Diphenyl-benzo[1,3]dioxol-5-yl)-2-oxo-2-((1S,5R)-7-oxo-2,6-diazabicyclo[3.2.0]hept-2-yl)-äthyl]carbaminsäure-t-butylester
wird analog Beispiel 5(a) synthetisiert:
Smp. 198-201°C

| Elementaranalyse: C₃₁H₃₁N₃O₆ | | | |
|---|---|---|---|
| ber. | C 68.75 | H 5.77 | N 7.76 |
| gef. | C 68.71 | H 5.89 | N 7.63 |

### Beispiel 22

### (1S,4R,5S)-2-Benzyloxycarbonyl-4-(1-methyl-1H-tetrazol-5-yl-thio)-7-oxo-2,6-diazabicyclo[3.2.0]heptan-6-sulfonsäure-Natriumsalz

wird durch Sulfonierung in gleicher Weise wie in Beispiel 1 angegeben hergestellt.
IR(KBr): 1771, 1710 cm⁻¹
MS (ISN): 439.9 (M-Na)⁻
Der hierzu verwendete (1S,5S)-4-(1-Methyl-1H-tetrazol-5-thio)-7-oxo-2,6-diazabicyclo[3.2.0]heptan-2-carbonsäure-benzylester wird wie folgt hergestellt:
(1S,4R,5S)-6-(t-Butyl-dimethylsilanyl)-7-oxo-4-trifluormethansulfonyloxy-2,6-diazabicyclo[3.2.0]heptan-2-carbonsäure-benzylester (1.9 g, 1.96 mmol) wird in 20 ml DMF gelöst, mit 600 mg (2.16 mmol) 5-Mercapto-1-methyltetrazol-Natriumsalz-Hydrat versetzt und bei 80°C unter Argon 2 Tage gerührt. Das Lösungsmittel wird entfernt, der Rest in Aethylacetat aufgenommen und 2 mal mit Wasser gewaschen. Man trocknet über Magnesiumsulfat und chromatographiert (Kieselgel, Aethylacetat), Ausbeute: 150 mg (21%).
IR(Film): 1769, 1708 cm⁻¹
MS (ISP): 383.2 (M+Na)⁺.

### Beispiel 23

### Herstellung von Trockenampullen für die intramuskuläre Verabreichung:

Es wird in üblicher Weise ein Lyophilisat von 0,5 g des Natriumsalzes der (1S,5R)-2-[D-2-(p-Hydroxyphenyl)glycyl]-7-oxo-2,6-diazabicyclo[3.2.0]heptan-6-sulfonsäure sowie 1 g des Dinatriumsalzes der (6R,7R)-7-[2-(2-Amino-4-thiazolyl)-2-(Z-methoxyimino)acetamido]-3-/[(2,5-dihydro-6-hydroxy-2-methyl-5-oxo-as-triazin-3-yl)thio]methyl/-8-oxo-5-thia-1-azabicyclo[4.2.0]oct-2-en-2-carbonsäure hergestellt und in eine Ampulle abgefüllt. Vor der Verabreichung wird das Lyophilisat mit 4 ml einer 2%igen wässrigen Lidocainhydrochlorid-Lösung versetzt.

Die beiden Wirkstoffe können wahlweise separat in zwei verschiedene Ampullen abgefüllt werden.

## Patentansprüche (Patentansprüche für folgende(n) Vertragsstaat(en): AT, BE, CH, DE, DK, FR, GB, IT, LI, LU, MC, NL, PT, SE)

1. β-Lactame der allgemeinen Formel 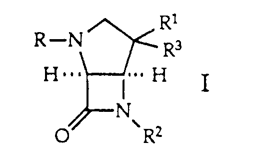 in der R C₁-C₇ Alkoxycarbonyl, C₁-C₇ Alkoxycarbonylamino, den Acylrest einer α- oder β-Aminosäure oder einen Rest der allgemeinen Formel
Q-X-Y- (a)
bedeutet, worin Q einen 3-6-gliedrigen, gegebenenfalls stickstoff-, schwefel- und/oder sauerstoffhaltigen, gegebenenfalls mit einem ankondensierten Ring verbundenen Ring, X eine direkte Bindung oder einen aus Kohlenstoff, Stickstoff, Sauerstoff und/oder Schwefel bestehenden linearen "Spacer" mit bis zu 6 Atomen bedeutet, wovon bis zu 2 Atomen Stickstoffatome und 1 Atom Sauerstoff oder Schwefel sein können, und Y eine der Gruppen -CO-, -CS-, -CONH- und (falls X als Endglied weder Schwefel noch Carbonyl enthält) -SO₂- darstellt;
und worin ferner R¹ Wasserstoff, Halogen, Carbamoyloxy, C₁-C₇ Alkanoyloxy oder eine Gruppe der Formel -S-Het, Het eine 5-6-gliedrige, stickstoff-, schwefel- und/oder sauerstoffhaltige heteroaromatische Gruppe und R² die Sulfogruppe -SO₃H darstellt oder R¹ und R² zusammen eine Gruppe der Formel 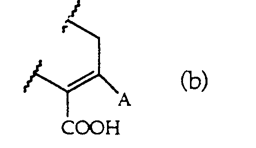 bedeutet, worin A Wasserstoff oder einen in 3-Stellung von Cephalosporinantibiotika verwendbaren Rest darstellt, und worin ferner R³ Wasserstoff oder R¹ und R³ zusammen eine Gruppe der allgemeinen Formel
=CH-R^{a} (c)
darstellen, worin R^{a} eine der Gruppen 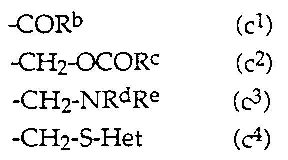 bedeutet, in denen R^{b} C₁-C₇ Alkoxy oder Amino, R^{c} C₁₋C₇ Alkyl, Phenyl oder Amino, R^{d} und R^{e} jeweils Wasserstoff oder C₁-C₇ Alkyl oder zusammen mit dem N-Atom einen 5- oder 6-gliedrigen, gegebenenfalls ein weiteres Stickstoff-, Sauerstoff- oder Schwefelatom enthaltenden N-Heterocyclus und Het eine 5-6-gliedrige, stickstoff-, schwefel- und/oder sauerstoffhaltige heteroaromatische Gruppe darstellt,
und pharmazeutisch verträgliche Salze dieser Verbindungen.

2. Verbindungen gemäss Anspruch 1 der allgemeinen Formel 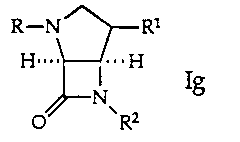 in der R C₁₋C₇ Alkoxycarbonyl, den Acylrest einer α-Aminosäure oder eine (Hetero)aromatische Acylgruppe der allgemeinen Formel
Ar-X-CO- (aa)
bedeutet, worin Ar einen 5- oder 6-gliedrigen, gegebenenfalls ein Stickstoff-, Schwefel- und/oder Sauerstoffatom enthaltenden, gegebenenfalls mit einem weiteren ankondensierten Phenylring verbundenen (Hetero)aromatischen Ring und X eine direkte Bindung oder eine der Gruppen -NH-, -CH₂-, -CO-, -CH₂O-, -CH=CH-, -CH=C(NHCOCH₃)-, -NH-CH₂-, -CH₂-NH-, -S-CH₂-, -O-CH₂-, -CHOH-, -CH(COOH)-, -CH(OSO₃H)- darstellt,
und worin ferner R¹ Wasserstoff und R² die Sulfogruppe -SO₃H darstellt oder R¹ und R² zusammen eine Gruppe der Formel 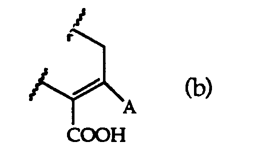 bedeutet, worin A Wasserstoff oder einen in 3-Stellung von Cephalosporinantibiotika verwendbaren Rest darstellt,
und pharmazeutisch verträgliche Salze dieser Verbindungen.

3. Verbindungen gemäss Anspruch 2, worin R t-Butoxycarbonyl darstellt.

4. t-Butoxy-(1S,5R)-7-oxo-6-sulfo-2,6-diazabicyclo[3.2.0]heptan-2-carboxylat und dessen pharmazeutisch verträgliche Salze.

5. (1aS,3aR,6bR)-1,1a,3a,6b-Tetrahydro-5-methoxy-1-oxo-2,6a-diazacyclobuta[cd]inden-2,6(3H,4H)-dicarbonsäure-2-t-butylmonoester und dessen pharmazeutisch verträgliche Salze.

6. (1S,5R)-7-Oxo-2-(phenylcarbamoyl)-2,6-diazabicyclo[3.2.0]heptan-6-sulfonsäure und dessen pharmazeutisch verträgliche Salze.

7. (1S,5R)-2-(Benzylcarbamoyl)-7-oxo-2,6-diazabicyclo[3.2.0]heptan-6-sulfonsäure und dessen pharmazeutisch verträgliche Salze.

8. (1S,5R)-7-Oxo-2-(N-phenylglycyl)-2,6-diazabicyclo[3.2.0]heptan-6-sulfonsäure und deren pharmazeutisch verträgliche Salze.

9. (1S,5R)-2-(Cyclobutylcarbamoyl-7-oxo-2,6-diazabicyclo[3.2.0]heptan-6-sulfonsäure und deren pharmazeutisch verträgliche Salze.

10. (1S,5R)-7-Oxo-2-(R,S)-(2-oxo-3-tetrahydro-thienyl-carbamoyl)-2,6-diazabicyclo[3.2.0]heptan-6-sulfonsäure und deren pharmazeutisch verträgliche Salze.

11. (1S,5R)-7-Oxo-2-(R)-(2-oxo-3-tetrahydrothienyl-carbamoyl)-2,6-diazabicyclo[3.2.0]heptan-6-sulfonsäure und deren pharmazeutisch verträgliche Salze.

12. (1S,5R)-2-(4-Hydroxy-phenylcarbamoyl)-7-oxo-2,6-diazabicyclo[3.2.0]heptan-6-sulfonsäure und deren pharmazeutisch verträgliche Salze.

13. Verbindungen gemäss Anspruch 2 der allgemeinen Formel 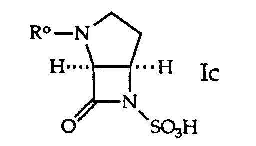 worin R^{o} eine Gruppe der allgemeinen Formel 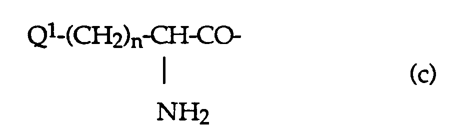 darstellt, und Q¹ einen 5- oder 6-gliedrigen, gegebenenfalls ein Stickstoff-, Schwefel- und/oder Sauerstoffatom enthaltenden, gegebenenfalls mit einem weiteren ankondensierten Phenylring verbundenen (hetero)aromatischen Ring und n die Zahl 0 oder 1 bedeutet,
und deren pharmazeutisch verträgliche Salze.

14. (1S,5R)-7-Oxo-2-L-tyrosyl-2,6-diazabicyclo[3.2.0]heptan-6-sulfonsäure und deren pharmazeutisch verträgliche Salze.

15. (1S,5R)-7-Oxo-2-L-tryptophanyl-2,6-diazabicyclo[3.2.0]heptan-6-sulfonsäure und deren pharmazeutisch verträgliche Salze.

16. (1S,5R)-2-[(R oder S)-α-Amino-(2-thienyl)acetyl]-7-oxo-2,6-diazabicyclo[3.2.0]heptan-6-sulfonsäure und deren pharmazeutisch verträgliche Salze.

17. (1S,5R)-2-[DL-2-(2-Amino-4-thiazolyl)glycyl]-7-oxo-2,6-diazabicyclo[3.2.0]heptan-6-sulfonsäure und deren pharmazeutisch verträgliche Salze.

18. Verbindungen gemäss Anspruch 13, worin R^{o} eine Gruppe der allgemeinen Formel 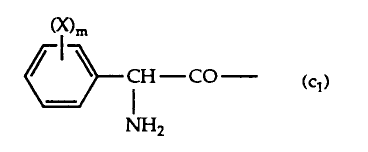 in der X Wasserstoff, Hydroxy oder Fluor und n die Zahl 1 oder 2 darstellt, bedeutet.

19. (1S,5R)-7-Oxo-2-(D-2-phenylglycyl)-2,6-diazabicyclo[3.2.0]heptan-6-sulfonsäure und deren pharmazeutisch verträgliche Salze.

20. (1S,5R)-7-Oxo-2-(L-2-phenylglycyl)-2,6-diazabicyclo[3.2.0]heptan-6-sulfonsäure und deren pharmazeutisch verträgliche Salze.

21. (1S,5R)-2-[D-2-(p-Hydroxyphenyl)glycyl]-7-oxo-2,6-diazabicyclo[3.2.0]heptan-6-sulfonsäure und deren pharmazeutisch verträgliche Salze.

22. (1S,5R)-2-[R-2-(m-Hydroxyphenyl)glycyl]]-7-oxo-2,6-diazabicyclo[3.2.0]heptan-6-sulfonsäure und deren pharmazeutisch verträgliche Salze.

23. (1S,5R)-2-[DL-2-(m-Hydroxyphenyl)glycyl-7-oxo-2,6-diazabicyclo[3.2.0]heptan-6-sulfonsäure und deren pharmazeutisch verträgliche Salze.

24. (1S,5R)-2-[DL-2-(o-Fluorphenyl)glycyl]-7-oxo-2,6-diazabicyclo[3.2.0]heptan-6-sulfonsäure und deren pharmazeutisch verträgliche Salze.

25. (1S,5R)-2-[(R,S)-Amino-(3,4-dihydroxy-phenyl)-acetyl]-7-oxo-2,6-diazabicyclo[3.2.0]heptan-6-sulfonsäure und deren pharmazeutisch verträgliche Salze.

26. Verbinungen gemäss Anspruch 1, worin R den Rest der Formel Q-X-Y- bedeutet, in welcher X einen der "Spacer"
-O-, -S-, -NH-, -NH-NH-, -CH₂-, -CO-, -CH₂O-, -CH₂CH₂-, -CH=CH-, -CH₂NH-, -S-CH₂-, -SO₂-CH₂-, -O-CH₂-, -S-CH₂CH₂-, -CH₂CH₂-NH-; -CH₂-O-NH-CO-CH₂CH₂-, -CHOH-, -CH(COOH)-, -CH(OSO₃H)-, -CH(OCONH₂)-, -CH[CH(CH₃)₂]-
und Y eine der Gruppen -CO-, CS- und -CONH- darstellt.

27. Verbinungen gemäss Anspruch 1, worin R den Rest der Formel Q-X-Y- bedeutet, in welcher X einen der "Spacer"
-O-, -NH-, -NH-NH-, -CH₂-, -CH₂O-, -CH₂CH₂-, -CH=CH-, -CH₂NH-, -O-CH₂-, -CH₂CH₂-NH-, -CHOH-, -CH[CH(CH₃)₂]
und Y die Gruppe -SO₂- darstellt.

28. Verbindungen gemäss Anspruch 1 der allgemeinen Formel 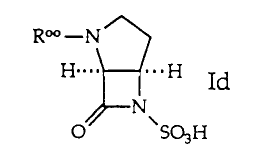 worin R^{oo} eine Gruppe der allgemeinen Formel
Q²-CH=CHCO- (d)
darstellt und Q² einen Phenylring oder einen 5-6-gliedrigen, Stickstoff, Schwefel und/oder Sauerstoff enthaltenden, gegebenenfalls mit einem ankondensierten Phenylring verbundenen heteroaromatischen Ring bedeutet,
und deren pharmazeutisch verträgliche Salze.

29. (1S,5R)-2-[(E)-3-(2-Furyl)acryloyl]-7-oxo-2,6-diazabicyclo[3.2.0]heptan-6-sulfonsäure und deren pharmazeutisch verträgliche Salze.

30. (1S,5R)-2-[(E)-3-(3-Indolyl)acryloyl]-7-oxo-2,6-diazabicyclo[3.2.0]heptan-6-sulfonsäure und deren pharmazeutisch verträgliche Salze.

31. (1S,5R)-2-[(E)-3-(4-Imidazolyl)acryloyl]-7-oxo-2,6-diazabicyclo[3.2.0]heptan-6-sulfonsäure und deren pharmazeutisch verträgliche Salze.

32. (1S,5R)-2-[(E)-3-(2-Thienyl)acryloyl]-7-oxo-2,6-diazabicyclo[3.2.0]heptan-6-sulfonsäure und deren pharmazeutisch verträgliche Salze.

33. (1S,5R)-(E)-2-[3-4-Hydroxy-phenyl)acryloyl]-7-oxo-2,6-diazabicyclo[3.2.0]heptan-6-sulfonsäure und deren pharmazeutisch verträgliche Salze.

34. Verbindungen gemäss Anspruch 1 der allgemeinen Formel 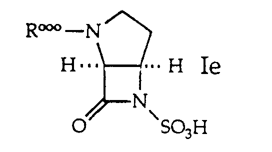 worin R^{ooo} eine Gruppe der allgemeinen Formel
Q³-(S)ₙ-CH₂CO- (e)
darstellt, n 0 oder 1 und Q³ einen Phenylring oder einen 5-6-gliedrigen, gegebenenfalls Schwefel enthaltenden, gegebenenfalls durch C₁-C₇ Alkyl oder Amino substituierten, gegebenenfalls mit einem ankondensierten Phenylring verbundenen N-Heterocyclus bedeutet,
und deren pharmazeutisch verträgliche Salze.

35. (1S,5R)-2-[[(1-Methyl-1H-tetrazol-5-yl)thio]acetyl]-7-oxo-2,6-diazabicyclo[3.2.0]heptan-6-sulfonsäure und deren pharmazeutisch verträgliche Salze.

36. (1S,5R)-2-[[1H-benzotriazol-1-yl)thio]acetyl]-7-oxo-2,6-diazabicyclo[3.2.0]heptan-6-sulfonsäure und deren pharmazeutisch verträgliche Salze.

37. (1S,5R)-2-[[(5-Methyl-1,3,4-thiadiazol-2-yl)thio]acetyl]-7-oxo-2,6-diazabicyclo[3.2.0]heptan-6-sulfonsäure und deren pharmazeutisch verträgliche Salze.

38. (1S,5R)-7-Oxo-2-(1H-tetrazol-1-ylacetyl)-2,6-diazabicyclo[3.2.0]heptan-6-sulfonsäure und deren pharmazeutisch verträgliche Salze.

39. (1S, 5R)-2-[(2-Isopropy-2H-tetrazol-5-yl)-acetyl]-7-oxo-2,6-diazabicyclo[3.2.0]heptan-6-sulfonsäure und deren pharmazeutisch verträgliche Salze.

40. Verbindungen der allgemeinen Formel 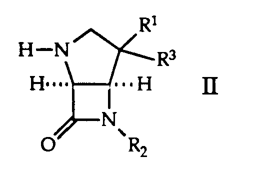 in der R¹, R² und R³ die in Anspruch 1 gegebene Bedeutung haben.

41. Verbindungen der allgemeinen Formel 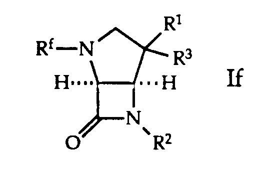 in der R^{1,} R² und R³ die in Anspruch 1 gegebene Bedeutung haben und R^{f} einen unter R in Anspruch 1 definierten Rest mit einer geschützten Aminogruppe bzw. Hydroxygruppe(n) darstellt.

42. Verbindungen der allgemeinen Formel 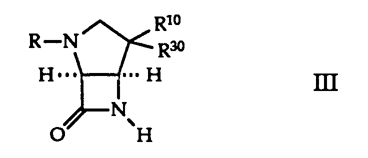 in der R die in Anspruch 1 gegebene Bedeutung und R¹⁰ und R³⁰ die in Anspruch 46 gegebenen Bedeutungen haben.

43. Verbindungen der allgemeinen Formel 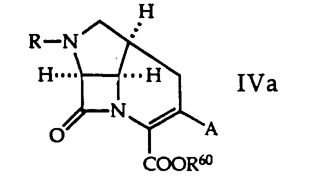 in der R die in Anspruch 1 gegebene Bedeutung hat und R⁶⁰ t-Butyl, p-Nitrobenzyl, Benzyl, Benzhydryl oder Allyl darstellt.

44. Verbindungen gemäss einem der Ansprüche 1 bis 39 als pharmazeutische Wirkstoffe.

45. Verbindungen gemäss einem der Ansprüche 1 bis 39 als β-Lactamase hemmende Wirkstoffe.

46. Verfahren zur Herstellung von Verbindungen gemäss einem der Ansprüche 1 bis 39, dadurch gekennzeichnet, dass man
(a) eine Verbindung der allgemeinen Formel 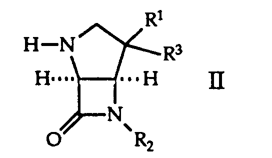 in der R¹, R² und R³ die in Anspruch 1 gegebene Bedeutung haben, mit den Rest R abgebenden Mitteln behandelt, oder dass man
(b) zur Herstellung einer Verbindung der Formel I, worin R eine freie Aminogruppe bzw. Hydroxygruppe(n) enthält, in einer Verbindung der allgemeinen Formel 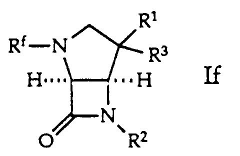 in der R¹, R² und R³ die in Anspruch 1 gegebene Bedeutung haben und R^{f} einen unter R definierten Rest mit einer geschützten Aminogruppe bzw. Hydroxygruppe(n) darstellt,
die Aminoschutzgruppe bzw. Hydroxyschutzgruppe(n) abspaltet, oder dass man
(c) zur Herstellung einer Verbindung der Formel I, worin R¹ und R² von der oben definierten Gruppe (b) verschieden sind, eine Verbindung der allgemeinen Formel 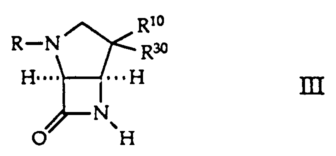 in der R die in Anspruch 1 gegebene Bedeutung hat, R¹⁰ Wasserstoff, Halogen, Carbamoyloxy, C₁-C₇ Alkanoyloxy oder eine Gruppe der Formel -S-Het, Het eine 5-6-gliedrige heteroaromatische Gruppe und R³⁰ Wasserstoff oder R¹⁰ und R³⁰ zusammen eine Gruppe der allgemeinen Formel
=CH-R^{a} (c)
darstellen, worin R^{a} eine der Gruppen 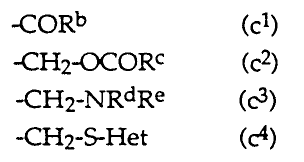 bedeutet, in denen R^{b} C₁-C₇ Alkoxy oder Amino, R^{c} C₁-C₇ Alkyl, Phenyl oder Amino, R^{d} und R^{e} jeweils Wasserstoff oder C₁-C₇ Alkyl oder zusammen mit dem N-Atom einen 5- oder 6-gliedrigen, gegebenenfalls ein weiteres Stickstoff-, Sauerstoff- oder Schwefelatom enthaltenden N-Heterocyclus und Het eine 5-6-gliedrige, stickstoff-, schwefel- und/oder sauerstoffhaltige heteroaromatische Gruppe darstellt,
mit die Sulfogruppe -SO₃H abgebenden Mitteln behandelt, oder dass man
(d) zur Herstellung einer Verbindung der Formel I, worin R¹ und R² zusammen die in Anspruch 1 definierte Gruppe (b) darstellt, eine Verbindung der allgemeinen Formel 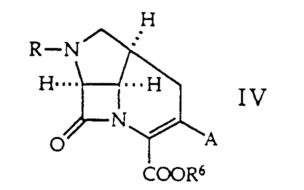 in der R und A die in Anspruch 1 angegebenen Bedeutungen haben und R⁶ eine Carbonsäureschutzgruppe darstellt,
mit die Carbonsäureschutzgruppe R⁶ abspaltenden Mitteln umsetzt, oder dass man
(e) zur Herstellung von pharmazeutisch verträglichen Salzen einer Verbindung der Formel I eine Verbindung der Formel I in ein solches Salz überführt.

47. Arzneimittel enthaltend eine Verbindung gemäss einem der Ansprüche 1 bis 39.

48. β-Lactamase hemmende Arzneimittel enthaltend eine Verbindung gemäss einem der Ansprüche 1 bis 39.

49. Arzneimittel gemäss Anspruch 47 oder 48 enthaltend ein β-Lactam-Antibiotikum.

50. Arzneimittel gemäss Anspruch 49 enthaltend ein Penicillin oder Cephalosporin als β-Lactam-Antibiotikum.

51. Arzneimittel gemäss Anspruch 50 enthaltend Benzylpenicillin, Piperacillin, Phenoxymethylpenicillin, Carbenicillin, Methicillin, Propicillin, Tricarcillin, Ampicillin, Amoxicillin, Mecillinam, Ceftazidim, Cefetamet, Cefatamet Pivoxil, Cefotaxim, Cefmenoxim, Ceftizoxim, Cefuroxim, Cephaloridin, Cephalotin, Cefazolin, Cephalexin, Cefoxitin, Cephacetril, Cefamandol, Cephapirin, Cephradin, Cephaloglycin, (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carbonsäure oder (E)-2-(Isobutoxycarbonyl)-2-pentenyl (6R,7R)-7-[(Z)-2-(2-Amino-4-thiazolyl)-2-(methoxyimino)acetamido]-3-(azidomethyl)-8-oxo-5-thia-1-azabicyclo[4.2.0]oct-2-en-2-carboxylat oder ein pharmazeutisch verträgliches Salz einer dieser Verbindungen.

52. Arzneimittel gemäss Anspruch 50 enthaltend Ceftriaxon oder eines seiner pharmazeutisch verträglichen Salze.

53. Arzneimittel gemäss Anspruch 52 enthaltend eine Verbindung gemäss Anpruch 21.

54. Arzneimittel gemäss einem der Ansprüche 47-53 als Kombinationspräparat zur gleichzeitigen, getrennten oder zeitlich abgestuften Anwendung in der antibakteriellen Therapie.

55. Verwendung von Verbindungen gemäss einem der Ansprüche 1-39 bei der Herstellung von antibakteriell wirksamen Mitteln.

## Patentansprüche (Patentansprüche für folgende(n) Vertragsstaat(en): ES, GR)

1. Verfahren zur Herstellung von β-Lactamen der allgemeinen Formel 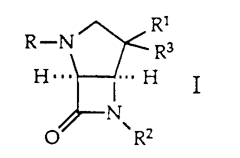 in der R C₁-C₇ Alkoxycarbonyl, C₁-C₇ Alkoxycarbonylamino, den Acylrest einer α- oder β-Aminosäure oder einen Rest der allgemeinen Formel
Q-X-Y- (a)
bedeutet, worin Q einen 3-6-gliedrigen, gegebenenfalls stickstoff-, schwefel- und/oder sauerstoffhaltigen, gegebenenfalls mit einem ankondensierten Ring verbundenen Ring, X eine direkte Bindung oder einen aus Kohlenstoff, Stickstoff, Sauerstoff und/oder Schwefel bestehenden linearen "Spacer" mit bis zu 6 Atomen bedeutet, wovon bis zu 2 Atomen Stickstoffatome und 1 Atom Sauerstoff oder Schwefel sein können, und Y eine der Gruppen -CO-,-CS-, -CONH- und (falls X als Endprodukt weder Schwefel noch Carbonyl enthält) -SO₂- darstellt;
und worin ferner R¹ Wasserstoff, Halogen, Carbamoyloxy, C₁-C₇ Alkanoyloxy oder eine Gruppe der Formel -S-Het, Het eine 5-6-gliedrige heteroaromatische Gruppe und R² die Sulfogruppe -SO₃H darstellt oder R¹ und R² zusammen eine Gruppe der Formel 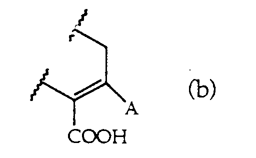 bedeutet, worin A Wasserstoff oder einen in 3-Stellung von Cephalosporinantibiotika verwendbaren Rest darstellt, und worin ferner R³ Wasserstoff oder R¹ und R³ zusammen eine Gruppe der allgemeinen Formel
=CH-R^{a} (c)
darstellen, worin R^{a} eine der Gruppen 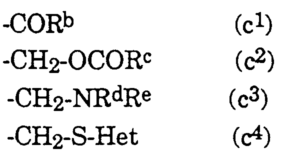 bedeutet, in denen R^{b} C₁-C₇ Alkoxy oder Amino, R^{c} C₁-C₇ Alkyl, Phenyl oder Amino, R^{d} und R^{e} jeweils Wasserstoff oder C₁-C₇ Alkyl oder zusammen mit dem N-Atom einen 5- oder 6-gliedrigen, gegebenenfalls ein weiteres Stickstoff-, Sauerstoff- oder Schwefelatom enthaltenden N-Heterocyclus und Het eine 5-6-gliedrige, stickstoff-, schwefel- und/oder sauerstoffhaltige heteroaromatische Gruppe darstellt,
und von pharmazeutisch verträglichen Salzen dieser Verbindungen, dadurch gekennzeichnet, dass man
(a) eine Verbindung der allgemeinen Formel 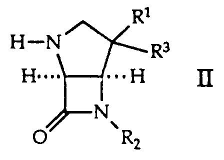 in der R¹, R² und R³ die oben gegebene Bedeutung haben, mit den Rest R abgebenden Mitteln behandelt, oder dass man
(b) zur Herstellung einer Verbindung der Formel I, worin R eine freie Aminogruppe bzw. Hydroxygruppe(n) enthält, in einer Verbindung der allgemeinen Formel 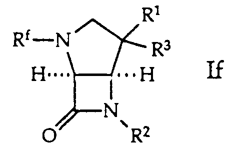 in der R¹, R² und R³ die oben gegebene Bedeutung haben und R^{f} einen unter R definierten Rest mit einer geschützten Aminogruppe bzw. Hydroxygruppe(n) darstellt,
die Aminoschutzgruppe bzw. Hydroxyschutzgruppe(n) abspaltet, oder dass man
(c) zur Herstellung einer Verbindung der Formel I, worin R¹ und R² von der oben definierten Gruppe (b) verschieden sind, eine Verbindung der allgemeinen Formel 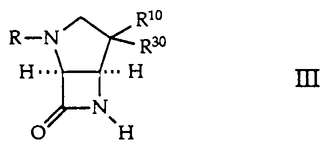 in der R die oben gegebene Bedeutung hat, R¹⁰ Wasserstoff, Halogen, Carbamoyloxy, C₁-C₇ Alkanoyloxy oder eine Gruppe der Formel -S-Het, Het eine 5-6-gliedrige heteroaromatische Gruppe und R³⁰ Wasserstoff oder R¹⁰ und R³⁰ zusammen eine Gruppe der allgemeinen Formel
=CH-R^{a} (c)
darstellen, worin R^{a} eine der Gruppen 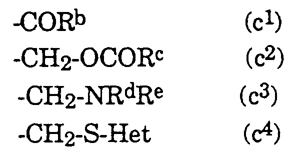 bedeutet, in denen R^{b} C₁-C₇ Alkoxy oder Amino, R^{c} C₁-C₇ Alkyl, Phenyl oder Amino, R^{d} und R^{e} jeweils Wasserstoff oder C₁-C₇ Alkyl oder zusammen mit dem N-Atom einen 5- oder 6-gliedrigen, gegebenenfalls ein weiteres Stickstoff-, Sauerstoff- oder Schwefelatom enthaltenden N-Heterocyclus und Het eine 5-6-gliedrige, stickstoff-, schwefel- und/oder sauerstoffhaltige heteroaromatische Gruppe darstellt,
mit die Sulfogruppe -SO₃H abgebenden Mitteln behandelt, oder dass man
(d) zur Herstellung einer Verbindung der Formel I, worin R¹ und R² zusammen die oben definierte Gruppe (b) darstellt, eine Verbindung der allgemeinen Formel 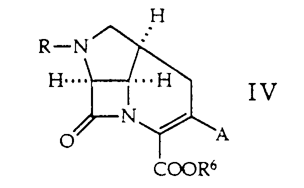 in der R und A die oben angegebenen Bedeutungen haben und R⁶ eine Carbonsäureschutzgruppe darstellt,
mit die Carbonsäureschutzgruppe R⁶ abspaltenden Mitteln umsetzt, oder dass man
(e) zur Herstellung von pharmazeutisch verträglichen Salzen einer Verbindung der Formel I eine Verbindung der Formel I in ein solches Salz überführt.

2. Verfahren zur Herstellung von Verbindungen gemäss Anspruch 1 der allgemeinen Formel 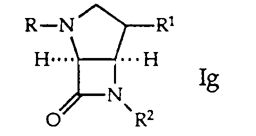 in der R C₁-C₇ Alkoxycarbonyl, den Acylrest einer α-Aminosäure oder eine (Hetero)aromatische Acylgruppe der allgemeinen Formel
Ar-X-CO- (aa)
bedeutet, worin Ar einen 5- oder 6-gliedrigen, gegebenenfalls ein Stickstoff-, Schwefel- und/oder Sauerstoffatom enthaltenden, gegebenenfalls mit einem weiteren ankondensierten Phenylring verbundenen (hetero)aromatischen Ring und X eine direkte Bindung oder eine der Gruppen -NH-, -CH₂-, -CO-, -CH₂O-, -CH=CH-, -CH=C(NHCOCH₃)-, -NH-CH₂-, -CH₂-NH-, -S-CH₂-, -O-CH₂-, -CHOH-, -CH(COOH)-, -CH(OSO₃H)- darstellt,
und worin ferner R¹ Wasserstoff und R² die Sulfogruppe -SO₃H darstellt oder R¹ und R² zusammen eine Gruppe der Formel 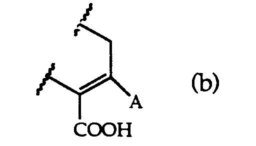 bedeutet, worin A Wasserstoff oder einen in 3-Stellung von Cephalosporinantibiotika verwendbaren Rest darstellt,
und von pharmazeutisch verträglichen Salzen dieser Verbindungen, dadurch gekennzeichnet, dass man entsprechend substituierte Ausgangsverbindungen einsetzt.

3. Verfahren zur Herstellung von Verbindungen gemäss Anspruch 2, worin R t-Butoxycarbonyl darstellt, dadurch gekennzeichnet, dass man entsprechend substituierte Ausgangsverbindungen einsetzt.

4. Verfahren zur Herstellung von Verbindungen gemäss Anspruch 2, und zwar von t-Butoxy-(1S,5R)-7-oxo-6-sulfo-2,6-diazabicyclo[3.2.0]heptan-2-carboxylat und von dessen pharmazeutisch verträglichen Salzen, dadurch gekennzeichnet, dass man entsprechend substituierte Ausgangsverbindungen einsetzt.

5. Verfahren zur Herstellung von Verbindungen gemäss Anspruch 2, und zwar von (1aS,3aR,6bR)-1,1a,3a,6b-Tetrahydro-5-methoxy-1-oxo-2,6a-diazacyclobuta[cd]inden-2,6(3H,4H)-dicarbonsäure-2-t-butylmononoester und von dessen pharmazeutisch verträglichen Salzen, dadurch gekennzeichnet, dass man entsprechend substituierte Ausgangsverbindungen einsetzt.

6. Verfahren zur Herstellung von Verbindungen gemäss Anspruch 2, und zwar von (1S,5R)-7-Oxo-2-(phenylcarbamoyl)-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

7. Verfahren zur Herstellung von Verbindungen gemäss Anspruch 2, und zwar von (1S,5R)-2-(Benzylcarbamoyl)-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

8. Verfahren zur Herstellung von Verbindungen gemäss Anspruch 2, und zwar von (1S,5R)-7-Oxo-2-(N-phenylglycyl)-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

9. Verfahren zur Herstellung von Verbindungen gemäss Anspruch 1, und zwar von (1S,5R)-2-Cyclobutylcarbamoyl-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

10. Verfahren zur Herstellung von Verbindungen gemäss Anspruch 1, und zwar von (1S,5R)-7-Oxo-2-(R,S)-(2-oxo-3-tetrahydrothienyl-carbamoyl)-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

11. Verfahren zur Herstellung von Verbindungen gemäss Anspruch 1, und zwar von (1S,5R)-7-Oxo-2-(R)-(2-oxo-3-tetrahydrothienyl-carbamoyl)-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

12. Verfahren zur Herstellung von Verbindungen gemäss Anspruch 1, und zwar von (1S,5R)-2-(4-Hydroxy-phenylcarbamoyl)-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

13. Verfahren zur Herstellung von Verbindungen gemäss Anspruch 2 der allgemeinen Formel 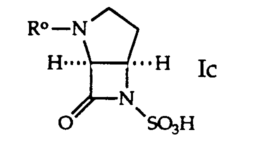 worin R^{o} eine Gruppe der allgemeinen Formel 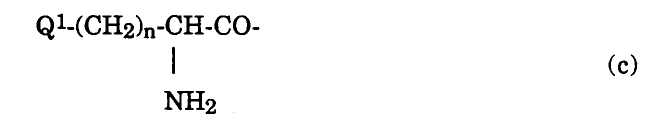 darstellt, und Q¹ einen 5- oder 6-gliedrigen, gegebenenfalls ein Stickstoff-, Schwefel- und/oder Sauerstoffatom enthaltenden, gegebenenfalls mit einem weiteren ankondensierten Phenylring verbundenen (hetero)aromatischen Ring und n die Zahl 0 oder 1 bedeutet,
und von deren pharmazeutisch verträglichen Salzen, dadurch gekennzeichnet, dass man entsprechend substituierte Ausgangsverbindungen einsetzt.

14. Verfahren zur Herstellung von Verbindungen gemäss Anspruch 2, und zwar von (1S,5R)-7-Oxo-2-L-tyrosyl-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

15. Verfahren zur Herstellung von Verbindungen gemäss Anspruch 2, und zwar von (1S,5R)-7-Oxo-2-L-tryptophanyl-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

16. Verfahren zur Herstellung von Verbindungen gemäss Anspruch 2, und zwar von (1S,5R)-2-[(R oder S)-α-Amino-(2-thienyl)acetyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

17. Verfahren zur Herstellung von Verbindungen gemäss Anspruch 1, und zwar von (1S,5R)-2-[DL-2-(2-Amino-4-thiazolyl)glycyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

18. Verfahren zur Herstellung von Verbindungen gemäss Anspruch 13, worin R eine Gruppe der allgemeinen Formel 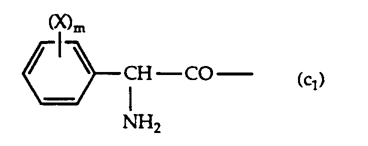 in der X Wasserstoff, Hydroxy oder Fluor und n die Zahl 1 oder 2 darstellt,
bedeutet, dadurch gekennzeichnet, dass man entsprechend substituierte Ausgangsverbindungen einsetzt.

19. Verfahren zur Herstellung von Verbindungen gemäss Anspruch 2, und zwar von (1S,5R)-7-Oxo-2-(D-2-phenylglycyl)-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

20. Verfahren zur Herstellung von Verbindungen gemäss Anspruch 2, und zwar von (1S,5R)-7-Oxo-2-(L-2-phenylglycyl)--2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

21. Verfahren zur Herstellung von Verbindungen gemäss Anspruch 2, und zwar von (1S,5R)-2-[D-2-(p-Hydroxyphenyl)glycyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

22. Verfahren zur Herstellung von Verbindungen gemäss Anspruch 1, und zwar von (1S,5R)-2-[R-2-(m-Hydroxyphenyl)glycyl]]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

23. Verfahren zur Herstellung von Verbindungen gemäss Anspruch 1, und zwar von (1S,5R)-2-[DL-2-(m-Hydroxyphenyl)glycyl-7-oxo-2,6-diazabicyclo[3.2.0]heptan-6-sulfonsäure und von deren pharmazeutisch verträglichen Salzen, dadurch gekennzeichnet, dass man entsprechend substituierte Ausgaugsverbindungen einsetzt.

24. Verfahren zur Herstellung von Verbindungen gemäss Anspruch1, und zwar von (1S,5R)-2-[DL-2-(o-Fluorphenyl)glycyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

25. Verfahren zur Herstellung von Verbindungen gemäss Anspruch 1, und zwar von (1S,5R)-2-[(R,S)-Amino-(3,4-dihydroxy-phenyl)-acetyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

26. Verfahren zur Herstellung von Verbindungen gemäss Anspruch 1, worin R den Rest der Formel Q-X-Y- bedeutet, in welcher X einen der "Spacer"
-O-, -S-, -NH-, -NH-NH-, -CH₂-, -CO-, -CH₂O-, -CH₂CH₂-, -CH=CH-, -CH₂NH-, -S-CH₂-, -SO₂-CH₂-, -O-CH₂-, -S-CH₂CH₂-, -CH₂CH₂-NH-; -CH₂-O-NH-CO-CH₂CH₂-, -CHOH-, -CH(COOH)-, -CH(OSO₃H)-, -CH(OCONH₂)-, -CH[CH(CH₃)₂]-
und Y eine der Gruppen -CO-, CS- und -CONH- darstellt, dadurch gekennzeichnet, dass man entsprechend substituierte Ausgangsverbindungen einsetzt.

27. Verfahren zur Herstellung von Verbindungen gemäss Anspruch 1, worin R den Rest der Formel Q-X-Y- bedeutet, in welcher X einen der "Spacer"
-O-, -NH-, -NH-NH-, -CH₂-, -CH₂O-, -CH₂CH₂-, -CH=CH-, -CH₂NH-, -O-CH₂-, -CH₂CH₂-NH-, -CHOH-, -CH[CH(CH₃)₂]
und Y die Gruppe -SO₂- darstellt, dadurch gekennzeichnet, dass man entsprechend substituierte Ausgangsverbindungen einsetzt.

28. Verfahren zur Herstellung von Verbindungen gemäss Anspruch 1 der allgemeinen Formel 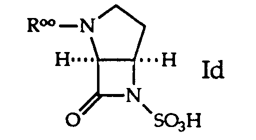 worin R^{oo} eine Gruppe der allgemeinen Formel
Q²-CH=CHCO- (d)
darstellt und Q² einen Phenylring oder einen 5-6-gliedrigen, Stickstoff, Schwefel und/oder Sauerstoff enthaltenden, gegebenenfalls mit einem ankondensierten Phenylring verbundenen heteroaromatischen Ring bedeutet,
und von deren pharmazeutisch verträglichen Salzen, dadurch gekennzeichnet, dass man entsprechend substituierte Ausgangsverbindungen einsetzt.

29. Verfahren zur Herstellung von Verbindungen gemäss Anspruch 2, und zwar von (1S,5R)-2-[(E)-3-(2-Furyl)acryloyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

30. Verfahren zur Herstellung von Verbindungen gemäss Anspruch 2, und zwar von (1S,5R)-2-[(E)-3-(3-Indolyl)acryloyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

31. Verfahren zur Herstellung von Verbindungen gemäss Anspruch 1, und zwar von (1S,5R)-2-[(E)-3-(4-Imidazolyl)acryloyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

32. Verfahren zur Herstellung von Verbindungen gemäss Anspruch 1, und zwar von (1S,5R)-2-[(E)-3-(2-Thinenyl)acryloyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

33. Verfahren zur Herstellung von Verbindungen gemäss Anspruch 1, und zwar von (1S,5R)-(E)-2-[3-4-Hydroxy-phenyl)acryloyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

34. Verfahren zur Herstellung von Verbindungen gemäss Anspruch 1 der allgemeinen Formel 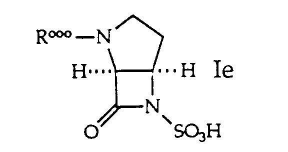 worin R^{ooo} eine Gruppe der allgemeinen Formel
Q³-(S)ₙ-CH₂CO- (e)
darstellt, n 0 oder 1 und Q³ einen Phenylring oder einen 5-6-gliedrigen, gegebenenfalls Schwefel enthaltenden, gegebenenfalls durch C₁-C₇ Alkyl oder Amino substituierten, gegebenenfalls mit einem ankondensierten Phenylring verbundenen N-Heterocyclus bedeutet,
und von deren pharmazeutisch verträglichen Salzen, dadurch gekennzeichnet, dass man entsprechend substituierte Ausgangsverbindungen einsetzt.

35. Verfahren zur Herstellung von Verbindungen gemäss Anspruch 2, und zwar von (1S,5R)-2-[[(1-Methyl-1H-tetrazol-5-yl)thio]acetyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

36. Verfahren zur Herstellung von Verbindungen gemäss Anspruch 2, und zwar von (1S,5R)-2-[[1H-benzotriazol-1-yl)thio]acetyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

37. Verfahren zur Herstellung von Verbindungen gemäss Anspruch 1, und zwar von (1S,5R)-2-[[(5-Methyl-1,3,4-thiadiazol-2-yl)thio]acetyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

38. Verfahren zur Herstellung von Verbindungen gemäss Anspruch 1, und zwar von (1S,5R)-7-Oxo-2-(1H-tetrazol-1-ylacetyl)-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

39. Verfahren zur Herstellung von Verbindungen gemäss Anspruch 1, und zwar von (1S, 5R)-2-[(Isopropyl-2H-tetrazol-5-yl)acetyl]-7-oxo-2,6-diazabicyclo[3.2.0]heptan-6-sulfonsäure und von deren pharmazeutisch verträglichen Salzen, dadurch gekennzeichnet, dass man entsprechend substituierte Ausgangsverbindungen einsetzt.

40. Verfahren zur Herstellung von pharmazeutischen Präparaten, dadurch gekennzeichnet, dass man ein in Anspruch 1 definiertes β-Lactam der Formel I oder ein pharmazeutisch verträgliches Salz dieser Verbindung mit zur therapeutischen Verabreichung geeigneten, nicht-toxischen, inerten, an sich in solchen Präparaten üblichen festen und flüssigen Trägern und/oder Excipientien vermischt.

41. Verfahren nach Anspruch 40, dadurch gekennzeichnet, dass man zusätzlich ein β-Lactam-Antibiotikum beimischt.

42. Verfahren nach Anspruch 41, dadurch gekennzeichnet, dass man als β-Lactam-Antibiotikum ein Penicillin oder Cephalosporin einsetzt.

43. Verfahren nach Anspruch 42, dadurch gekennzeichnet, dass man als β-Lactam-Antibiotikum Ceftriaxon oder eines seiner pharmazeutisch verträglichen Salze einsetzt.

44. Verfahren nach Anspruch 43, dadurch gekennzeichnet, dass man eine in Anspruch 21 definierte Verbindung einsetzt.

45. Verwendung von Verbindungen gemäss einem der Ansprüche 1-39 bei der Herstellung von Arzneimitteln für die Behandlung bzw. Prophylaxe von Infektionskrankheiten.

46. Die Erfindung, im wesentlichen wie sie hiervor beschrieben ist.

47. Verbindungen der allgemeinen Formel 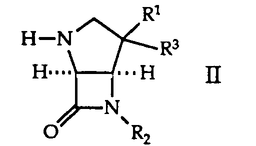 in der R¹, R² und R³ die in Anspruch 1 gegebene Bedeutung haben.

48. Verbindungen der allgemeinen Formel 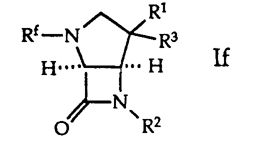 in der R^{1,} R² und R³ die in Anspruch 1 gegebene Bedeutung haben und R^{f} einen unter R in Anspruch 1 definierten Acylrest mit einer geschützten Aminogruppe bzw. Hydroxygruppe(n) darstellt.

49. Verbindungen der allgemeinen Formel 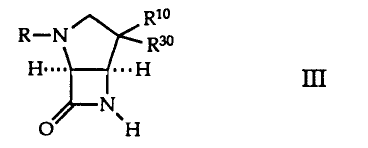 in der R, R¹⁰ und R³⁰ die in Anspruch 1 gegebene Bedeutung haben.

50. Verbindungen der allgemeinen Formel 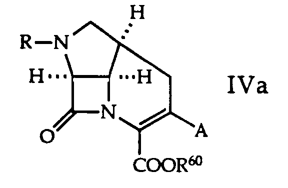 in der R die in Anspruch 1 gegebene Bedeutung hat und R⁶⁰ t-Butyl, p-Nitrobenzyl, Benzyl, Benzhydryl oder Allyl darstellt.

## Claims (Claims for the following Contracting State(s): AT, BE, CH, DE, DK, FR, GB, IT, LI, LU, MC, NL, PT, SE)

1. β-Lactams of the general formula 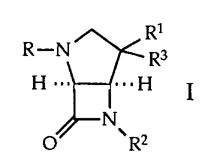 in which R signifies C₁-C₇ alkoxycarbonyl, C₁-C₇ alkoxycarbonylamino, the acyl residue of an α- or β-amino acid or a residue of the general formula
Q-X-Y- (a)
wherein Q signifies a 3-6-membered ring which optionally contains nitrogen, sulphur and/or oxygen and which is optionally bonded with a fused ring, X signifies a direct bond or a linear "spacer" with up to 6 atoms consisting of carbon, nitrogen, oxygen and/or sulphur, of which up to 2 atoms can be nitrogen atoms and 1 atom can be oxygen or sulphur, and Y represents one of the groups -CO-, -CS-, -CONH- and (where X contains neither sulphur nor carbonyl as a terminal component) -SO₂-;
and in which R¹ signifies hydrogen, halogen, carbamoyloxy, C₁-C₇ alkanoyloxy or a group of the formula -S-Het, Het represents a 5-6-membered heteroaromatic group containing nitrogen, sulphur and/or oxygen, and R² represents the sulpho group -SO₃H or R¹ and R² together signify a group of the formula 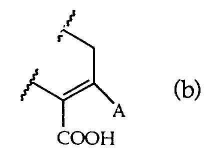 wherein A represents hydrogen or a residue which is usable in the 3-position of cephalosporin antibiotics, and in which R³ represents hydrogen or R¹ and R³ together represent a group of the general formula
=CH-R^{a} (c)
wherein R^{a} signifies one of the groups 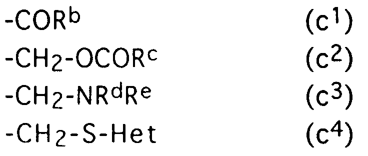 in which R^{b} represents C₁-C₇ alkoxy or amino, R^{c} represents C₁-C₇ alkyl, phenyl or amino, R^{d} and R^{e} each represent hydrogen or C₁-C₇ alkyl or together with the N atom represent a 5- or 6-membered N-heterocycle which optionally contains a further nitrogen, oxygen or sulphur atom and Het represents a 5-6-membered heteroaromatic group containing nitrogen, sulphur and/or oxygen,
and pharmaceutically compatible salts of these compounds.

2. Compounds in accordance with claim 1 of the general formula 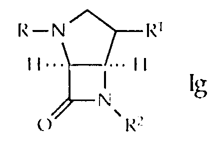 in which R signifies C₁-C₇ alkoxycarbonyl, the acyl residue of an α-amino acid or a (hetero)aromatic acyl group of the general formula
Ar-X-CO- (aa)
wherein Ar represents a 5- or 6-membered (hetero)aromatic ring which optionally contains a nitrogen, sulphur and/or oxygen atom and which is optionally bonded with a further fused phenyl ring and X represents a direct bond or one of the groups -NH-, -CH₂-, -CO-, -CH₂O-, -CH=CH-, -CH=C(NHCOCH₃)-, -NH-CH₂-, -CH₂-NH-, -S-CH₂-, -O-CH₂-, -CHOH-, -CH(COOH)- and -CH(OSO₃H)-, and in which R¹ represents hydrogen and R² represents the sulpho group -SO₃H or R¹ and R² together signify a group of the formula 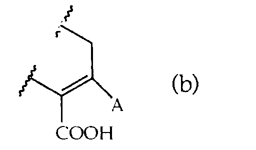 wherein A represents hydrogen or a residue which is usable in the 3-position of cephalosorin antibiotics,
and pharmaceutically compatible salts of these compounds.

3. Compounds in accordance with claim 2, wherein R represents t-butoxycarbonyl.

4. t-Butoxy (1S,5R)-7-oxo-6-sulpho-2,6-diazabicyclo[3.2.0]heptane-2-carboxylate and its pharmaceutically compatible salts.

5. (1aS,3aR,6bR)-1,1a,3a,6b-Tetrahydro-5-methoxy-1-oxo-2,6a-diazacyclobuta[cd]indene-2,6(3H,4H)-dicarboxylic acid 2-t-butyl monoester and its pharmaceutically compatible salts.

6. (1S,5R)-7-Oxo-2-(phenylcarbamoyl)-2,6-diazabicyclo[3.2.0]heptane-6-sulphonic acid and its pharmaceutically compatible salts.

7. (1S,5R)-2-(Benzylcarbamoyl)-7-oxo-2,6-diazabicyclo[3.2.0]heptane-6-suiphonic acid and its pharmaceutically compatible salts.

8. (1S,5R)-7-Oxo-2-(N-phenylglycyl)-2,6-diazabicyclo[3.2.0]heptane-6-sulphonic acid and its pharmaceutically compatible salts.

9. (1S,5R)-2-Cyclobutylcarbamoyl-7-oxo-2,6-diazabicyclo[3.2.0]heptane-6-sulphonic acid and its pharmaceutically compatible salts.

10. (1S,5R)-7-Oxo-2-(R,S)-(2-oxo-3-tetrahydro-thienylcarbamoyl)-2,6-diazabicyclo[3.2.0]heptane-6-sulphonic acid and its pharmaceutically compatible salts.

11. (1S,5R)-7-Oxo-2-(R)-(2-oxo-3-tetrahydrothienylcarbamoyl)-2,6-diazabicyclo[3.2.0]heptane-6-sulphonic acid and its pharmaceutically compatible salts.

12. (1S,5R)-2-(4-Hydroxy-phenylcarbamoyl)-7-oxo-2,6-diazabicyclo[3.2.0]heptane-6-sulphonic acid and its pharmaceutically compatible salts.

13. Compounds in accordance with claim 2 of the general formula 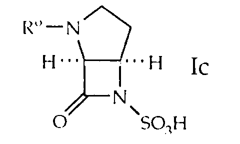 in which R^{o} represents a group of the general formula 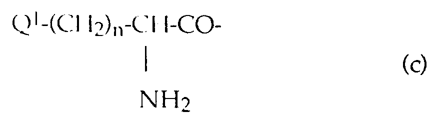 wherein Q¹ signifies a 5- or 6-membered (hetero)aromatic ring which optionally contains a nitrogen, sulphur and/or oxygen atom and which is optionally bonded with a further fused phenyl ring and n signifies the number 0 or 1,
and their pharmaceutically compatible salts.

14. (1S,5R)-7-Oxo-2-L-tyrosyl-2,6-diazabicyclo[3.2.0]heptane-6-sulphonic acid and its pharmaceutically compatible salts.

15. (1S,5R)-7-Oxo-2-L-tryptophanyl-2,6-diazabicyclo[3.2.0]heptane-6-sulphonic acid and its pharmaceutically compatible salts.

16. (1S,5R)-2-[(R or S)-α-Amino-(2-thienyl)acetyl]-7-oxo-2,6-diazabicyclo[3.2.0]heptane-6-sulphonic acid and its pharmaceutically compatible salts.

17. (1S,5R)-2-[DL-2-(2-Amino-4-thiazolyl)glycyl]-7-oxo-2,6-diazabicyclo[3.2.0]heptane-6-sulphonic acid and its pharmaceutically compatible salts.

18. Compounds in accordance with claim 13, wherein R^{o} signifies a group of the general formula 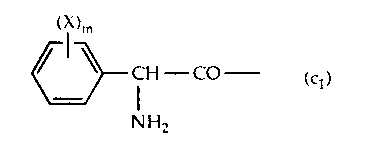 in which X represents hydrogen, hydroxy or fluorine and m represents the number 1 or 2.

19. (1S,5R)-7-Oxo-2-(D-2-phenylglycyl)-2,6-diazabicyclo[3.2.0]heptane-6-sulphonic acid and its pharmaceutically compatible salts.

20. (1S,5R)-7-Oxo-2-(L-2-phenylglycyl)-2,6-diazabicyclo[3.2.0]heptane-6-sulphonic acid and its pharmaceutically compatible salts.

21. (1S,5R)-2-[D-2-(p-Hydroxyphenyl)glycyl]-7-oxo-2,6-diazabicyclo[3.2.0]heptane-6-sulphonic acid and its pharmaceutically compatible salts.

22. (1S,5R)-2-[R-2-(m-Hydroxyphenyl)glycyl]-7-oxo-2,6-diazabicyclo[3.2.0]heptane-6-sulphonic acid and its pharmaceutically compatible salts.

23. (1S,5R)-2-[DL-2-(m-Hydroxyphenyl)glycyl]-7-oxo-2,6-diazabicyclo[3.2.0]heptane-6-sulphonic acid and its pharmaceutically compatible salts.

24. (1S,5R)-2-[DL-2-(o-Fluorophenyl)glycyl]-7-oxo-2,6-diazabicyclo[3.2.0]heptane-6-sulphonic acid and its pharmaceutically compatible salts.

25. (1S,5R)-2-[(R,S)-Amino-(3,4-dihydroxyphenyl)acetyl]-7-oxo-2,6-diazabicyclo[3.2.0]heptane-6-sulphonic acid and its pharmaceutically compatible salts.

26. Compounds in accordance with claim 1, wherein R signifies the residue of the formula Q-X-Y- in which X represents one of the "spacers" -O-, -S-, -NH-, -NH-NH-, -CH₂-, -CO-, -CH₂O-, -CH₂CH₂-, -CH=CH-, -CH₂NH-, -S-CH₂-, -SO₂-CH₂-, -O-CH₂-, -S-CH₂CH₂-, -CH₂CH₂-NH-; -CH₂-O-NH-CO-CH₂CH₂-, -CHOH-, -CH(COOH)-, -CH(OSO₃H)-, -CH(OCONH₂)- and -CH[CH(CH₃)₂]- and Y represents one of the groups -CO-, -CS- and -CONH-.

27. Compounds in accordance with claim 1, wherein R signifies the residue of the formula Q-X-Y- in which X represents one of the "spacers" -O-, -NH-, -NH-NH-, -CH₂-, -CH₂O-, -CH₂CH₂-, -CH=CH-, -CH₂NH-, -O-CH₂-, -CH₂CH₂-NH-, -CHOH- and -CH[CH(CH₃)₂] and Y represents the -SO₂- group.

28. Compounds in accordance with claim 1 of the general formula 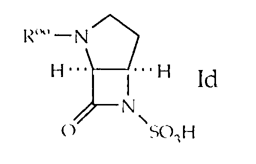 in which R^{oo} represents a group of the general formula
Q²-CH=CHCO- (d)
wherein Q² signifies a phenyl ring or a 5-6-membered heteroaromatic ring which contains nitrogen, sulphur and/or oxygen and which is optionally bonded with a fused phenyl ring,
and their pharmaceutically compatible salts.

29. (1S,5R)-2-[(E)-3-(2-Furyl)acryloyl]-7-oxo-2,6-diazabicyclo[3.2.0]heptane-6-sulphonic acid and its pharmaceutically compatible salts.

30. (1S,5R)-2-[(E)-3(3-Indolyl)acryloyl]-7-oxo-2,6-diazabicyclo[3.2.0]heptane-6-sulphonic acid and its pharmaceutically compatible salts.

31. (1S,5R)-2-[(E)-3-(4-Imidazolyl)acryloyl]-7-oxo-2,6-diazabicyclo[3.2.0]heptane-6-sulphonc acid and its pharmaceutically compatible salts.

32. (1S,5R)-2-[(E)-3-(2-Thienyl)acryloyl]-7-oxo-2,6-diazabicyclo[3.2.0]heptane-6-sulphonic acid and its pharmaceutically compatible salts.

33. (1S,5R)-(E)-2-[3-4-Hydroxy-phenyl)acryloyl]-7-oxo-2,6-diazabicyclo[3.2.0]heptane-6-sulphonic acid and its pharmaceutically compatible salts.

34. Compounds in accordance with claim 1 of the general formula 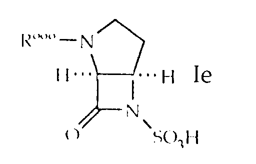 in which R^{ooo} represents a group of the general formula
Q³(S)ₙ-CH₂CO- (e)
wherein n signifies 0 or 1 and Q³ signifies a phenyl ring or a 5-6-membered N-heterocycle which optionally contains sulphur, which is optionally substituted by C₁-C₇ alkyl or amino and which is optionally bonded with a fused phenyl ring,
and their pharmaceutically compatible salts.

35. (1S,5R)-2-[[(1-Methyl-1H-tetrazol-5-yl)thio]acetyl]-7-oxo-2,6-diazabicyclo[3.2.0]heptane-6-sulphonic acid and its pharmaceutically compatible salts.

36. (1S,5R)-2-[[1H-Benzotriazol-1-yl)thio]acetyl]-7-oxo-2,6-diazabicyclo[3.2.0]heptane-6-sulphonic acid and its pharmaceutically compatible salts.

37. (1S,5R)-2-[[(5-Methyl-1,3,4-thiadiazol-2-yl)thio]acetyl]-7-oxo-2,6-diazabicyclo[3.2.0]heptane-6-sulphonic acid and its pharmaceutically compatible salts.

38. (1S,5R)-7-Oxo-2-(1H-tetrazol-1-ylacetyl)-2,6-diazabicyclo[3.2.0]heptane-6-sulphonic acid and its pharmaceutically compatible salts.

39. (1S,5R)-2-[(2-Isopropyl-2H-tetrazol-5-yl)-acetyl]-7-oxo-2,6-diazabicyclo[3.2.0]heptane-6-sulphonic acid and its pharmaceutically compatible salts.

40. Compounds of the general formula 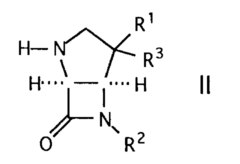 wherein R¹, R² and R³ have the significance given in claim 1.

41. Compounds of the general formula 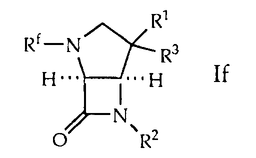 wherein R¹, R² and R³ have the significance given in claim 1 and R^{f} represents a residue defined under R in claim 1 having a protected amino group or hydroxy group(s).

42. Compounds of the general formula 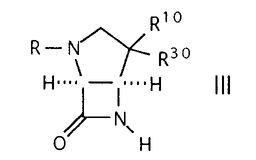 wherein R has the significance given in claim 1 and R¹⁰ and R³⁰ have the significances given in claim 46.

43. Compounds of the general formula 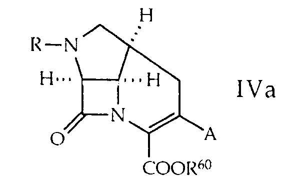 wherein R has the significance given in claim 1 and R⁶⁰ represents t-butyl, p-nitrobenzyl, benzyl, benzhydryl or allyl.

44. Compounds in accordance with any one of claims 1 to 39 as pharmaceutically active substances.

45. Compounds in accordance with any one of claims 1 to 39 as active substances which inhibit β-lactamase.

46. A process for the manufacture of compounds in accordance with any one of claims 1 to 39, characterized by
(a) treating a compound of the general formula 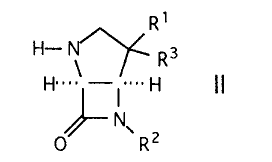 in which R¹, R² and R³ have the significance given in claim 1,
with agents yielding the residue R, or
(b) for the manufacture of a compound of formula I in which R contains a free amino group or hydroxy group(s), cleaving off the amino protecting group or hydroxy protecting group(s) in a compound of the general formula 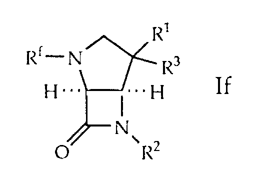 in which R¹, R² and R³ have the significance given in claim 1 and R^{f} represents a residue defined under R having a protected amino group or hydroxy group(s),
or
(c) for the manufacture of a compound of formula I in which R¹ and R² are different from group (b), treating a compound of the general formula 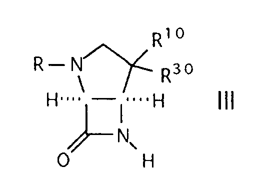 in which R has the significance given in claim 1, R¹⁰ represents hydrogen, halogen, carbamoyloxy, C₁-C₇ alkanoyloxy or a group of the formula -S-Het, Het represents a 5-6-membered heteroaromatic group, and R³⁰ represents hydrogen or R¹⁰ and R³⁰ together represent a group of the general formula
=CH-R^{a} (c)
wherein R^{a} signifies one of the groups 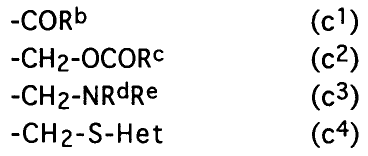 in which R^{b} represents C₁-C₇ alkoxy or amino, R^{c} represents C₁-C₇ alkyl, phenyl or amino, R^{d} and R^{e} each represent hydrogen or C₁-C₇ alkyl or together with the N atom represent a 5- or 6-membered saturated N-heterocycle which optionally contains a further nitrogen, oxygen or sulphur atom and Het represents a 5-6-membered heteroaromatic group containing nitrogen, sulphur and/or oxygen,
with agents yielding the sulpho group -SO₃H, or
(d) for the manufacture of a compound of formula I in which R¹ and R² together represent the group (b) defined above, reacting a compound of the general formula 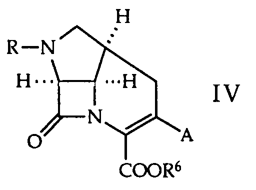 in which R and A have the significances given in claim 1 and R⁶ represents a carboxylic acid protecting group,
with agents which cleave the carboxylic acid protecting group R⁶, or
(e) for the manufacture of a pharmaceutically compatible salt of a compound of formula I, converting a compound of formula I into such a salt.

47. A medicament containing a compound in accordance with any one of claims 1 to 39.

48. A β-lactamase inhibiting medicament containing a compound in accordance with any one of claims 1 to 39.

49. A medicament in accordance with claim 47 or 48 containing a β-lactam antibiotic.

50. A medicament in accordance with claim 49 containing a penicillin or cephalosporin as the β-lactam antibiotic.

51. A medicament in accordance with claim 50 containing benzylpenicillin, piperacillin, phenoxymethylpenicillin, carbenicillin, methicillin, propicillin, tricarcillin, ampicillin, amoxicillin, mecillinam, ceftazidime, cefetamet, cefatamet pivoxil, cefotaxime, cefmenoxime, ceftizoxime, cefuroxime, cephaloridine, cephalotin, cefazolin, cephalexin, cefoxitin, cephacetrile, cefamandole, cephapirin, cephradine, cephaloglycine, (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-ene-2-carboxylic acid or (E)-2-(isobutoxycarbonyl)-2-pentenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-ene-2-carboxylate or a pharmaceutically compatible salt of one of these compounds.

52. A medicament in accordance with claim 50 containing ceftriaxone or one of its pharmaceutically compatible salts.

53. A medicament in accordance with claim 52 containing a compound in accordance with claim 21.

54. A medicament in accordance with any one of claims 47-53 as a combination preparation for the simultaneous, separate or chronologically stepwise use in antibacterial therapy.

55. The use of compounds in accordance with any one of claims 1-39 for the production of antibacterially-active medicaments.

## Claims (Claims for the following Contracting State(s): ES, GR)

1. A process for the manufacture of β-lactams of the general formula 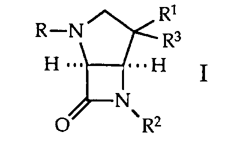 in which R signifies C₁-C₇ alkoxycarbonyl, C₁-C₇ alkoxycarbonylamino, the acyl residue of an α- or β-amino acid or a residue of the general formula
Q-X-Y- (a)
wherein Q signifies a 3-6-membered ring which optionally contains nitrogen, sulphur and/or oxygen and which is optionally bonded with a fused ring, X signifies a direct bond or a linear "spacer" with up to 6 atoms consisting of carbon, nitrogen, oxygen and/or sulphur, of which up to 2 atoms can be nitrogen atoms and 1 atom can be oxygen or sulphur, and Y represents one of the groups -CO-, -CS-, -CONH- and (where X contains neither sulphur nor carbonyl as a terminal component) -SO₂-;
and in which R¹ signifies hydrogen, halogen, carbamoyloxy, C₁-C₇ alkanoyloxy or a group of the formula -S-Het, Het represents a 5-6-membered heteroaromatic group containing nitrogen, sulphur and/or oxygen, and R² represents the sulpho group -SO₃H or R¹ and R² together signify a group of the formula 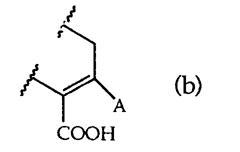 wherein A represents hydrogen or a residue which is usable in the 3-position of cephalosporin antibiotics, and in which R³ represents hydrogen or R¹ and R³ together represent a group of the general formula
=CH-R^{a} (c)
wherein R^{a} signifies one of the groups 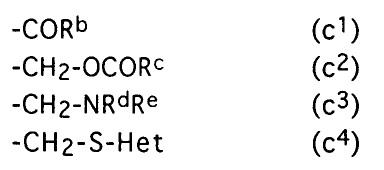 in which R^{b} represents C₁-C₇ alkoxy or amino, R^{c} represents C₁-C₇ alkyl, phenyl or amino, R^{d} and R^{e} each represent hydrogen or C₁-C₇ alkyl or together with the N atom represent a 5- or 6-membered N-heterocycle which optionally contains a further nitrogen, oxygen or sulphur atom and Het represents a 5-6-membered heteroaromatic group containing nitrogen, sulphur and/or oxygen,
and of pharmaceutically compatible salts of these compounds,
characterized by
(a) treating a compound of the general formula 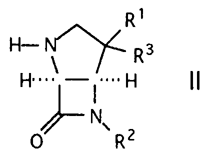 in which R¹, R² and R³ have the significance given above, with agents yielding the residue R, or
(b) for the manufacture of a compound of formula I in which R contains a free amino group or hydroxy group(s), cleaving off the amino protecting group or hydroxy protecting group(s) in a compound of the general formula 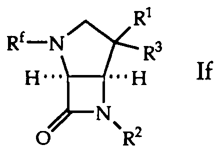 in which R¹, R² and R³ have the significance given above and R^{f} represents a residue defined under R having a protected amino group or hydroxy group(s),
or
(c) for the manufacture of a compound of formula I in which R¹ and R² are different from group (b), treating a compound of the general formula 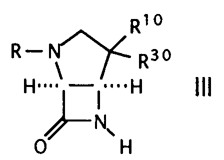 in which R has the significance given above, R¹⁰ represents hydrogen, halogen, carbamoyloxy, C₁-C₇ alkanoyloxy or a group of the formula -S-Het, Het represents a 5-6-membered heteroaromatic group, and R³⁰ represents hydrogen or R¹⁰ and R³⁰ together represent a group of the general formula
=CH-R^{a} (c)
wherein R^{a} signifies one of the groups 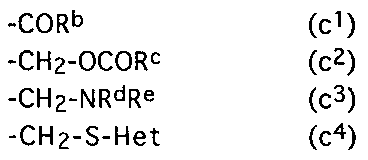 in which R^{b} represents C₁-C₇ alkoxy or amino, R^{c} represents C₁-C₇ alkyl, phenyl or amino, R^{d} and R^{e} each represent hydrogen or C₁-C₇ alkyl or together with the N atom represent a 5- or 6-membered saturated N-heterocycle which optionally contains a further nitrogen, oxygen or sulphur atom and Het represents a 5-6-membered hetero-aromatic group containing nitrogen, sulphur and/or oxygen,
with agents yielding the sulpho group -SO₃H, or
(d) for the manufacture of a compound of formula I in which R¹ and R² together represent the group (b) defined above, reacting a compound of the general formula 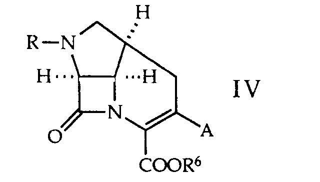 in which R and A have the significances given above and R⁶ represents a carboxylic acid protecting group,
with agents which cleave the carboxylic acid protecting group R⁶, or
(e) for the manufacture of a pharmaceutically compatible salt of a compound of formula I, converting a compound of formula I into such a salt.

2. A process for the manufacture of compounds in accordance with claim 1 of the general formula 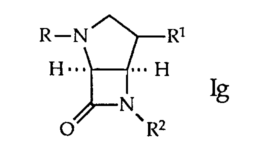 in which R signifies C₁-C₇ alkoxycarbonyl, the acyl residue of an α-amino acid or a (hetero)aromatic acyl group of the general formula
Ar-X-CO- (aa)
wherein Ar represents a 5- or 6-membered (hetero)aromatic ring which optionally contains a nitrogen, sulphur and/or oxygen atom and which is optionally bonded with a further fused phenyl ring and X represents a direct bond or one of the groups -NH-, -CH₂-, -CO-, -CH₂O-, -CH=CH-, -CH=C(NHCOCH₃)-, -NH-CH₂-, -CH₂-NH-, -S-CH₂-, -O-CH₂-, -CHOH-, -CH(COOH)- and -CH(OSO₃H)-, and in which R¹ represents hydrogen and R² represents the sulpho group -SO₃H or R¹ and R² together signify a group of the formula 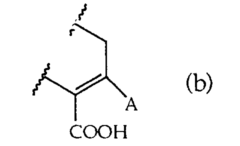 wherein A represents hydrogen or a residue which is usable in the 3-position of cephalosorin antibiotics,
and of pharmaceutically compatible salts of these compounds, characterized in that correspondingly substituted starting compounds are used.

3. A process for the manufacture of compounds in accordance with claim 2, wherein R represents t-butoxycarbonyl, characterized in that correspondingly substituted starting compounds are used.

4. A process for the manufacture of compounds in accordance with claim 2, namely of t-butoxy (1S,5R)-7-oxo-6-sulpho-2,6-diazabicyclo-[3.2.0]heptane-2-carboxylate and of its pharmaceutically compatible salts, characterized in that correspondingly substituted starting compounds are used.

5. A process for the manufacture of compounds in accordance with claim 2, namely of (1aS,3aR,6bR)-1,1a,3a,6b-tetrahydro-5-methoxy-1-oxo-2,6a-diazacyclobuta[cd]indene-2,6(3H,4H)-dicarboxylic acid 2-t-butyl monoester and of its pharmaceutically compatible salts, characterized in that correspondingly substituted starting compounds are used.

6. A process for the manufacture of compounds in accordance with claim 2, namely of (1S,5R)-7-oxo-2-(phenylcarbamoyl)-2,6-diazabicyclo[3.2.0]heptane-6-sulphonic acid and of its pharmaceutically compatible salts, characterized in that correspondingly substituted starting compounds are used.

7. A process for the manufacture of compounds in accordance with claim 2, namely of (1S,5R)-2-(benzylcarbamoyl)-7-oxo-2,6-diazabicyclo[3.2.0]heptane-6-sulphonic acid and of its pharmaceutically compatible salts, characterized in that correspondingly substituted starting compounds are used.

8. A process for the manufacture of compounds in accordance with claim 2, namely of (1S,5R)-7-Oxo-2-(N-phenylglycyl)-2,6-diazabicyclo[3.2.0]heptane-6-sulphonic acid and of its pharmaceutically compatible salts, characterized in that correspondingly substituted starting compounds are used.

9. A process for the manufacture of compounds in accordance with claim 1, namely of (1S,5R)-2-cyclobutylcarbamoyl-7-oxo-2,6-diazabicyclo[3.2.0]heptane-6-sulphonic acid and of its pharmaceutically compatible salts, characterized in that correspondingly substituted starting compounds are used.

10. A process for the manufacture of compounds in accordance with claim 1, namely of (1S,5R)-7-oxo-2-(R,S)-(2-oxo-3-tetrahydro-thienyl-carbamoyl)-2,6-diazabicyclo[3.2.0]heptane-6-sulphonic acid and of its pharmaceutically compatible salts, characterized in that correspondingly substituted starting compounds are used.

11. A process for the manufacture of compounds in accordance with claim 1, namely of (1S,5R)-7-oxo-2-(R)-(2-oxo-3-tetrahydrothienyl-carbamoyl)-2,6-diazabicyclo[3.2.0]heptane-6-sulphonic acid and of its pharmaceutically compatible salts, characterized in that correspondingly substituted starting compounds are used.

12. A process for the manufacture of compounds in accordance with claim 1, namely of (1S,5R)-2-(4-hydroxyphenylcarbamoyl)-7-oxo-2,6-diazabicyclo[3.2.0]heptane-6-sulphonic acid and of its pharmaceutically compatible salts, characterized in that correspondingly substituted starting compounds are used.

13. A process for the manufacture of compounds in accordance with claim 2 of the general formula 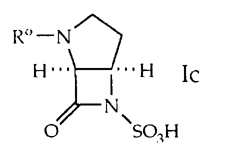 in which R^{o} represents a group of the general formula 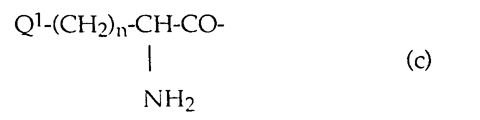 wherein Q¹ signifies a 5- or 6-membered (hetero)aromatic ring which optionally contains a nitrogen, sulphur and/or oxygen atom and which is optionally bonded with a further fused phenyl ring and n signifies the number 0 or 1,
and of their pharmaceutically compatible salts, characterized in that correspondingly substituted starting compounds are used.

14. A process for the manufacture of compounds in accordance with claim 2, namely of (1S,5R)-7-oxo-2-L-tyrosyl-2,6-diazabicyclo[3.2.0]heptane-6-sulphonic acid and of its pharmaceutically compatible salts, characterized in that correspondingly substituted starting compounds are used.

15. A process for the manufacture of compounds in accordance with claim 2, namely of (1S,5R)-7-oxo-2-L-tryptophanyl-2,6-diazabicyclo[3.2.0]heptane-6-sulphonic acid and of its pharmaceutically compatible salts, characterized in that correspondingly substituted starting compounds are used.

16. A process for the manufacture of compounds in accordance with claim 2, namely of (1S,5R)-2-[(R or S)-α-amino(2-thienyl)acetyl]-7-oxo-2,6-diazabicyclo[3.2.0]heptane-6-sulphonic acid and of its pharmaceutically compatible salts, characterized in that correspondingly substituted starting compounds are used.

17. A process for the manufacture of compounds in accordance with claim 1, namely of (1S,5R)-2-[DL-2-(2-amino-4-thiazolyl)glycyl]-7-oxo-2,6-diazabicyclo[3.2.0]heptane-6-sulphonic acid and of its pharmaceutically compatible salts, characterized in that correspondingly substituted starting compounds are used.

18. A process for the manufacture of compounds in accordance with claim 13, wherein R^{o} signifies a group of the general formula 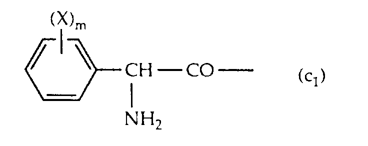 in which X represents hydrogen, hydroxy or fluorine and m represents the number 1 or 2,
characterized in that correspondingly substituted starting compounds are used.

19. A process for the manufacture of compounds in accordance with claim 2, namely of (1S,5R)-7-oxo-2-(D-2-phenylglycyl)-2,6-diazabicyclo[3.2.0]heptane-6-sulphonic acid and of its pharmaceutically compatible salts, characterized in that correspondingly substituted starting compounds are used.

20. A process for the manufacture of compounds in accordance with claim 2, namely of (1S,5R)-7-oxo-2-(L-2-phenylglycyl)-2,6-diazabicyclo[3.2.0]heptane-6-sulphonic acid and of its pharmaceutically compatible salts, characterized in that correspondingly substituted starting compounds are used.

21. A process for the manufacture of compounds in accordance with claim 2, namely of (1S,5R)-2-[D-2-(p-hydroxyphenyl)glycyl]-7-oxo-2,6-diazabicyclo[3.2.0]heptane-6-sulphonic acid and of its pharmaceutically compatible salts, characterized in that correspondingly substituted starting compounds are used.

22. A process for the manufacture of compounds in accordance with claim 1, namely of (1S,5R)-2-[R-2-(m-hydroxyphenyl)glycyl]-7-oxo-2,6-diazabicyclo[3.2.0]heptane-6-sulphonic acid and of its pharmaceutically compatible salts, characterized in that correspondingly substituted starting compounds are used.

23. A process for the manufacture of compounds in accordance with claim 1, namely of (1S,5R)-2-[DL-2-(m-hydroxyphenyl)glycyl]-7-oxo-2,6-diazabicyclo[3.2.0]heptane-6-sulphonic acid and of its pharmaceutically compatible salts, characterized in that correspondingly substituted starting compounds are used.

24. A process for the manufacture of compounds in accordance with claim 1, namely of (1S,5R)-2-[DL-2-(o-fluorophenyl)glycyl]-7-oxo-2,6-diazabicyclo[3.2.0]heptane-6-sulphonic acid and of its pharmaceutically compatible salts, characterized in that correspondingly substituted starting compounds are used.

25. A process for the manufacture of compounds in accordance with claim 1, namely of (1S,5R)-2-[(R,S)-amino-(3,4-dihydroxy-phenyl)-acetyl]-7-oxo-2,6-diazabicyclo[3.2.0]heptane-6-sulphonic acid and of its pharmaceutically compatible salts, characterized in that correspondingly substituted starting compounds are used.

26. A process for the manufacture of compounds in accordance with claim 1, wherein R signifies the residue of the formula Q-X-Y- in which X represents one of the "spacers" -O-, -S-, -NH-, -NH-NH-, -CH₂-, -CO-, -CH₂O-, -CH₂CH₂-, -CH=CH-, -CH₂NH-, -S-CH₂-, -SO₂-CH₂-, -O-CH₂-, -S-CH₂CH₂-, -CH₂CH₂-NH-; -CH₂-O-NH-CO-CH₂CH₂-, -CHOH-, -CH(COOH)-, -CH(OSO₂H)-, -CH(OCONH₂)- and -CH[CH(CH₃)₂]- and Y represents one of the groups -CO-, -CS- and -CONH-, characterized in that correspondingly substituted starting compounds are used.

27. A process for the manufacture of compounds in accordance with claim 1, wherein R signifies the residue of the formula Q-X-Y- in which X represents one of the "spacers" -O-, -NH-, -NH-NH-, -CH₂-, -CH₂O-, -CH₂CH₂-, -CH=CH-, -CH₂NH-, -O-CH₂-, -CH₂CH₂-NH-, -CHOH- and -CH[CH(CH₃)₂] and Y represents the -SO₂- group, characterized in that correspondingly substituted starting compounds are used.

28. A process for the manufacture of compounds in accordance with claim 1 of the general formula 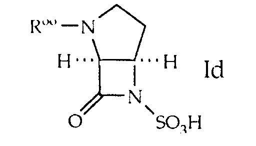 in which R^{oo} represents a group of the general formula
Q²-CH=CHCO- (d)
wherein Q² signifies a phenyl ring or a 5-6-membered heteroaromatic ring which contains nitrogen, sulphur and/or oxygen and which is optionally bonded with a fused phenyl ring,
and of their pharmaceutically compatible salts, characterized in that correspondingly substituted starting compounds are used.

29. A process for the manufacture of compounds in accordance with claim 2, namely of (1S,5R)-2-[(E)-3-(2-furyl)acryloyl]-7-oxo-2,6-diazabicyclo[3.2.0]heptane-6-sulphonic acid and of its pharmaceutically compatible salts, characterized in that correspondingly substituted starting compounds are used.

30. A process for the manufacture of compounds in accordance with claim 2, namely of (1S,5R)-2-[(E)-3-(3-indolyl)acryloyl]-7-oxo-2,6-diazabicyclo[3.2.0]heptane-6-sulphonic acid and of its pharmaceutically compatible salts, characterized in that correspondingly substituted starting compounds are used.

31. A process for the manufacture of compounds in accordance with claim 1, namely of (1S,5R)-2-[(E)-3-(4-imidazolyl)acryloyl]-7-oxo-2,6-diazabicyclo[3.2.0]heptane-6-sulphonic acid and of its pharmaceutically compatible salts, characterized in that correspondingly substituted starting compounds are used.

32. A process for the manufacture of compounds in accordance with claim 1, namely of (1S,5R)-2-[(E)-3-(2-thienyl)acryloyl]-7-oxo-2,6-diazabicyclo[3.2.0]heptane-6-sulphonic acid and of its pharmaceutically compatible salts, characterized in that correspondingly substituted starting compounds are used.

33. A process for the manufacture of compounds in accordance with claim 1, namely of (1S,5R)-(E)-2-[3-4-hydroxyphenyl)acryloyl]-7-oxo-2,6-diazabicyclo[3.2.0]heptane-6-sulphonic acid and of its pharmaceutically compatible salts, characterized in that correspondingly substituted starting compounds are used.

34. A process for the manufacture of compounds in accordance with claim 1 of the general formula 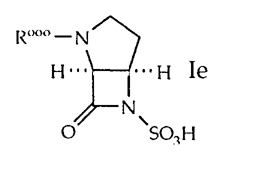 in which R^{ooo} represents a group of the general formula
Q³(S)ₙ-CH₂CO- (e)
wherein n signifies 0 or 1 and Q³ signifies a phenyl ring or a 5-6-membered N-heterocycle which optionally contains sulphur, which is optionally substituted by C₁-C₇ alkyl or amino and which is optionally bonded with a fused phenyl ring,
and of their pharmaceutically compatible salts, characterized in that correspondingly substituted starting compounds are used.

35. A process for the manufacture of compounds in accordance with claim 2, namely of (1S,5R)-2-[[(1-methyl-1H-tetrazol-5-yl)thio]acetyl]-7-oxo-2,6-diazabicyclo[3.2.0]heptane-6-sulphonic acid and of its pharmaceutically compatible salts, characterized in that correspondingly substituted starting compounds are used.

36. A process for the manufacture of compounds in accordance with claim 2, namely of (1S,5R)-2-[[1H-benzotriazol-1-yl)thio]acetyl]-7-oxo-2,6-diazabicyclo[3.2.0]heptane-6-sulphonic acid and of its pharmaceutically compatible salts, characterized in that correspondingly substituted starting compounds are used.

37. A process for the manufacture of compounds in accordance with claim 1, namely of (1S,5R)-2-[[(5-methyl-1,3,4-thiadiazol-2-yl)thio]acetyl]-7-oxo-2,6-diazabicyclo[3.2.0]heptane-6-sulphonic acid and of its pharmaceutically compatible salts, characterized in that correspondingly substituted starting compounds are used.

38. A process for the manufacture of compounds in accordance with claim 1, namely of (1S,5R)-7-oxo-2-(1H-tetrazol-1-ylacetyl)-2,6-diazabicyclo[3.2.0]heptane-6-sulphonic acid and of its pharmaceutically compatible salts, characterized in that correspondingly substituted starting compounds are used.

39. A process for the manufacture of compounds in accordance with claim 1, namely of (1S,5R)-2-[(2-isopropyl-2H-tetrazol-5-yl)-acetyl]-7-oxo-2,6-diazabicyclo[3.2.0]heptane-6-sulphonic acid and of its pharmaceutically compatible salts, characterized in that correspondingly substituted starting compounds are used.

40. A process for the production of pharmaceutical preparations, characterized by mixing a β-lactam of formula I defined in claim 1 or a pharmaceutically compatible salt of this compound with non-toxic, inert, solid and liquid carriers and/or excipients which are usual in such preparations and which are suitable for therapeutic administration.

41. A process according to claim 40, characterized in that a β-lactam antibiotic is also admixed.

42. A process according to claim 41, characterized in that a penicillin or cephalosporin is used as the β-lactam antibiotic.

43. A process according to claim 42, characterized in that ceftriaxone or one of its pharmaceutical compatible salts is used.

44. A process according to claim 43, characterized in that a compound defined in claim 21 is used.

45. The use of compounds according to any one of claims 1-39 for the production of medicaments for the treatment or prophylaxis of infectious diseases.

46. The invention, substantially as hereinbefore described.

47. Compounds of the general formula 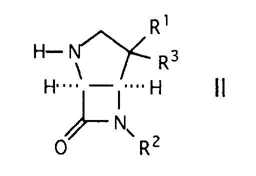 wherein R¹, R² and R³ have the significance given in claim 1.

48. Compounds of the general formula 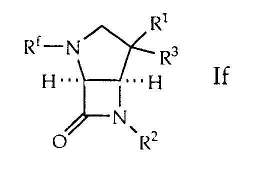 wherein R¹, R² and R³ have the significance given in claim 1 and R^{f} represents an acyl residue defined under R in claim 1 having a protected amino group or hydroxy group(s).

49. Compounds of the general formula 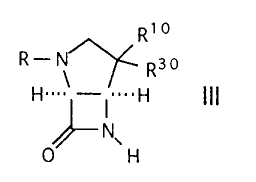 wherein R, R¹⁰ and R³⁰ have the significance given in claim 1.

50. Compounds of the general formula 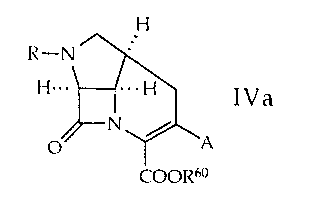 wherein R has the significance given in claim 1 and R⁶⁰ represents t-butyl, p-nitrobenzyl, benzyl, benzhydryl or allyl.

## Revendications (Revendications pour l'(les) Etat(s) contractant(s) suivant(s): AT, BE, CH, DE, DK, FR, GB, IT, LI, LU, MC, NL, PT, SE)

1. β-lactames de formule générale 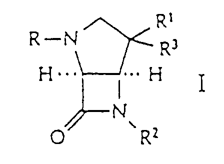 dans laquelle R représente un groupe alcoxy(C₁-C₇)carbonyle, alcoxy(C₁-C₇)carbonylamino, le reste acyle d'un α- ou β-aminoacide ou un radical de formule générale
Q-X-Y- (a)
dans laquelle Q représente un cycle à 3-6 chaînons, éventuellement azoté, soufré et/ou oxygéné, et comportant éventuellement un cycle soudé, X représente une liaison directe ou un espaceur linéaire constitué de carbone, azote, oxygène et/ou soufre ayant jusqu'à 6 atomes, dont jusqu'à 2 atomes peuvent être des atomes d'azote et 1 atome peut être un atome d'oxygène ou de soufre, et Y représente l'un des groupes -CO-, -CS-, -CONH- et (lorsque X ne contient comme chaînon terminal ni un atome de soufre ni le groupe carbonyle) -SO₂-;
et dans laquelle en outre R¹ représente un atome d'hydrogène ou d'halogène ou un groupe carbamoyloxy, alcanoyloxy en C₁-C₇ ou un groupe de formule -S-Het, Het étant un groupe hétéroaromatique azoté, soufré et/ou oxygéné à 5 ou 6 chaînons, et R² représente le groupe sulfo -SO₃H, ou R¹ et R² représentent ensemble un groupe de formule 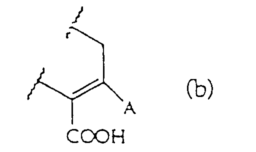 dans laquelle A représente un atome d'hydrogène ou un radical utilisable en position 3 d'antibiotiques de type céphalosporine,
et dans laquelle en outre R³ représente un atome d'hydrogène, ou R¹ et R³ forment ensemble un groupe de formule générale
=CH-R^{a} (c)
dans laquelle R^{a} représente l'un des groupes 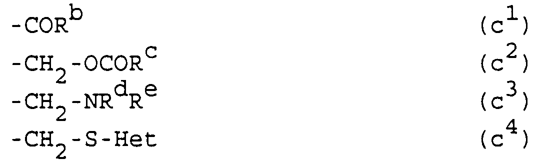 dans lesquels R^{b} représente un groupe alcoxy en C₁-C₇ ou amino, R^{c} représente un groupe alkyle en C₁-C₇, phényle ou amino, R^{d} et R^{e} représentent chacun un atome d'hydrogène ou un groupe alkyle en C₁-C₇ ou forment ensemble, avec l'atome d'azote, un hétérocycle azoté à 5 ou 6 chaînons, contenant éventuellement un autre atome d'azote, un atome d'oxygène ou un atome de soufre, et Het représente un groupe hétéro-aromatique azoté, soufré et/ou oxygéné à 5-6 chaînons, et sels pharmaceutiquement acceptables de ces composés.

2. Composés selon la revendication 1, de formule générale 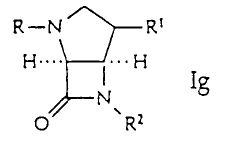 dans laquelle R représente un groupe alcoxy(C₁-C₇)carbonyle, le radical acyle d'un α-aminoacide ou un groupe acyle (hétéro)aromatique de formule générale
Ar-X-CO- (aa)
dans laquelle Ar représente un cycle (hétéro)aromatique à 5 ou 6 chaînons, contenant éventuellement un atome d'azote, de soufre et/ou un atome d'oxygène, et éventuellement lié à un autre noyau phényle soudé, et X représente une liaison directe ou l'un des groupes -NH-, -CH₂-, -CO-, -CH₂O-, -CH=CH-, -CH=C(NHCOCH₃)-, -NH-CH₂ -, -CH₂-NH-, -S-CH₂-, -O-CH₂-, -CHOH-, -CH(COOH)-, -CH(OSO₃H)-, et dans laquelle en outre R¹ représente un atome d'hydrogène et R² représente le groupe sulfo -SO₃H, ou R¹ et R² représentent ensemble un groupe de formule 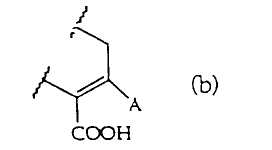 dans laquelle A représente un atome d'hydrogène ou un radical utilisable en position 3 d'antibiotiques de type céphalosporine,
et sels pharmaceutiquement acceptables de ces composés.

3. Composés selon la revendication 2, dans lesquels R représente le groupe tert-butoxycarbonyle.

4. Tert-butoxy-(1S,5R)-7-oxo-6-sulfo-2,6-diazabicyclo[3.2.0]heptane-2-carboxylate et ses sels pharmaceutiquement acceptables.

5. Monoester 2-tert-butylique d'acide (1aS,3aR,6bR)-1,1a,3a,6b-tétrahydro-5-méthoxy-1-oxo-2,6a-diazabicyclobuta[cd]indène-2,6(3H,4H)dicarboxylique et ses sels pharmaceutiquement acceptables.

6. Acide (1S,5R)-7-oxo-2-(phénylcarbamoyl)-2,6-diazabicyclo[3.2.0]heptane-6-sulfonique et ses sels pharmaceutiquement acceptables.

7. Acide (1S,5R)-2-(benzylcarbamoyl)-7-oxo-2,6-diazabicyclo[3.2.0]heptane-6-sulfonique et ses sels pharmaceutiquement acceptables.

8. Acide (1S,5R)-7-oxo-2-(N-phénylglycyl)-2,6-diazabicyclo[3.2.0]heptane-6-sulfonique et ses sels pharmaceutiquement acceptables.

9. Acide (1S,5R)-2-cyclobutylcarbamoyl-7-oxo-2,6-diazabicyclo[3.2.0]heptane-6-sulfonique et ses sels pharmaceutiquement acceptables.

10. Acide (1S,5R)-7-oxo-2-(R,S)-(2-oxo-3-tétrahydrothiénylcarbamoyl)-2,6-diazabicyclo[3.2.0]heptane-6-sulfonique et ses sels pharmaceutiquement acceptables.

11. Acide (1S,5R)-7-oxo-2-(R)-(2-oxo-3-tétrahydrothiénylcarbamoyl)-2,6-diazabicyclo[3.2.0]heptane-6-sulfonique et ses sels pharmaceutiquement acceptables.

12. Acide (1S,5R)-2-(4-hydroxyphénylcarbamoyl)-7-oxo-2,6-diazabicyclo[3.2.0]heptane-6-sulfonique et ses sels pharmaceutiquement acceptables.

13. Composés selon la revendication 2, de formule générale 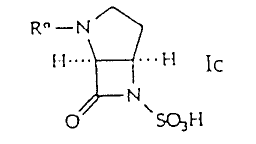 dans laquelle R^{o} représente un groupe de formule générale 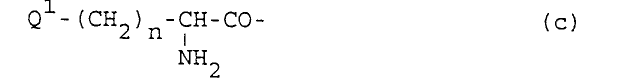 et Q¹ représente un cycle (hétéro)aromatique à 5 ou 6 chaînons contenant éventuellement un atome d'azote, de soufre et/ou un atome d'oxygène, et éventuellement lié à un autre noyau phényle soudé, et n représente le nombre 0 ou 1, et leurs sels pharmaceutiquement acceptables.

14. Acide (1S,5R)-7-oxo-2-L-tyrosyl-2,6-diazabicyclo[3.2.0]heptane-6-sulfonique et ses sels pharmaceutiquement acceptables.

15. Acide (1S,5R)-7-oxo-2-L-tryptophanyl-2,6-diazabicyclo[3.2.0]heptane-6-sulfonique et ses sels pharmaceutiquement acceptables.

16. Acide (1S,5R)-2-[(R ou S)-α-amino-(2-thiényl)acétyl]-7-oxo-2,6-diazabicyclo[3.2.0]heptane-6-sulfonique et ses sels pharmaceutiquement acceptables.

17. Acide (1S,5R)-2-[DL-2-(2-amino-4-thiazolyl)glycyl]-7-oxo-2,6-diazabicyclo[3.2.0]heptane-6-sulfonique et ses sels pharmaceutiquement acceptables.

18. Composés selon la revendication 13, dans lesquels R^{o} représente un groupe de formule générale 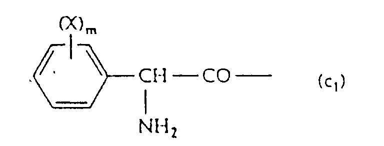 dans laquelle X représente un atome d'hydrogène ou de fluor ou le groupe hydroxy, et n représente le nombre 1 ou 2.

19. Acide (1S,5R)-7-oxo-2-(D-2-phénylglycyl)-2,6-diazabicyclo[3.2.0]heptane-6-sulfonique et ses sels pharmaceutiquement acceptables.

20. Acide (1S,5R)-7-oxo-2-(L-2-phénylglycyl)-2,6-diazabicyclo[3.2.0]heptane-6-sulfonique et ses sels pharmaceutiquement acceptables.

21. Acide (1S,5R)-2-[D-2-(p-hydroxyphényl)glycyl]-7-oxo-2,6-diazabicyclo[3.2.0]heptane-6-sulfonique et ses sels pharmaceutiquement acceptables.

22. Acide (1S,5R)-2-[R-2-(m-hydroxyphényl)glycyl]-7-oxo-2,6-diazabicyclo[3.2.0]heptane-6-sulfonique et ses sels pharmaceutiquement acceptables.

23. Acide (1S,5R)-2-[DL-2-(m-hydroxyphényl)glycyl]-7-oxo-2,6-diazabicyclo[3.2.0]heptane-6-sulfonique et ses sels pharmaceutiquement acceptables.

24. Acide (1S,5R)-2-[DL-2-(o-fluorophényl)glycyl]-7-oxo-2,6-diazabicyclo[3.2.0]heptane-6-sulfonique et ses sels pharmaceutiquement acceptables.

25. Acide (1S,5R)-2-[(R,S)-amino-(3,4-dihydroxyphényl)acétyl]-7-oxo-2,6-diazabicyclo[3.2.0]heptane-6-sulfonique et ses sels pharmaceutiquement acceptables.

26. Composés selon la revendication 1, dans lesquels R représente le radical de formule Q-X-Y-, dans laquelle X représente l'un des espaceurs
-O-, -S-, -NH-, -NH-NH-, -CH₂-, -CO-, -CH₂O-, -CH₂CH₂-, -CH=CH-, -CH₂NH-, -S-CH₂-, -SO₂-CH₂-, -O-CH₂-, -S-CH₂CH₂-, -CH₂CH₂-NH-, -CH₂-O-NH-CO-CH₂CH₂-, -CHOH-, -CH(COOH)-, -CH(OSO₃H)-, -CH(OCONH₂)-, -CH[CH(CH₃)₂]-
et Y représente l'un des groupes -CO-, -CS- et -CONH-.

27. Composés selon la revendication 1, dans lesquels R représente le radical de formule Q-X-Y-, dans lequel X représente l'un des espaceurs
-O-, -NH-, -NH-NH-, -CH₂-, -CH₂O-, -CH₂CH₂-, -CH=CH-, -CH₂NH-, -O-CH₂-, -CH₂CH₂-NH-, -CHOH-, -CH[CH(CH₃)₂]-
et Y représente le groupe -SO₂-.

28. Composés selon la revendication 1 de formule générale 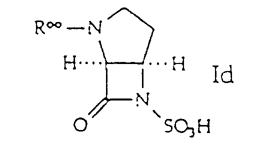 dans laquelle R^{oo} représente un groupe de formule générale
Q²-CH=CHCO- (d)
et Q² représente le noyau phényle ou un cycle hétéroaromatique à 5-6 chaînons, contenant un atome d'azote, de soufre et/ou un atome d'oxygène, et éventuellement lié à un noyau phényle soudé,
et leurs sels pharmaceutiquement acceptables.

29. Acide (1S,5R)-2-[(E)-3-(2-furyl)acryloyl]-7-oxo-2,6-diazabicyclo[3.2.0]heptane-6-sulfonique et ses sels pharmaceutiquement acceptables.

30. Acide (1S,5R)-2-[(E)-3-(3-indolyl)acryloyl]-7-oxo-2,6-diazabicyclo[3.2.0]heptane-6-sulfonique et ses sels pharmaceutiquement acceptables.

31. Acide (1S,5R)-2-[(E)-3-(4-imidazolyl)acryloyl]-7-oxo-2,6-diazabicyclo[3.2.0]heptane-6-sulfonique et ses sels pharmaceutiquement acceptables.

32. Acide (1S,5R)-2-[(E)-3-(2-thiényl)acryloyl]-7-oxo-2,6-diazabicyclo[3.2.0]heptane-6-sulfonique et ses sels pharmaceutiquement acceptables.

33. Acide (1S,5R)-(E)-2-[3-4-hydroxyphényl)acryloyl]-7-oxo-2,6-diazabicyclo[3.2.0]heptane-6-sulfonique et ses sels pharmaceutiquement acceptables.

34. Composés selon la revendication 1 de formule générale 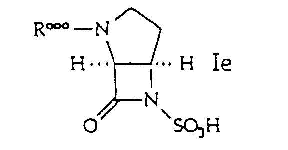 dans laquelle R^{ooo} représente un groupe de formule générale
Q³-(S)ₙ-CH₂CO- (e)
n représente 0 ou 1 et Q³ représente le noyau phényle ou un hétérocycle azoté à 5-6 chaînons, contenant éventuellement un atome de soufre, et éventuellement substitué par un groupe alkyle en C₁-C₇ ou amino, et éventuellement lié à un noyau phényle soudé,
et leurs sels pharmaceutiquement acceptables.

35. Acide (1S,5R)-2-[[(1-méthyl-1H-tétrazol-5-yl)thio]acétyl]-7-oxo-2,6-diazabicyclo[3.2.0]heptane-6-sulfonique et ses sels pharmaceutiquement acceptables.

36. Acide (1S,5R)-2-[[1H-benzotriazol-1-yl)thio]acétyl]-7-oxo-2,6-diazabicyclo[3.2.0]heptane-6-sulfonique et ses sels pharmaceutiquement acceptables.

37. Acide (1S,5R)-2-[[(5-méthyl-1,3,4-thiadiazol-2-yl)thio]acétyl]-7-oxo-2,6-diazabicyclo[3.2.0]heptane-6-sulfonique et ses sels pharmaceutiquement acceptables.

38. Acide (1S,5R)-7-oxo-2-(1H-tétrazol-1-ylacétyl)-2,6-diazabicyclo[3.2.0]heptane-6-sulfonique et ses sels pharmaceutiquement acceptables.

39. Acide (1S,5R)-2-[(2-isopropyl-2H-tétrazol-5-yl)acétyl]-7-oxo-2,6-diazabicyclo[3.2.0]heptane-6-sulfonique et ses sels pharmaceutiquement acceptables.

40. Composés de formule générale 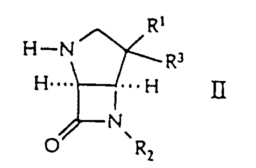 dans laquelle R¹, R² et R³ ont les significations données dans la revendication 1.

41. Composés de formule générale 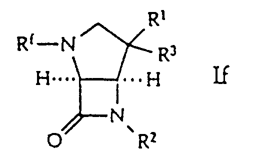 dans laquelle R¹, R² et R³ ont les significations données dans la revendication 1 et R^{f} représente un radical défini à propos de R dans la revendication 1, et comportant un(des) groupe(s) hydroxy ou un groupe amino protégés.

42. Composés de formule générale 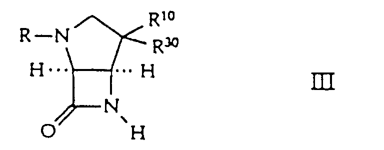 dans laquelle R a la signification donnée dans la revendication 1 et R¹⁰ et R³⁰ ont les significations données dans la revendication 46.

43. Composés de formule générale 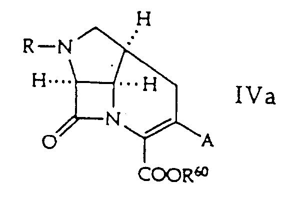 dans laquelle R a la signification donnée dans la revendication 1 et R⁶⁰ représente le groupe tert-butyle, p-nitrobenzyle, benzyle, benzhydryle ou allyle.

44. Composés selon l'une des revendications 1 à 39, en tant que substances actives pharmaceutiques.

45. Composés selon l'une des revendications 1 à 39, en tant que substances actives inhibitrices de β-lactamase.

46. Procédé pour la préparation des composés selon l'une des revendications 1 à 39, caractérisé en ce que
(a) on traite par des agents fournissant le radical R un composé de formule générale 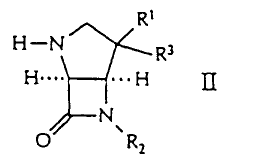 dans laquelle R¹, R² et R³ ont les significations données dans la revendication 1, ou en ce que
(b) pour la préparation d'un composé de formule I dans lequel R contient un (des) groupe(s) hydroxy ou un groupe amino libres, on élimine le(s) groupe(s) protecteur(s) de fonction hydroxy ou le groupe protecteur de fonction amino dans un composé de formule générale 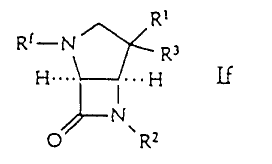 dans laquelle R¹, R² et R³ ont les significations données dans la revendication 1, et R^{f} représente un radical défini à propos de R, qui comporte un(des) groupe(s) hydroxy ou un groupe amino protégés, ou en ce que
(c) pour la préparation d'un composé de formule I dans lequel R¹ et R² sont différents du groupe (b) défini plus haut, on traite par des agents fournissant le groupe sulfo -SO₃H un composé de formule générale 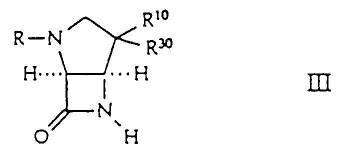 dans laquelle R a la signification donnée dans la revendication 1, R¹⁰ représente un atome d'hydrogène ou d'halogène ou un groupe carbamoyloxy, alcanoyloxy en C₁-C₇ ou un groupe de formule -S-Het, Het représentant un groupe hétéroaromatique à 5-6 chaînons et R³⁰ représentant un atome d'hydrogène, ou R¹⁰ et R³⁰ représentent ensemble un groupe de formule générale
=CH-R^{a} (c) (c)
dans laquelle R^{a} représente l'un des groupes 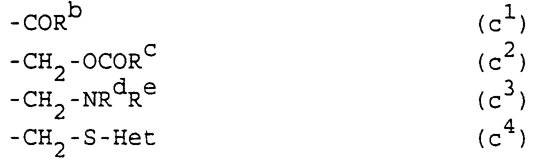 dans lesquels R^{b} représente un groupe alcoxy en C₁-C₇ ou amino, R^{c} représente un groupe alkyle en C₁-C₇, phényle ou amino, R^{d} et R^{e} représentent chacun un atome d'hydrogène ou un groupe alkyle en C₁-C₇ ou forment ensemble, avec l'atome d'azote, un hétérocycle azoté à 5 ou 6 chaînons, contenant éventuellement un autre atome d'azote, un atome d'oxygène ou un atome de soufre, et Het représente un groupe hétéroaromatique azoté, soufré et/ou oxygéné à 5-6 chaînons, ou en ce que
(d) pour la préparation d'un composé de formule I dans lequel R¹ et R² représentent ensemble le groupe (b) défini dans la revendication 1, on fait réagir avec des agents éliminant le groupe R⁶ protecteur de fonction carboxy un composé de formule générale 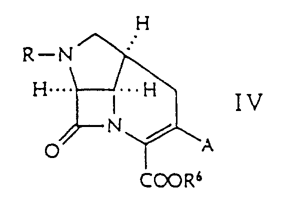 dans laquelle R et A ont les significations données dans la revendication 1 et R⁶ représente un groupe protecteur de fonction carboxy, ou en ce que
(e) pour la préparation de sels pharmaceutiquement acceptables d'un composé de formule I, on convertit en un tel sel un composé de formule I.

47. Médicaments contenant un composé selon l'une des revendications 1 à 39.

48. Médicaments inhibiteurs de β-lactamase, contenant un composé selon l'une des revendications 1 à 39.

49. Médicaments selon la revendication 47 ou 48, contenant un antibiotique de type β-lactame.

50. Médicaments selon la revendication 49, contenant une pénicilline ou une céphalosporine, en tant qu'antibiotique de type β-lactame.

51. Médicaments selon la revendication 50, contenant de la benzylpénicilline, pipéracilline, phénoxyméthylpénicilline, carbénicilline, méthicilline, propicilline, tricarcilline, ampicilline, amoxicilline, du mécillinam, ceftazidime, céfétamet, pivoxil, céfotaxime, cefménoxime, ceftizoxime, céfuroxime, de la céfaloridine, céfalotine, céfazoline, céfalexine, céfoxitine, du céfacétrile, céfamandole, de la céfapirine, céfradine, céphaloglycine, de l'acide (6R,7R)-7-[(Z)-2-(2-amino-4-thiazolyl)-2-(méthoxyimino)acétamido]-3-(azidométhyl)-8-oxo-5-thia-1-azabicyclo[4.2.0]oct-2-ène-2-carboxylique ou du (6R,7R)-7-[(Z)-2-(2-amino-4-thiazolyl)-2-(méthoxyimino)acétamido]-3-(azidométhyl)-8-oxo-5-thia-1-azabicyclo[4.2.0]oct-2-ène-2-carboxylate de (E)-2-(isobutoxycarbonyl)-2-pentényle ou un sel pharmaceutiquement acceptable de l'un de ces composés.

52. Médicaments selon la revendication 50, contenant de la ceftriaxone ou un de ses sels pharmaceutiquement acceptables.

53. Médicaments selon la revendication 52, contenant un composé selon la revendication 21.

54. Médicaments selon l'une des revendication 47 à 53, en tant que composition à plusieurs composants, pour l'administration simultanée, séparée ou échelonnée dans le temps, dans la thérapie antibactérienne.

55. Utilisation des composés selon l'une des revendications 1 à 39, dans la fabrication de produits à activité antibactérienne.

## Revendications (Revendications pour l'(les) Etat(s) contractant(s) suivant(s): ES, GR)

1. Procédé pour la préparation de β-lactames de formule générale 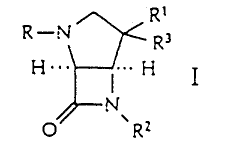 dans laquelle R représente un groupe alcoxy(C₁-C₇)carbonyle, alcoxy(C₁-C₇)carbonylamino, le reste acyle d'un α- ou β-aminoacide ou un radical de formule générale
Q-X-Y- (a)
dans laquelle Q représente un cycle à 3-6 chaînons, éventuellement azoté, soufré et/ou oxygéné, et comportant éventuellement un cycle soudé, X représente une liaison directe ou un espaceur linéaire constitué de carbone, azote, oxygène et/ou soufre ayant jusqu'à 6 atomes, dont jusqu'à 2 atomes peuvent être des atomes d'azote et 1 atome peut être un atome d'oxygène ou de soufre, et Y représente l'un des groupes -CO-, -CS-, -CONH- et (lorsque X ne contient comme chaînon terminal ni un atome de soufre ni le groupe carbonyle) -SO₂-;
et dans laquelle en outre R¹ représente un atome d'hydrogène ou d'halogène ou un groupe carbamoyloxy, alcanoyloxy en C₁-C₇ ou un groupe de formule -S-Het, Het étant un groupe hétéroaromatique à 5 ou 6 chaînons, et R² représente le groupe sulfo -SO₃H, ou R¹ et R² représentent ensemble un groupe de formule 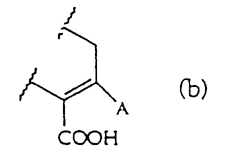 dans laquelle A représente un atome d'hydrogène ou un radical utilisable en position 3 d'antibiotiques de type céphalosporine,
et dans laquelle en outre R³ représente un atome d'hydrogène, ou R¹ et R³ forment ensemble un groupe de formule générale
=CH-R^{a} (c)
dans laquelle R^{a} représente l'un des groupes 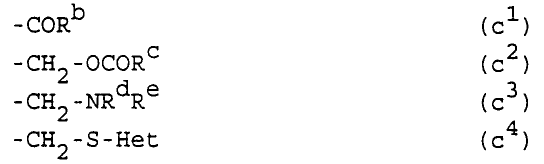 dans lesquels R^{b} représente un groupe alcoxy en C₁-C₇ ou amino, R^{c} représente un groupe alkyle en C₁-C₇, phényle ou amino, R^{d} et R^{e} représentent chacun un atome d'hydrogène ou un groupe alkyle en C₁-C₇ ou forment ensemble, avec l'atome d'azote, un hétérocycle azoté à 5 ou 6 chaînons, contenant éventuellement un autre atome d'azote, un atome d'oxygène ou un atome de soufre, et Het représente un groupe hétéroaromatique azoté, soufré et/ou oxygéné à 5-6 chaînons,
et de sels pharmaceutiquement acceptables de ces composés, caractérisé en ce que
(a) on traite par des agents fournissant le radical R un composé de formule générale 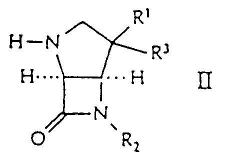 dans laquelle R¹, R² et R³ ont les significations données ci-dessus, ou en ce que
(b) pour la préparation d'un composé de formule I dans lequel R contient un (des) groupe(s) hydroxy ou un groupe amino libres, on élimine le(s) groupe(s) protecteur(s) de fonction hydroxy ou le groupe protecteur de fonction amino dans un composé de formule générale 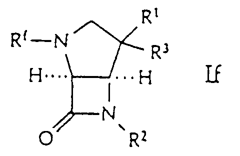 dans laquelle R¹, R² et R³ ont les significations données plus haut, et R^{f} représente un radical défini à propos de R, qui comporte un (des) groupe(s) hydroxy ou un groupe amino protégés, ou en ce que
(c) pour la préparation d'un composé de formule I dans lequel R¹ et R² sont différents du groupe (b) défini plus haut, on traite par des agents fournissant le groupe sulfo -SO₃H un composé de formule générale 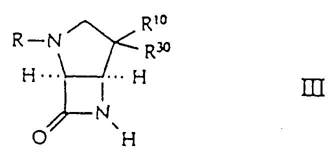 dans laquelle R a la signification donnée plus haut, R¹⁰ représente un atome d'hydrogène ou d'halogène ou un groupe carbamoyloxy, alcanoyloxy en C₁-C₇ ou un groupe de formule -S-Het, Het représentant un groupe hétéroaromatique à 5-6 chaînons et R³⁰ représentant un atome d'hydrogène, ou R¹⁰ et R³⁰ représentent ensemble un groupe de formule générale
=CH-R^{a} (c)
dans laquelle R^{a} représente l'un des groupes 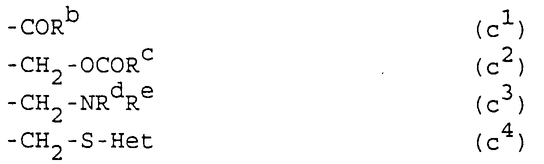 dans lesquels R^{b} représente un groupe alcoxy en C₁-C₇ ou amino, R^{c} représente un groupe alkyle en C₁-C₇, phényle ou amino, R^{d} et R^{e} représentent chacun un atome d'hydrogène ou un groupe alkyle en C₁-C₇ ou forment ensemble, avec l'atome d'azote, un hétérocycle azoté à 5 ou 6 chaînons, contenant éventuellement un autre atome d'azote, un atome d'oxygène ou un atome de soufre, et Het représente un groupe hétéroaromatique azoté, soufré et/ou oxygéné à 5-6 chaînons, ou en ce que
(d) pour la préparation d'un composé de formule I dans lequel R¹ et R² représentent ensemble le groupe (b) défini plus haut, on fait réagir avec des agents éliminant le groupe R⁶ protecteur de fonction carboxy un composé de formule générale 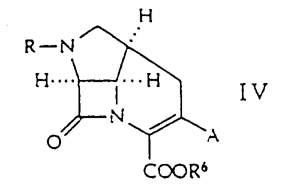 dans laquelle R et A ont les significations données plus haut et R⁶ représente un groupe protecteur de fonction carboxy, ou en ce que
(e) pour la préparation de sels pharmaceutiquement acceptables d'un composé de formule I, on convertit en un tel sel un composé de formule I.

2. Procédé pour la préparation de composés selon la revendication 1, de formule générale 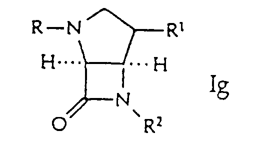 dans laquelle R représente un groupe alcoxy(C₁-C₇)carbonyle, le radical acyle d'un α-aminoacide ou un groupe acyle (hétéro)aromatique de formule générale
Ar-X-CO- (aa)
dans laquelle Ar représente un cycle (hétéro)aromatique à 5 ou 6 chaînons, contenant éventuellement un atome d'azote, de soufre et/ou un atome d'oxygène, et éventuellement lié à un autre noyau phényle soudé, et X représente une liaison directe ou l'un des groupes -NH-, -CH₂-, -CO-, -CH₂O-, -CH=CH-, -CH=C(NHCOCH₃)-, -NH-CH₂-, -CH₂-NH-, -S-CH₂-, -O-CH₂-, -CHOH-, -CH(COOH)-, -CH(OSO₃H)-, et dans laquelle en outre R¹ représente un atome d'hydrogène et R² représente le groupe sulfo -SO₃H, ou R¹ et R² représentent ensemble un groupe de formule 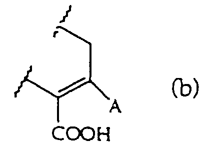 dans laquelle A représente un atome d'hydrogène ou un radical utilisable en position 3 d'antibiotiques de type céphalosporine,
et de sels pharmaceutiquement acceptables de ces composés, caractérisé en ce que l'on utilise des composés de départ convenablement substitués.

3. Procédé pour la préparation des composés selon la revendication 2, dans lesquels R représente le groupe tert-butoxycarbonyle, caractérisé en que l'on utilise des composés de départ convenablement substitués.

4. Procédé pour la préparation de composés selon la revendication 2, à savoir du tert-butoxy-(1S,5R)-7-oxo-6-sulfo-2,6-diazabicyclo[3.2.0]heptane-2-carboxylate et de ses sels pharmaceutiquement acceptables, caractérisé en que l'on utilise des composés de départ convenablement substitués.

5. Procédé pour la préparation de composés selon la revendication 2, à savoir du monoester 2-tert-butylique d'acide (1aS,3aR,6bR)-1,1a,3a,6b-tétrahydro-5-méthoxy-1-oxo-2,6a-diazabicyclobuta[cd]indène-2,6(3H,4H)dicarboxylique et de ses sels pharmaceutiquement acceptables, caractérisé en que l'on utilise des composés de départ convenablement substitués.

6. Procédé pour la préparation de composés selon la revendication 2, à savoir de l'acide (1S,5R)-7-oxo-2-(phénylcarbamoyl)-2,6-diazabicyclo[3.2.0]heptane-6-sulfonique et de ses sels pharmaceutiquement acceptables, caractérisé en que l'on utilise des composés de départ convenablement substitués.

7. Procédé pour la préparation de composés selon la revendication 2, à savoir de l'acide (1S,5R)-2-(benzylcarbamoyl)-7-oxo-2,6-diazabicyclo[3.2.0]heptane-6-sulfonique et de ses sels pharmaceutiquement acceptables, caractérisé en que l'on utilise des composés de départ convenablement substitués.

8. Procédé pour la préparation de composés selon la revendication 2, à savoir de l'acide (1S,5R)-7-oxo-2-(N-phénylglycyl)-2,6-diazabicyclo[3.2.0]heptane-6-sulfonique et de ses sels pharmaceutiquement acceptables, caractérisé en que l'on utilise des composés de départ convenablement substitués.

9. Procédé pour la préparation de composés selon la revendication 1, à savoir de l'acide (1S,5R)-2-cyclobutylcarbamoyl-7-oxo-2,6-diazabicyclo[3.2.0]heptane-6-sulfonique et de ses sels pharmaceutiquement acceptables, caractérisé en que l'on utilise des composés de départ convenablement substitués.

10. Procédé pour la préparation de composés selon la revendication 1, à savoir de l'acide (1S,5R)-7-oxo-2-(R,S)-(2-oxo-3-tétrahydrothiénylcarbamoyl)-2,6-diazabicyclo[3.2.0]heptane-6-sulfonique et de ses sels pharmaceutiquement acceptables, caractérisé en que l'on utilise des composés de départ convenablement substitués.

11. Procédé pour la préparation de composés selon la revendication 1, à savoir de l'acide (1S,5R)-7-oxo-2-(R)-(2-oxo-3-tétrahydrothiénylcarbamoyl)-2,6-diazabicyclo[3.2.0]heptane-6-sulfonique et de ses sels pharmaceutiquement acceptables, caractérisé en que l'on utilise des composés de départ convenablement substitués.

12. Procédé pour la préparation de composés selon la revendication 1, à savoir de l'acide (1S,5R)-2-(4-hydroxyphénylcarbamoyl)-7-oxo-2,6-diazabicyclo[3.2.0]heptane-6-sulfonique et de ses sels pharmaceutiquement acceptables, caractérisé en que l'on utilise des composés de départ convenablement substitués.

13. Procédé pour la préparation des composés selon la revendication 2, de formule générale 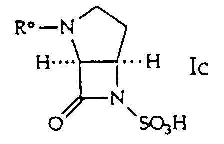 dans laquelle R^{o} représente un groupe de formule générale 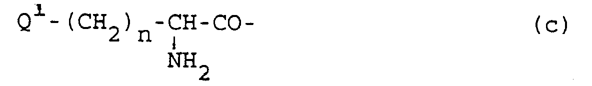 et Q¹ représente un cycle (hétéro)aromatique à 5 ou 6 chaînons contenant éventuellement un atome d'azote, de soufre et/ou un atome d'oxygène, et éventuellement lié à un autre noyau phényle soudé, et n représente le nombre 0 ou 1,et de leurs sels pharmaceutiquement acceptables, caractérisé en que l'on utilise des composés de départ convenablement substitués.

14. Procédé pour la préparation de composés selon la revendication 2, à savoir de l'acide (1S,5R)-7-oxo-2-L-tyrosyl-2,6-diazabicyclo[3.2.0]heptane-6-sulfonique et de ses sels pharmaceutiquement acceptables, caractérisé en que l'on utilise des composés de départ convenablement substitués.

15. Procédé pour la préparation de composés selon la revendication 2, à savoir de l'acide (1S,5R)-7-oxo-2-L-tryptophanyl-2,6-diazabicyclo[3.2.0]heptane-6-sulfonique et de ses sels pharmaceutiquement acceptables, caractérisé en que l'on utilise des composés de départ convenablement substitués.

16. Procédé pour la préparation de composés selon la revendication 2, à savoir de l'acide (1S,5R)-2-[(R ou S)-α-amino-(2-thiényl)acétyl]-7-oxo-2,6-diazabicyclo[3.2.0]heptane-6-sulfonique et de ses sels pharmaceutiquement acceptables, caractérisé en que l'on utilise des composés de départ convenablement substitués.

17. Procédé pour la préparation de composés selon la revendication 1, à savoir de l'acide (1S,5R)-2-[DL-2-(2-amino-4-thiazolyl)glycyl]-7-oxo-2,6-diazabicyclo[3.2.0]heptane-6-sulfonique et de ses sels pharmaceutiquement acceptables, caractérisé en que l'on utilise des composés de départ convenablement substitués.

18. Procédé pour la préparation des composés selon la revendication 13, dans lesquels R^{o} représente un groupe de formule générale 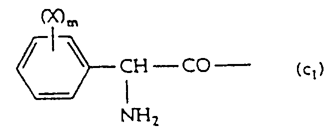 dans laquelle X représente un atome d'hydrogène ou de fluor ou le groupe hydroxy, et n représente le nombre 1 ou 2, caractérisé en ce que l'on utilise des composés de départ convenablement substitués.

19. Procédé pour la préparation de composés selon la revendication 2, à savoir de l'acide (1S,5R)-7-oxo-2-(D-2-phénylglycyl)-2,6-diazabicyclo[3.2.0]heptane-6-sulfonique et de ses sels pharmaceutiquement acceptables, caractérisé en que l'on utilise des composés de départ convenablement substitués.

20. Procédé pour la préparation de composés selon la revendication 2, à savoir de l'acide (1S,5R)-7-oxo-2-(L-2-phénylglycyl)-2,6-diazabicyclo[3.2.0]heptane-6-sulfonique et de ses sels pharmaceutiquement acceptables, caractérisé en que l'on utilise des composés de départ convenablement substitués.

21. Procédé pour la préparation de composés selon la revendication 2, à savoir de l'acide (1S,5R)-2-[D-2-(p-hydroxyphényl)glycyl]-7-oxo-2,6-diazabicyclo[3.2.0]heptane-6-sulfonique et de ses sels pharmaceutiquement acceptables, caractérisé en ce que l'on utilise des composés de départ convenablement substitués.

22. Procédé pour la préparation de composés selon la revendication 1, à savoir de l'acide (1S,5R)-2-[R-2-(m-hydroxyphényl)glycyl]-7-oxo-2,6-diazabicyclo[3.2.0]heptane-6-sulfonique et de ses sels pharmaceutiquement acceptables, caractérisé en ce que l'on utilise des composés de départ convenablement substitués.

23. Procédé pour la préparation de composés selon la revendication 1, à savoir de l'acide (1S,5R)-2-[DL-2-(m-hydroxyphényl)glycyl]-7-oxo-2,6-diazabicyclo[3.2.0]heptane-6-sulfonique et de ses sels pharmaceutiquement acceptables, caractérisé en ce que l'on utilise des composés de départ convenablement substitués.

24. Procédé pour la préparation de composés selon la revendication 1, à savoir de l'acide (1S,5R)-2-[DL-2-(o-fluorophényl)glycyl]-7-oxo-2,6-diazabicyclo[3.2.0]heptane-6-sulfonique et de ses sels pharmaceutiquement acceptables, caractérisé en ce que l'on utilise des composés de départ convenablement substitués.

25. Procédé pour la préparation de composés selon la revendication 1, à savoir de l'acide (1S,5R)-2-[(R,S)-amino-(3,4-dihydroxyphényl)acétyl]-7-oxo-2,6-diazabicyclo[3.2.0]heptane-6-sulfonique et de ses sels pharmaceutiquement acceptables, caractérisé en ce que l'on utilise des composés de départ convenablement substitués.

26. Procédé pour la préparation des composés selon la revendication 1, dans lesquels R représente le radical de formule Q-X-Y-, dans laquelle X représente l'un des espaceurs
-O-, -S-, -NH-, -NH-NH-, -CH₂-, -CO-, -CH₂O-, -CH₂CH₂-, -CH=CH-, -CH₂NH-, -S-CH₂-, -SO₂-CH₂-, -O-CH₂-, -S-CH₂CH₂-, -CH₂CH₂-NH-, -CH₂-O-NH-CO-CH₂CH₂-, -CHOH-, -CH(COOH)-, -CH(OSO₃H)-, -CH(OCONH₂)-, -CH[CH(CH₃)₂]-
et Y représente l'un des groupes -CO-, -CS- et -CONH-,
caractérisé en ce que l'on utilise des composés de départ convenablement substitués.

27. Procédé pour la préparation des composés selon la revendication 1, dans lesquels R représente le radical de formule Q-X-Y-, dans lequel X représente l'un des espaceurs
-O-, -NH-, -NH-NH-, -CH₂-, -CH₂O-, -CH₂CH₂-, -CH=CH-, -CH₂NH-, -O-CH₂-, -CH₂CH₂-NH-, -CHOH-, -CH[CH(CH₃)₂]-
et Y représente le groupe -SO₂-, caractérisé en ce que l'on utilise des composés de départ convenablement substitues.

28. Procédé pour la préparation des composés selon la revendication 1 de formule générale 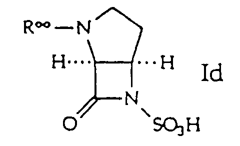 dans laquelle R^{oo} représente un groupe de formule générale
Q²-CH=CHCO- (d)
et Q² représente le noyau phényle ou un cycle hétéro-aromatique à 5-6 chaînons, contenant un atome d'azote, de soufre et/ou un atome d'oxygène, et éventuellement lié à un noyau phényle soudé,
et de leurs sels pharmaceutiquement acceptables, caractérisé en ce que l'on utilise des composés de départ convenablement substitués.

29. Procédé pour la préparation de composés selon la revendication 2, à savoir de l'acide (1S,5R)-2-[(E)-3-(2-furyl)acryloyl]-7-oxo-2,6-diazabicyclo[3.2.0]heptane-6-sulfonique et de ses sels pharmaceutiquement acceptables, caractérisé en ce que l'on utilise des composés de départ convenablement substitués.

30. Procédé pour la préparation de composés selon la revendication 2, à savoir de l'acide (1S,5R)-2-[(E)-3-(3-indolyl)acryloyl]-7-oxo-2,6-diazabicyclo[3.2.0]heptane-6-sulfonique et de ses sels pharmaceutiquement acceptables, caractérisé en ce que l'on utilise des composés de départ convenablement substitués.

31. Procédé pour la préparation de composés selon la revendication 1, à savoir de l'acide (1S,5R)-2-[(E)-3-(4-imidazolyl)acryloyl]-7-oxo-2,6-diazabicyclo[3.2.0]heptane-6-sulfonique et de ses sels pharmaceutiquement acceptables, caractérisé en ce que l'on utilise des composés de départ convenablement substitués.

32. Procédé pour la préparation de composés selon la revendication 1, à savoir de l'acide (1S,5R)-2-[(E)-3-(2-thiényl)acryloyl]-7-oxo-2,6-diazabicyclo[3.2.0]heptane-6-sulfonique et de ses sels pharmaceutiquement acceptables, caractérisé en ce que l'on utilise des composés de départ convenablement substitués.

33. Procédé pour la préparation de composés selon la revendication 2, à savoir de l'acide (1S,5R)-(E)-2-[3-4-hydroxyphényl)acryloyl]-7-oxo-2,6-diazabicyclo[3.2.0]heptane-6-sulfonique et de ses sels pharmaceutiquement acceptables, caractérisé en ce que l'on utilise des composés de départ convenablement substitués.

34. Procédé pour la préparation des composés selon la revendication 1, de formule générale 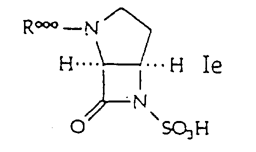 dans laquelle R^{ooo} représente un groupe de formule générale
Q³-(S)ₙ-CH₂CO- (e)
n représente 0 ou 1 et Q³ représente le noyau phényle ou un hétérocycle azoté à 5-6 chaînons, contenant éventuellement un atome de soufre, et éventuellement substitué par un groupe alkyle en C₁-C₇ ou amino, et éventuellement lié à un noyau phényle soudé,
et de leurs sels pharmaceutiquement acceptables, caractérisé en ce que l'on utilise des composés de départ convenablement substitués.

35. Procédé pour la préparation de composés selon la revendication 2, à savoir de l'acide (1S,5R)-2-[[(1-méthyl-1H-tétrazol-5-yl)-thio]acétyl]-7-oxo-2,6-diazabicyclo[3.2.0]heptane-6-sulfonique et de ses sels pharmaceutiquement acceptables, caractérisé en ce que l'on utilise des composés de départ convenablement substitués.

36. Procédé pour la préparation de composés selon la revendication 2, à savoir de l'acide (1S,5R)-2-[[1H-benzotriazol-1-yl)thio]acétyl]-7-oxo-2,6-diazabicyclo[3.2.0]heptane-6-sulfonique et de ses sels pharmaceutiquement acceptables, caractérisé en ce que l'on utilise des composés de départ convenablement substitués.

37. Procédé pour la préparation de composés selon la revendication 1, à savoir de l'acide (1S,5R)-2-[[(5-méthyl-1,3,4-thiadiazol-2-yl)thio]acétyl]-7-oxo-2,6-diazabicyclo[3.2.0]heptane-6-sulfonique et de ses sels pharmaceutiquement acceptables, caractérisé en ce que l'on utilise des composés de départ convenablement substitués.

38. Procédé pour la préparation de composés selon la revendication 1, à savoir de l'acide (1S,5R)-7-oxo-2-(1H-tétrazol-1-ylacétyl)-2,6-diazabicyclo[3.2.0]heptane-6-sulfonique et de ses sels pharmaceutiquement acceptables, caractérisé en ce que l'on utilise des composés de départ convenablement substitués.

39. Procédé pour la préparation de composés selon la revendication 1, à savoir de l'acide (1S,5R)-2-[(2-isopropyl-2H-tétrazol-5-yl)-acétyl]-7-oxo-2,6-diazabicyclo [3.2.0]heptane-6-sulfonique et de ses sels pharmaceutiquement acceptables, caractérisé en ce que l'on utilise des composés de départ convenablement substitués.

40. Procédé pour la préparation de compositions pharmaceutiques, caractérisé en ce que l'on mélange un β-lactame de formule I défini dans la revendication 1, ou un sel pharmaceutiquement acceptable de ce composé, avec des véhicules et/ou excipients solides ou liquides, inertes, non toxiques, usuels dans de telles compositions, et appropriés à l'administration thérapeutique.

41. Procédé selon la revendication 40, caractérisé en ce que l'on ajoute en plus un antibiotique de type β-lactame.

42. Procédé selon la revendication 41, caractérisé en ce que l'on utilise comme antibiotique de type β-lactame une pénicilline ou une céphalosporine.

43. Procédé selon la revendication 42, caractérisé en ce que l'on utilise comme antibiotique de type β-lactame la ceftriaxone ou un de ses sels pharmaceutiquement acceptables.

44. Procédé selon la revendication 43, caractérisé en ce que l'on utilise un composé défini dans la revendication 21.

45. Utilisation de composés selon l'une des revendications 1 à 39, dans la fabrication de médicaments destinés au traitement ou à la prophylaxie de maladies infectieuses.

46. L'invention, essentiellement telle qu'elle est décrite précédemment.

47. Composés de formule générale 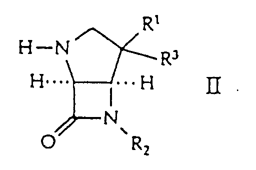 dans laquelle R¹, R² et R³ ont les significations données dans la revendication 1.

48. Composés de formule générale 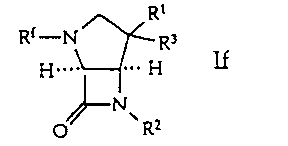 dans laquelle R¹, R² et R³ ont les significations données dans la revendication 1, et R^{f} représente un radical acyle défini à propos de R dans la revendication 1 et comportant un(des) groupe(s) hydroxy ou un groupe amino protégés.

49. Composés de formule générale 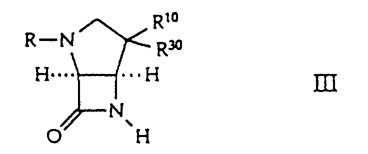 dans laquelle R, R¹⁰ et R³⁰ ont les significations données dans la revendication 1.

50. Composés de formule générale 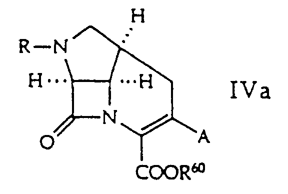 dans laquelle R a la signification donnée dans la revendication 1 et R⁶⁰ représente le groupe tert-butyle, p-nitrobenzyle, benzyle, benzhydryle ou allyle.
